(19) Europäisches Patentamt
European Patent Office
Office européen des brevets

(11) EP 4 289 853 A1

(12) **EUROPEAN PATENT APPLICATION**
published in accordance with Art. 153(4) EPC

(43) Date of publication:
**13.12.2023 Bulletin 2023/50**

(21) Application number: **22736516.0**

(22) Date of filing: **05.01.2022**

(51) International Patent Classification (IPC):
***C07D 519/00*** *(2006.01)* ***A61K 31/527*** *(2006.01)*
***A61K 31/519*** *(2006.01)* ***A61P 35/00*** *(2006.01)*

(52) Cooperative Patent Classification (CPC):
**A61K 31/519; A61K 31/527; A61P 35/00; C07D 519/00**

(86) International application number:
**PCT/CN2022/070195**

(87) International publication number:
**WO 2022/148354 (14.07.2022 Gazette 2022/28)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **05.01.2021 CN 202110007766**
**16.07.2021 CN 202110804449**

(71) Applicants:
- **Shandong Xuanzhu Pharma Co., Ltd.**
  **National High-Tech Development Zone**
  **Jinan, Shandong 250101 (CN)**
- **Xuanzhu Biopharmaceutical Co., Ltd.**
  **Shijiazhuang, Hebei 050035 (CN)**

(72) Inventors:
- **LIU, Bin**
  **Jinan, Shandong 250101 (CN)**
- **CHEN, Bo**
  **Jinan, Shandong 250101 (CN)**
- **LIU, Yuesheng**
  **Jinan, Shandong 250101 (CN)**
- **MA, Kelin**
  **Jinan, Shandong 250101 (CN)**

(74) Representative: **HGF**
**HGF Europe LLP**
**Neumarkter Straße 18**
**81673 München (DE)**

(54) **POLYCYCLIC KINASE INHIBITOR**

(57) The present disclosure belongs to the technical field of medicines, and specifically relates to a polycyclic DNA-PK kinase inhibitor compound as shown in formula (I), a pharmaceutically acceptable salt thereof or an isomer thereof, a pharmaceutical composition and formulation comprising the compound, the pharmaceutically acceptable salt thereof or the isomer thereof, a method for preparing the compound, the pharmaceutically acceptable salt thereof or the isomer thereof, and a use of the compound, the pharmaceutically acceptable salt thereof or the isomer thereof.

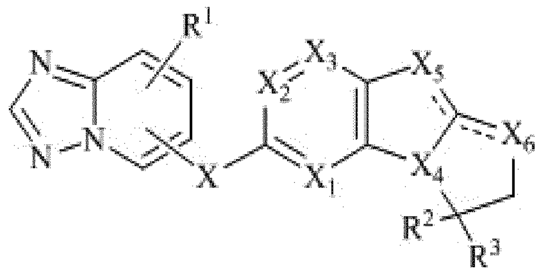

(I)

EP 4 289 853 A1

## Description

### Technical Field

The present disclosure belongs to the technical field of medicines, and specifically relates to a polycyclic DNA-PK kinase inhibitor compound, a pharmaceutically acceptable salt thereof or an isomer thereof, a pharmaceutical composition and formulation containing the compound, the pharmaceutically acceptable salt thereof or the isomer thereof, a method for preparing the compound, the pharmaceutically acceptable salt thereof or the isomer thereof, and a use of the compound, the pharmaceutically acceptable salt thereof or the isomer thereof.

### Background

As a kind of worldwide malignant disease, cancer is difficult to treat, and it has high mortality rate, which brings a heavy burden to patients and families, and is a main disease that affects the health of Chinese residents. In recent years, the incidence rate of the cancer in China has increased significantly, and the mortality rate of cancer also shows a gradual upward trend. Therefore, cancer prevention and treatment is facing a severe situation.

At present, radiotherapy and chemotherapy are the most effective measures for treating the cancer besides surgical resection, and the radiotherapy is the most effective non-surgical treatment for malignant tumors. Radiation and a considerable number of anti-cancer drugs may directly or indirectly act on DNA or DNA metabolic process, resulting in DNA damage. Herein, DNA double strand break (DSB) is the most lethal for cancer cells. After the DNA damage, it may trigger a series of cell responses such as damaged DNA repair, and the result of repair is to improve the survival of the cancer cells, this is also one of mechanisms of the tumor cells resistance to the radiotherapy and chemotherapy. If DSB is not repaired timely and completely, the cancer cells may die due to apoptosis or/and mitotic catastrophe. Therefore, as long as the repair of these DNA damages is inhibited, the sensitivity of the cancer cells to the radiotherapy and chemotherapy may be improved, and cell proliferation can be inhibited.

In human and other higher eukaryote cells, the repair of DSB is mainly performed by DNA nonhomologous end joining (NHEJ) dominated by DNA-dependent protein kinase (DNA-PK), thus the damaged DNA is repaired, and the cell activity and genome stability are maintained. NHEJ repair is mainly involved in G1/S-phase DNA damage repair and does not require a DNA end joining template. The NHEJ repair requires the co-coordination of many proteins and signaling pathways. A heterodimer of a Ku70/80 subunit and a catalytic subunit DNA dependent protein kinase (DNA-PKcs) form an active DNA-PK enzyme complex together.

DNA-PKcs belongs to the phosphatidylinositol 3 kinase (PI3K) superfamily and is a serine/threonine protein kinase; the PI3K superfamily also includes ATM, ATR, mTOR, and four PI3K subtypes. While DNA-PK binds to a broken DNA, its kinase activity may be activated. The important function of Ku is to bind to the terminal of DNA and recruit DNA-PKcs, which constitute a DNA-PK holoenzyme and activate DNA-PKcs; and the activated DNA-PKcs guide an Artemis protein (an endonuclease) to bind to the damaged site, and is subjected to DNA broken end treatment depending on its ribozyme activity to facilitate linkage repair, then an XRCC4/DNA-ligase IV complex is recruited by the activated DNA-PKcs, and finally, the DNA-ligase IV locates and links the ends of the broken DNA double strand so as to complete the repair. XRCC4 is a protein that forms a complex with the DNA-ligase IV and may increase the activity of the DNA-ligase IV. DNA-PKcs has 40 self-phosphorylation amino acid residues, and the most typical self-phosphorylation site occurs in Ser2056 (POR cluster) and Thr2609 (ABCDE cluster). NHEJ is considered to be performed by three key steps: DSB-Ku70/80 binding to an incomplete DNA end is identified, and two molecules of DNA-PKcs to the adjacent side of DSB are recruited; DNA engineering is performed to remove a non-connectable end or other damage forms from endpoints; and finally, the end of DNA is linked.

Because the tumor cells have a higher basic level of an endogenous replication pressure (oncogene-induced replication pressure) and DNA damage, and the efficiency of DNA repair mechanisms in the tumor cells is relatively low, the tumor cells are more sensitive to DNA-PK.

At present, the development of DNA-PK inhibitors with high efficiency and good selectivity has important clinical significance. It may synergistically enhance the effects of radiotherapy and chemotherapy, effectively inhibit the tumor growth, and effectively reduce the damage to normal cells and reduce the side effects.

### Summary

A technical problem to be solved by the present disclosure is to provide a polycyclic compound with a novel structure and a good inhibitory effect on DNA-PK. Further, this compound may be used to increase the sensitivity of a subject to radiotherapy and/or one or more anticancer agents. Further, this compound may be used to prevent and/or treat a benign tumor or cancer in combination with the radiotherapy and/or one or more anticancer agents.

Embodiments of the present disclosure are as follows.

In one aspect, the present disclosure provides a compound of general formula (I), a pharmaceutically acceptable salt thereof, or an isomer thereof,

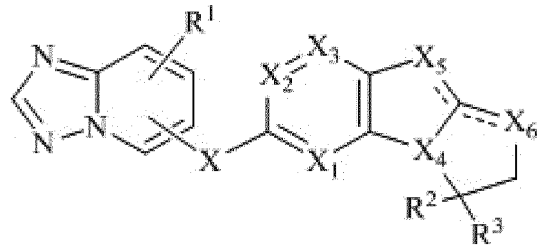

(I)

Wherein,

$X_1$, $X_2$, $X_3$, and $X_4$ are respectively independently selected from C ($R^4$) or N;
$X_5$ and $X_6$ are respectively independently selected from CH ($R^5$), C ($R^6$), N ($R^7$), or N;
Xis $CH_2$, NH, O, or S;
$R^1$ is selected from a group consisting of H, halogen, hydroxyl, amino, nitro, cyano, $C_{1-6}$ alkyl, $C_{1-6}$ alkyl amino, di ($C_{1-6}$ alkyl) amino, halo $C_{1-6}$ alkyl, hydroxy $C_{1-6}$ alkyl, amino $C_{1-6}$ alkyl, $C_{1-6}$ alkoxy, $C_{1-6}$ alkylthio, halo $C_{1-6}$ alkoxy, halo $C_{1-6}$ alkylthio, hydroxy $C_{1-6}$ alkoxy, hydroxy $C_{1-6}$ alkylthio, amino $C_{1-6}$ alkoxy, and amino $C_{1-6}$ alkylthio;
$R^2$ and $R^3$ are respectively independently selected from a group consisting of H, halogen, hydroxyl, amino, nitro, cyano, $C_{1-6}$ alkyl, halo $C_{1-6}$ alkyl, hydroxy $C_{1-6}$ alkyl, amino $C_{1-6}$ alkyl, $C_{1-6}$ alkoxy, halo $C_{1-6}$ alkoxy, hydroxy $C_{1-6}$ alkoxy, amino $C_{1-6}$ alkoxy, and 3-8 membered cycloalkyl or 3-8 membered heterocyclic group substituted optionally by 1-3 Q1; and each Q1 is independently selected from a group consisting of halogen, hydroxyl, amino, nitro, cyano, $C_{1-6}$ alkyl, halo $C_{1-6}$ alkyl, $C_{1-6}$ alkoxy and halo $C_{1-6}$ alkoxy;
or $R^2$, $R^3$ and the carbon atom linked to them together form 3-8 membered cycloalkyl or 3-8 membered heterocyclic group substituted optionally by 1-3 Q2; and each Q2 is independently selected from a group consisting of halogen, hydroxyl, amino, nitro, cyano, carbonyl, $C_{1-6}$ alkyl, halo $C_{1-6}$ alkyl, $C_{1-6}$ alkyl amino, ($C_{1-6}$ alkyl)$_2$ amino, $C_{1-6}$ alkoxy and halo $C_{1-6}$ alkoxy;
each $R^4$, each $R^5$, and each $R^6$ are respectively independently selected from a group consisting of H, halogen, hydroxyl, amino, nitro, cyano, $C_{1-6}$ alkyl, halo $C_{1-6}$ alkyl, hydroxy $C_{1-6}$ alkyl, amino $C_{1-6}$ alkyl, $C_{1-6}$ alkoxy, halo $C_{1-6}$ alkoxy, hydroxy $C_{1-6}$ alkoxy and amino $C_{1-6}$ alkoxy;
each $R^7$ is respectively independently selected from a group consisting of H, $C_{1-6}$ alkyl, halo $C_{1-6}$ alkyl, hydroxy $C_{1-6}$ alkyl and amino $C_{1-6}$ alkyl; and
the virtual bond --- is a chemical bond or does not exist, and the adjacent virtual bond is not a chemical bond at the same time.

In some embodiments, the compound of general formula (I), the pharmaceutically acceptable salt thereof, or the isomer thereof further has a structure of general formula (IIa),

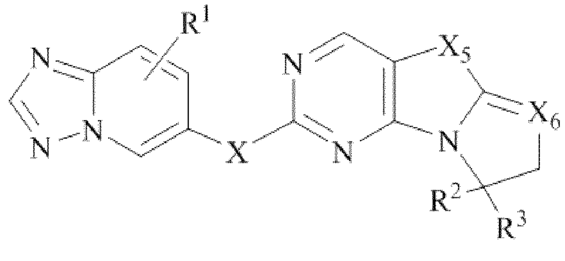

(IIa)

wherein, $X_5$ is CH ($R^5$) or N ($R^7$);
$X_6$ is C ($R^6$) or N;
Xis $CH_2$, NH, O, or S;
$R^1$ is selected from a group consisting of H, halogen, hydroxyl, amino, nitro, cyano, $C_{1-6}$ alkyl, halo $C_{1-6}$ alkyl, hydroxy $C_{1-6}$ alkyl, amino $C_{1-6}$ alkyl, $C_{1-6}$ alkoxy, halo $C_{1-6}$ alkoxy, hydroxy $C_{1-6}$ alkoxy, and amino $C_{1-6}$ alkoxy;
$R^2$ and $R^3$ are respectively independently selected from a group consisting of H, halogen, hydroxyl, amino, nitro, cyano, $C_{1-6}$ alkyl, halo $C_{1-6}$ alkyl, hydroxy $C_{1-6}$ alkyl, amino $C_{1-6}$ alkyl, $C_{1-6}$ alkoxy, halo $C_{1-6}$ alkoxy, hydroxy $C_{1-6}$

alkoxy, amino $C_{1-6}$ alkoxy, and 3-8 membered cycloalkyl or 3-8 membered heterocyclic group, which could be optionally substituted by 1-2 Q1; and each Q1 is independently selected from a group consisting of halogen, hydroxyl, amino, nitro, cyano, $C_{1-6}$ alkyl, halo $C_{1-6}$ alkyl, $C_{1-6}$ alkoxy and halo $C_{1-6}$ alkoxy;

or $R^2$, $R^3$ and the carbon atom linked to them together form 3-8 membered cycloalkyl or 3-8 membered heterocyclic group, which could be optionally substituted by 1-2 Q2; and each Q2 is independently selected from a group consisting of halogen, hydroxyl, amino, nitro, cyano, carbonyl, $C_{1-6}$ alkyl, halo $C_{1-6}$ alkyl, $C_{1-6}$ alkyl amino, $(C_{1-6}$ alkyl$)_2$ amino, $C_{1-6}$ alkoxy and halo $C_{1-6}$ alkoxy;

$R^5$ and $R^6$ are respectively independently selected from a group consisting of H, halogen, hydroxyl, amino, nitro, cyano, $C_{1-6}$ alkyl, halo $C_{1-6}$ alkyl, hydroxy $C_{1-6}$ alkyl, amino $C_{1-6}$ alkyl, $C_{1-6}$ alkoxy, halo $C_{1-6}$ alkoxy, hydroxy $C_{1-6}$ alkoxy and amino $C_{1-6}$ alkoxy; and

$R^7$ is selected from a group consisting of H, $C_{1-6}$ alkyl, halo $C_{1-6}$ alkyl, hydroxy $C_{1-6}$ alkyl, and amino $C_{1-6}$ alkyl.

In some embodiments, the compound of general formula (I), the pharmaceutically acceptable salt thereof, or the isomer thereof further has a structure of general formula (IIb),

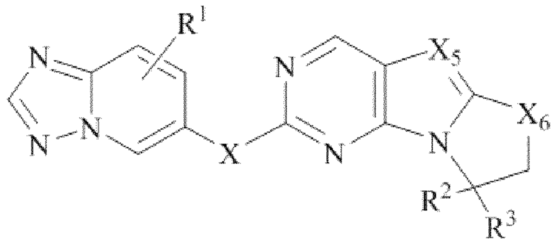

(IIb)

wherein, $X_5$ is C ($R^6$) or N;

$X_6$ is CH ($R^5$) or N ($R^7$);

Xis $CH_2$, NH, O, or S;

$R^1$ is selected from a group consisting of H, halogen, hydroxyl, amino, nitro, cyano, $C_{1-6}$ alkyl, halo $C_{1-6}$ alkyl, hydroxy $C_{1-6}$ alkyl, amino $C_{1-6}$ alkyl, $C_{1-6}$ alkoxy, halo $C_{1-6}$ alkoxy, hydroxy $C_{1-6}$ alkoxy, and amino $C_{1-6}$ alkoxy;

$R^2$ and $R^3$ are respectively independently selected from a group consisting of H, halogen, hydroxyl, amino, nitro, cyano, $C_{1-6}$ alkyl, halo $C_{1-6}$ alkyl, hydroxy $C_{1-6}$ alkyl, amino $C_{1-6}$ alkyl, $C_{1-6}$ alkoxy, halo $C_{1-6}$ alkoxy, hydroxy $C_{1-6}$ alkoxy, amino $C_{1-6}$ alkoxy, and 3-8 membered cycloalkyl or 3-8 membered heterocyclic group, which could be optionally substituted by 1-2 Q1; and each Q1 is independently selected from a group consisting of halogen, hydroxyl, amino, nitro, cyano, $C_{1-6}$ alkyl, halo $C_{1-6}$ alkyl, $C_{1-6}$ alkoxy or halo $C_{1-6}$ alkoxy;

or $R^2$, $R^3$ and the carbon atom linked to them together form 3-8 membered cycloalkyl or 3-8 membered heterocyclic group, which could be optionally substituted by 1-2 Q2; and each Q2 is independently selected from a group consisting of halogen, hydroxyl, amino, nitro, cyano, carbonyl, $C_{1-6}$ alkyl, halo $C_{1-6}$ alkyl, $C_{1-6}$ alkyl amino, $(C_{1-6}$ alkyl$)_2$ amino, $C_{1-6}$ alkoxy and halo $C_{1-6}$ alkoxy;

$R^5$ and $R^6$ are respectively independently selected from a group consisting of H, halogen, hydroxyl, amino, nitro, cyano, $C_{1-6}$ alkyl, halo $C_{1-6}$ alkyl, hydroxy $C_{1-6}$ alkyl, amino $C_{1-6}$ alkyl, $C_{1-6}$ alkoxy, halo $C_{1-6}$ alkoxy, hydroxy $C_{1-6}$ alkoxy and amino $C_{1-6}$ alkoxy; and

$R^7$ is selected from a group consisting of H, $C_{1-6}$ alkyl, halo $C_{1-6}$ alkyl, hydroxy $C_{1-6}$ alkyl, and amino $C_{1-6}$ alkyl.

In some embodiments, $R^2$ and $R^3$ are respectively independently selected from a group consisting of H, halogen, hydroxyl, amino, nitro, cyano, $C_{1-6}$ alkyl, halo $C_{1-6}$ alkyl, hydroxy $C_{1-6}$ alkyl, amino $C_{1-6}$ alkyl, $C_{1-6}$ alkoxy, halo $C_{1-6}$ alkoxy, hydroxy $C_{1-6}$ alkoxy, amino $C_{1-6}$ alkoxy, and 3-8 membered cycloalkyl or 3-8 membered heterocyclic group, which could be optionally substituted by 1-2 Q1; and each Q1 is independently selected from a group consisting of halogen, hydroxyl, amino, nitro, cyano, $C_{1-6}$ alkyl, halo $C_{1-6}$ alkyl, $C_{1-6}$ alkoxy and halo $C_{1-6}$ alkoxy.

In some embodiments, $R^2$ and $R^3$ are respectively independently selected from a group consisting of H, halogen, hydroxyl, amino, nitro, cyano, $C_{1-6}$ alkyl, halo $C_{1-6}$ alkyl, hydroxy $C_{1-6}$ alkyl, amino $C_{1-6}$ alkyl, $C_{1-6}$ alkoxy, halo $C_{1-6}$ alkoxy, hydroxy $C_{1-6}$ alkoxy and amino $C_{1-6}$ alkoxy.

In some embodiments, $R^2$, $R^3$ and the carbon atom linked to them together form 3-8 membered cycloalkyl or 3-8 membered heterocyclic group containing 1-2 heteroatoms, which could be optionally substituted by 1-2 Q2, and the heteroatom is N, O, or S; and each Q2 is independently selected from a group consisting of halogen, hydroxyl, amino, nitro, cyano, carbonyl, $C_{1-6}$ alkyl, halo $C_{1-6}$ alkyl, $C_{1-6}$ alkyl amino, $(C_{1-6}$ alkyl$)_2$ amino, $C_{1-6}$ alkoxy and halo $C_{1-6}$ alkoxy.

In some embodiments, $R^2$, $R^3$ and the carbon atom linked to them together form 3-7 membered cycloalkyl or 3-7 membered heterocyclic group, which could be optionally substituted by 1-2 Q2; and each Q2 is independently

selected from a group consisting of halogen, hydroxyl, amino, nitro, cyano, carbonyl, $C_{1-6}$ alkyl, halo $C_{1-6}$ alkyl, $C_{1-6}$ alkyl amino, $(C_{1-6}$ alkyl$)_2$ amino, $C_{1-6}$ alkoxy, or halo $C_{1-6}$ alkoxy.

In some embodiments, $R^2$, $R^3$ and the carbon atom linked to them together form 3-7 membered cycloalkyl or 3-7 membered heterocyclic group containing 1-2 heteroatoms, which could be optionally substituted by 1-2 Q2, and the heteroatom is N, O, or S; and each Q2 is independently selected from a group consisting of halogen, hydroxyl, amino, nitro, cyano, carbonyl, $C_{1-6}$ alkyl, halo $C_{1-6}$ alkyl, $C_{1-6}$ alkyl amino, $(C_{1-6}$ alkyl$)_2$ amino, $C_{1-6}$ alkoxy and halo $C_{1-6}$ alkoxy.

In some embodiments, $R^2$, $R^3$ and the carbon atom linked to them together form 5-7 membered cycloalkyl or 5-7 membered heterocyclic group, which could be optionally substituted by 1-2 Q2; and each Q2 is independently selected from a group consisting of halogen, hydroxyl, amino, nitro, cyano, carbonyl, $C_{1-6}$ alkyl, halo $C_{1-6}$ alkyl, $C_{1-6}$ alkyl amino, $(C_{1-6}$ alkyl$)_2$ amino, $C_{1-6}$ alkoxy, and halo $C_{1-6}$ alkoxy.

In some embodiments, $R^2$, $R^3$ and the carbon atom linked to them together form 5-7 membered cycloalkyl or 5-7 membered heterocyclic group containing 1-2 heteroatoms, which could be optionally substituted by 1-2 Q2, and the heteroatom is N, O, or S; and each Q2 is independently selected from a group consisting of halogen, hydroxyl, amino, nitro, cyano, carbonyl, $C_{1-6}$ alkyl, halo $C_{1-6}$ alkyl, $C_{1-6}$ alkyl amino, $(C_{1-6}$ alkyl$)_2$ amino, $C_{1-6}$ alkoxy and halo $C_{1-6}$ alkoxy.

In some embodiments, $R^2$, $R^3$ and the carbon atom linked to them together form 5-6 membered cycloalkyl or 5-6 membered heterocyclic group, which could be optionally substituted by 1-2 Q2; and each Q2 is independently selected from a group consisting of halogen, hydroxyl, amino, nitro, cyano, carbonyl, $C_{1-6}$ alkyl, halo $C_{1-6}$ alkyl, $C_{1-6}$ alkyl amino, $(C_{1-6}$ alkyl$)_2$ amino, $C_{1-6}$ alkoxy, or halo $C_{1-6}$ alkoxy.

In some embodiments, $R^2$, $R^3$ and the carbon atom linked to them together form 5-6 membered cycloalkyl or 5-6 membered heterocyclic group containing 1-2 heteroatoms, which could be optionally substituted by 1-2 Q2, and the heteroatom is N, O, or S; and each Q2 is independently selected from a group consisting of halogen, hydroxyl, amino, nitro, cyano, carbonyl, $C_{1-6}$ alkyl, halo $C_{1-6}$ alkyl, $C_{1-6}$ alkyl amino, $(C_{1-6}$ alkyl$)_2$ amino, $C_{1-6}$ alkoxy, or halo $C_{1-6}$ alkoxy.

In some embodiments, $R^2$, $R^3$ and the carbon atom linked to them together form the following groups substituted optionally by 1-2 Q2:

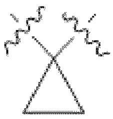, 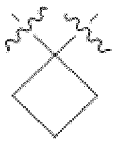, 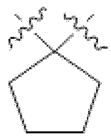, 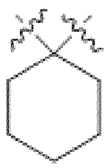, 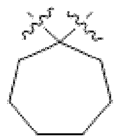, 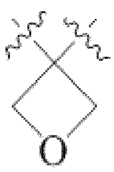, 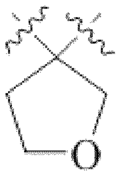, 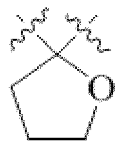,

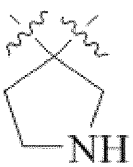, 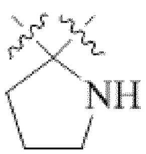, 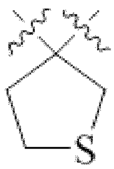, 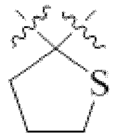, 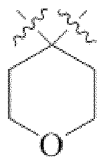, 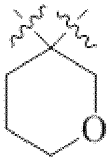, 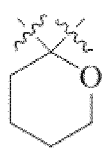, 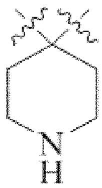,

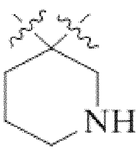, 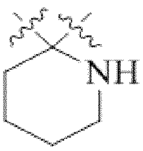, 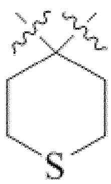, 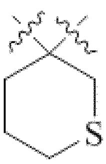, 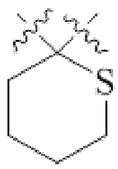, 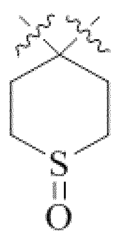, 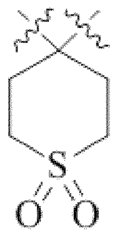, 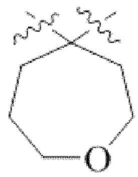,

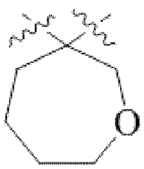, 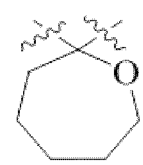, 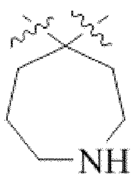, 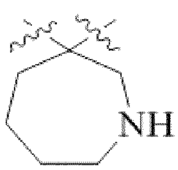, 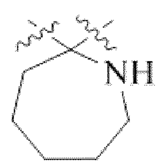, 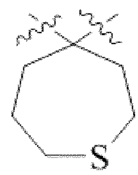, 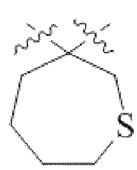

or

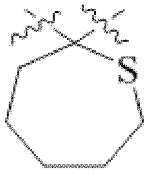;

each Q2 is independently selected from a group consisting of halogen, hydroxyl, amino, nitro, cyano, carbonyl, $C_{1-6}$ alkyl, halo $C_{1-6}$ alkyl, $C_{1-6}$ alkyl amino, ($C_{1-6}$ alkyl)$_2$ amino, $C_{1-6}$ alkoxy and halo $C_{1-6}$ alkoxy.

In some embodiments, $R^1$ is selected from a group consisting of H, halogen, hydroxyl, amino, nitro, cyano, $C_{1-6}$ alkyl, halo $C_{1-6}$ alkyl, hydroxy $C_{1-6}$ alkyl, amino $C_{1-6}$ alkyl, $C_{1-6}$ alkoxy, halo $C_{1-6}$ alkoxy, hydroxy $C_{1-6}$ alkoxy and amino $C_{1-6}$ alkoxy;

$R^2$ and $R^3$ are respectively independently selected from a group consisting of H, halogen, hydroxyl, amino, nitro, cyano, $C_{1-6}$ alkyl, halo $C_{1-6}$ alkyl, hydroxy $C_{1-6}$ alkyl, amino $C_{1-6}$ alkyl, $C_{1-6}$ alkoxy, halo $C_{1-6}$ alkoxy, hydroxy $C_{1-6}$ alkoxy and amino $C_{1-6}$ alkoxy;

or $R^2$, $R^3$ and the carbon atom linked to them together form the following groups substituted optionally by 1-2 Q2:

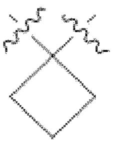, 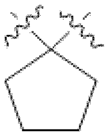, 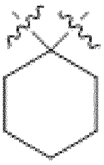, 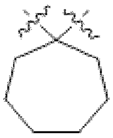, 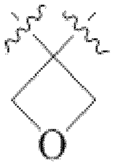, 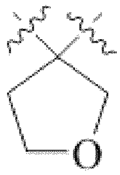, 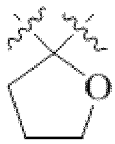,

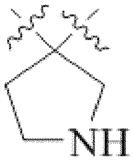, 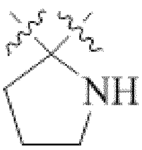, 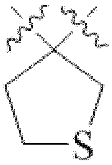, 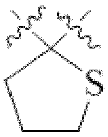, 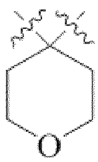, 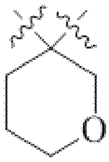, 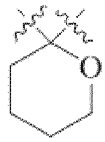, 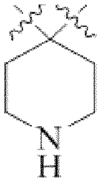,

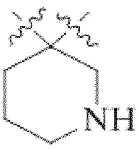, 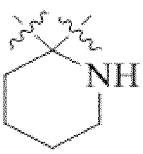, 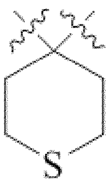, 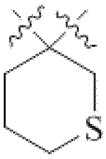, 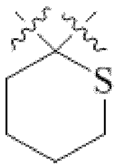, 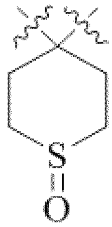, 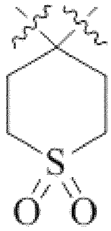, 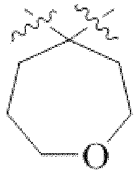,

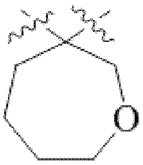, 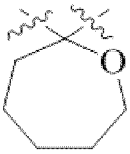, 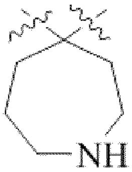, 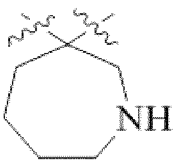, 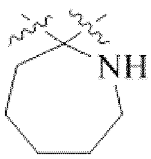, 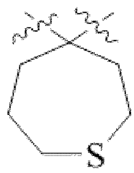, 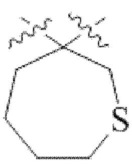

or

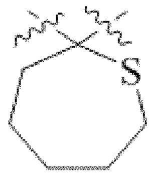;

each Q2 is independently selected from halogen, hydroxyl, amino, nitro, cyano, carbonyl, $C_{1-6}$ alkyl, halo $C_{1-6}$ alkyl, $C_{1-6}$ alkyl amino, $(C_{1-6}\ alkyl)_2$ amino, $C_{1-6}$ alkoxy and halo $C_{1-6}$ alkoxy;
$R^4$ is H; $R^5$ and $R^6$ are respectively independently selected from a group consisting of H, halogen, hydroxyl, amino, nitro, cyano, $C_{1-6}$ alkyl, halo $C_{1-6}$ alkyl, hydroxy $C_{1-6}$ alkyl, amino $C_{1-6}$ alkyl, $C_{1-6}$ alkoxy and halo $C_{1-6}$ alkoxy; and
$R^7$ is selected from a group consisting of H, $C_{1-6}$ alkyl, halo $C_{1-6}$ alkyl, hydroxy $C_{1-6}$ alkyl and amino $C_{1-6}$ alkyl.

In some embodiments, $R^2$, $R^3$ and the carbon atom linked to them together form the following groups substituted optionally by 1-2 Q2:

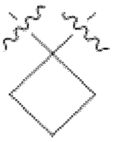, 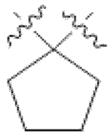, 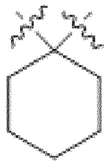, 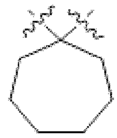, 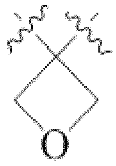, 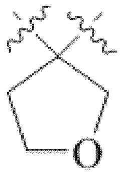, 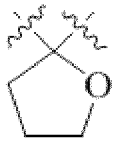,

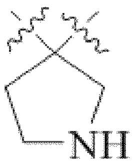, 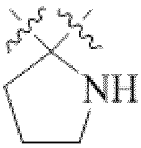, 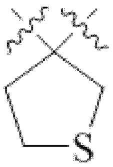, 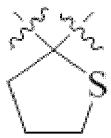, 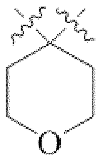, 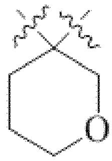, 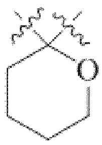, 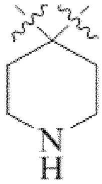,

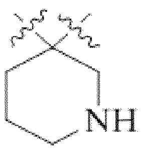, 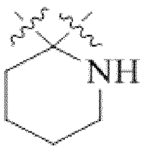, 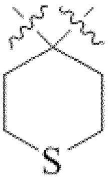, 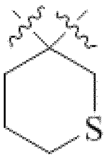, 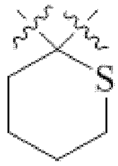, 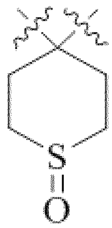, 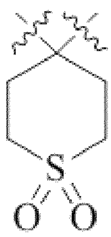, 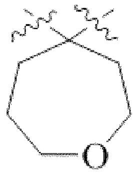,

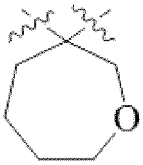, 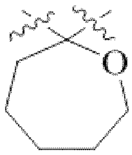, 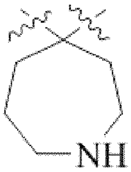, 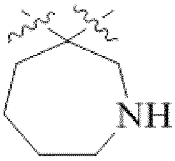, 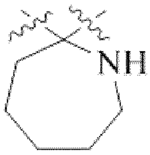, 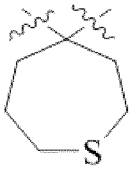, 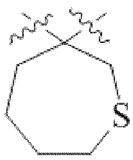

or

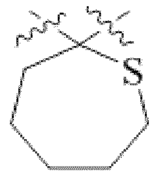

;

each Q2 is independently selected from a group consisting of halogen, hydroxyl, amino, nitro, cyano, carbonyl, methyl, ethyl, propyl, isopropyl, trifluoromethyl, methylamino, ethylamino, dimethylamino, diethylamino, methoxy, ethoxy and trifluoromethoxy.

In some embodiments, $R^1$ is selected from a group consisting of H, halogen, hydroxyl, amino, nitro, cyano, methyl, ethyl, propyl, isopropyl, monofluoromethyl, difluoromethyl, trifluoromethyl, hydroxymethyl, aminomethyl, methoxy, ethoxy, propoxy, isopropoxy, monofluoromethoxy, difluoromethoxy and trifluoromethoxy.

In some embodiments, $R^2$ and $R^3$ are respectively independently selected from a group consisting of H, halogen, hydroxyl, amino, nitro, cyano, methyl, ethyl, propyl, isopropyl, trifluoromethyl, trifluoroethyl, trifluoropropyl, hydroxymethyl, hydroxyethyl, hydroxypropyl, aminomethyl, aminoethyl, aminopropyl, methoxy, ethoxy, propoxy, and cyclobutyl, cyclopentyl, cyclohexyl, tetrahydrofuran, tetrahydrothienyl, tetrahydropyrrolidinyl, tetrahydropyrazolyl, tetrahydroimidazolidinyl, tetrahydropyranyl, tetrahydrothiopyranyl, piperidinyl, piperazinyl, hexahydropyrimidinyl, or morpholinyl, which could be optionally substituted by 1-2 Q1; and each Q1 is independently selected from a group consisting of halogen, hydroxyl, amino, nitro, cyano, methyl, ethyl, propyl, isopropyl, trifluoromethyl, methoxy, ethoxy and trifluoromethoxy.

In some embodiments, each $R^4$, each $R^5$, and each $R^6$ are respectively independently selected from a group consisting of H, halogen, hydroxyl, amino, nitro, cyano, methyl, ethyl, propyl, isopropyl, trifluoromethyl, methoxy, ethoxy and trifluoromethoxy.

In some embodiments, $R^5$ is selected from a group consisting of H, halogen, hydroxyl, amino, nitro, cyano, methyl, ethyl, propyl, isopropyl, trifluoromethyl, methoxy, ethoxy and trifluoromethoxy.

In some embodiments, $R^6$ is selected from a group consisting of H, halogen, hydroxyl, amino, nitro, cyano, methyl, ethyl, propyl, isopropyl, trifluoromethyl, methoxy, ethoxy and trifluoromethoxy.

In some embodiments, $R^7$ is selected from a group consisting of H, methyl, ethyl, propyl, isopropyl, trifluoromethyl, hydroxymethyl and aminomethyl.

In some embodiments, the compound of general formula (I), the pharmaceutically acceptable salt thereof, or the isomer thereof further has a structure of general formula (IIIa) or (IIIb),

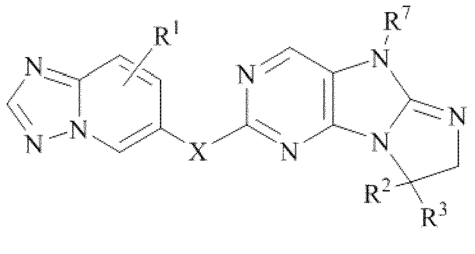

(IIIa),

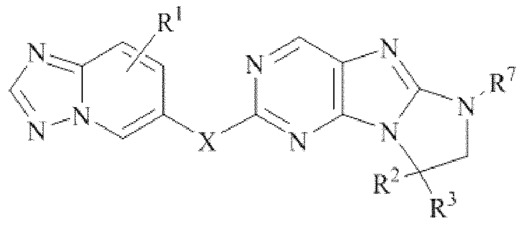

(IIIb).

Wherein, $R^1$, $R^2$, $R^3$, $R^7$, Q1 and Q2 are defined as in any one of the embodiments.

In some embodiments, the compound of general formula (I), the pharmaceutically acceptable salt thereof, or the isomer thereof further has a structure of general formula (IIIa),

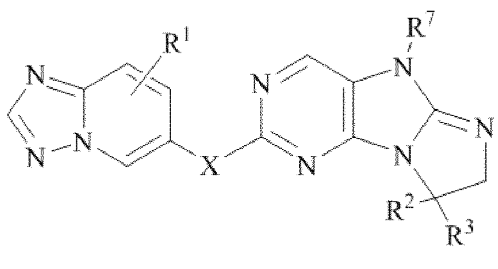

(IIIa).

$R^1$ is selected from a group consisting of H, halogen, hydroxyl, amino, nitro, cyano, $C_{1-6}$ alkyl, halo $C_{1-6}$ alkyl, hydroxy $C_{1-6}$ alkyl, amino $C_{1-6}$ alkyl, $C_{1-6}$ alkoxy, halo $C_{1-6}$ alkoxy, hydroxy $C_{1-6}$ alkoxy and amino $C_{1-6}$ alkoxy;

$R^2$, $R^3$ and the carbon atom linked to them together form 3-8 membered cycloalkyl or 3-8 membered heterocyclic group, which could be optionally substituted by 1-2 Q2; and each Q2 is independently selected from a group consisting of halogen, hydroxyl, amino, nitro, cyano, carbonyl, $C_{1-6}$ alkyl, halo $C_{1-6}$ alkyl, $C_{1-6}$ alkyl amino, $(C_{1-6}$ alkyl$)_2$ amino, $C_{1-6}$ alkoxy and halo $C_{1-6}$ alkoxy; and

$R^7$ is selected from a group consisting of H, $C_{1-6}$ alkyl, halo $C_{1-6}$ alkyl, hydroxy $C_{1-6}$ alkyl and amino $C_{1-6}$ alkyl.

In some embodiments, the compound of general formula (I), the pharmaceutically acceptable salt thereof, or the isomer thereof further has a structure of general formula (IIIa), wherein,

$R^1$ is selected from a group consisting of H, halogen, hydroxyl, amino, nitro, cyano, $C_{1-6}$ alkyl, halo $C_{1-6}$ alkyl, hydroxy $C_{1-6}$ alkyl, amino $C_{1-6}$ alkyl, $C_{1-6}$ alkoxy, halo $C_{1-6}$ alkoxy, hydroxy $C_{1-6}$ alkoxy and amino $C_{1-6}$ alkoxy;

$R^2$, $R^3$ and the carbon atom linked to them together form 5-7 membered cycloalkyl or 5-7 membered heterocyclic group , which could be optionally substituted by 1-2 Q2; and each Q2 is independently selected from a group consisting of halogen, hydroxyl, amino, nitro, cyano, carbonyl, $C_{1-6}$ alkyl, halo $C_{1-6}$ alkyl, $C_{1-6}$ alkyl amino, $(C_{1-6}$ alkyl$)_2$ amino, $C_{1-6}$ alkoxy and halo $C_{1-6}$ alkoxy; and

$R^7$ is selected from a group consisting of H, $C_{1-6}$ alkyl, halo $C_{1-6}$ alkyl, hydroxy $C_{1-6}$ alkyl and amino $C_{1-6}$ alkyl.

In some embodiments, the compound of general formula (I), the pharmaceutically acceptable salt thereof, or the isomer thereof further has a structure of general formula (IIIb),

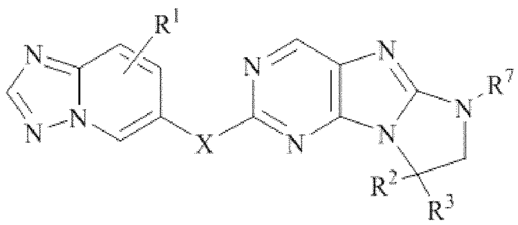
(IIIb)

$R^1$ is selected from a group consisting of H, halogen, hydroxyl, amino, nitro, cyano, $C_{1-6}$ alkyl, halo $C_{1-6}$ alkyl, hydroxy $C_{1-6}$ alkyl, amino $C_{1-6}$ alkyl, $C_{1-6}$ alkoxy, halo $C_{1-6}$ alkoxy, hydroxy $C_{1-6}$ alkoxy and amino $C_{1-6}$ alkoxy;

$R^2$, $R^3$ and the carbon atom linked to them together form 3-8 membered cycloalkyl or 3-8 membered heterocyclic group, which could be optionally substituted by 1-2 Q2; and each Q2 is independently selected from a group consisting of halogen, hydroxyl, amino, nitro, cyano, carbonyl, $C_{1-6}$ alkyl, halo $C_{1-6}$ alkyl, $C_{1-6}$ alkyl amino, $(C_{1-6}$ alkyl$)_2$ amino, $C_{1-6}$ alkoxy and halo $C_{1-6}$ alkoxy; and

$R^7$ is selected from a group consisting of H, $C_{1-6}$ alkyl, halo $C_{1-6}$ alkyl, hydroxy $C_{1-6}$ alkyl and amino $C_{1-6}$ alkyl.

In some embodiments, the compound of formula (I), the pharmaceutically acceptable salt thereof, or the isomer thereof further has a structure of general formula (IIIa), wherein,

$R^1$ is selected from a group consisting of H, halogen, hydroxyl, amino, nitro, cyano, $C_{1-6}$ alkyl, halo $C_{1-6}$ alkyl, hydroxy $C_{1-6}$ alkyl, amino $C_{1-6}$ alkyl, $C_{1-6}$ alkoxy, halo $C_{1-6}$ alkoxy, hydroxy $C_{1-6}$ alkoxy and amino $C_{1-6}$ alkoxy;

$R^2$, $R^3$ and the carbon atom linked to them together form 5-7 membered cycloalkyl or 5-7 membered heterocyclic group, which could be optionally substituted by 1-2 Q2; and each Q2 is independently selected from a group consisting of halogen, hydroxyl, amino, nitro, cyano, carbonyl, $C_{1-6}$ alkyl, halo $C_{1-6}$ alkyl, $C_{1-6}$ alkyl amino, $(C_{1-6}$ alkyl$)_2$ amino, $C_{1-6}$ alkoxy and halo $C_{1-6}$ alkoxy; and

$R^7$ is selected from a group consisting of H, $C_{1-6}$ alkyl, halo $C_{1-6}$ alkyl, hydroxy $C_{1-6}$ alkyl and amino $C_{1-6}$ alkyl.

In some of the above embodiments, in formula (IIIa) or (IIIb),

$R^2$, $R^3$ and the carbon atom linked to them together form 5-7 membered cycloalkyl or 5-7 membered heterocyclic group containing 1-2 heteroatoms, which could be optionally substituted by 1-2 Q2, the heteroatom is N, O, or S, and ring atoms of the cycloalkyl or heterocyclic group are oxygenated optionally; and

each Q2 is independently selected from a group consisting of halogen, hydroxyl, amino, nitro, cyano, carbonyl, $C_{1-6}$ alkyl, halo $C_{1-6}$ alkyl, $C_{1-6}$ alkyl amino, $(C_{1-6}$ alkyl$)_2$ amino, $C_{1-6}$ alkoxy and halo $C_{1-6}$ alkoxy.

In some of the above embodiments, in formula (IIIa) or (IIIb),

$R^2$, $R^3$ and the carbon atom linked to them together form the following groups substituted optionally by 1-2 Q2:

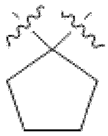 , 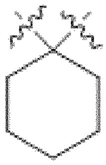 , 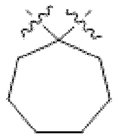 , 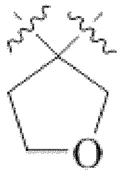 , 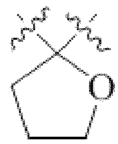 , 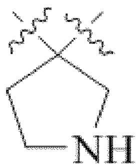 , 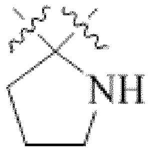 ,

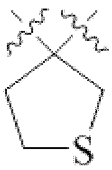 , 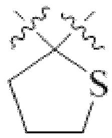 , 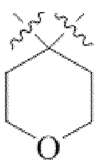 , 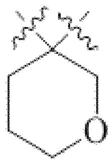 , 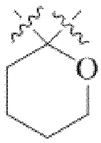 , 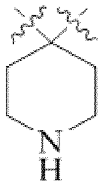 , 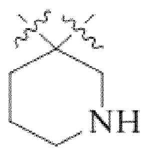 , 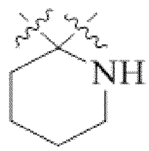 ,

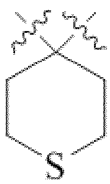 , 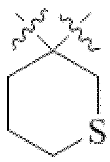 , 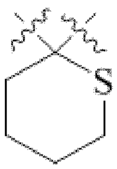 , 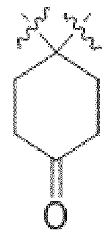 , 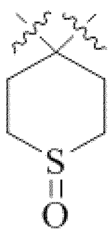 , 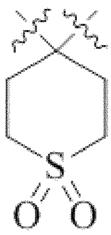 , 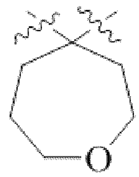 , 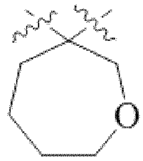 ,

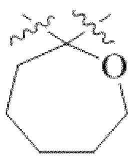 , 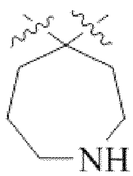 , 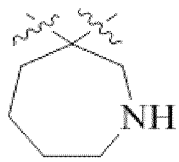 , 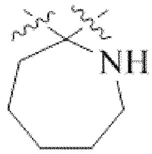 , 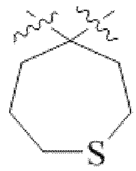 , 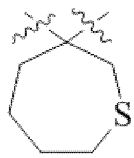 or 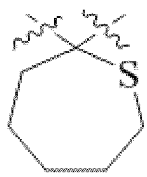 ;

each Q2 is independently selected from a group consisting of halogen, hydroxyl, amino, nitro, cyano, carbonyl, $C_{1-6}$ alkyl, halo $C_{1-6}$ alkyl, $C_{1-6}$ alkyl amino, $(C_{1-6}$ alkyl$)_2$ amino, $C_{1-6}$ alkoxy and halo $C_{1-6}$ alkoxy.

In some of the above embodiments, in formula (IIIa) or (IIIb),

$R^1$ is selected from a group consisting of H, halogen, hydroxyl, amino, nitro, cyano, $C_{1-6}$ alkyl, halo $C_{1-6}$ alkyl, hydroxy $C_{1-6}$ alkyl, amino $C_{1-6}$ alkyl, $C_{1-6}$ alkoxy, halo $C_{1-6}$ alkoxy, hydroxy $C_{1-6}$ alkoxy and amino $C_{1-6}$ alkoxy;
$R^2$, $R^3$ and the carbon atom linked to them together form the following groups substituted optionally by 1-2 Q2:

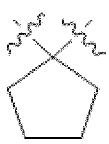 , 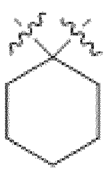 , 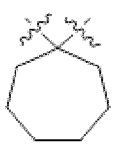 , 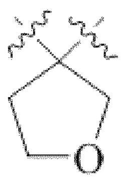 , 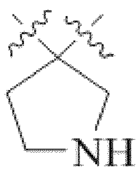 , 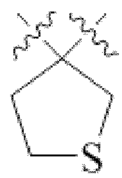 , 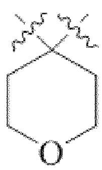 , 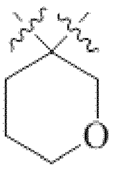 ,

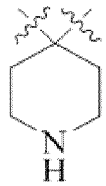, 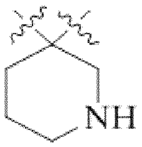, 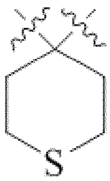, 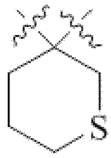, 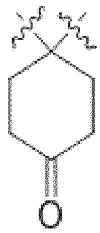, 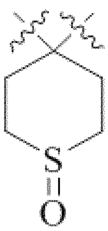, 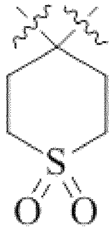, 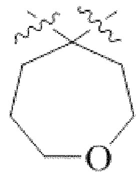,

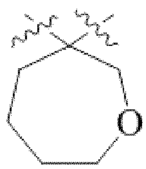, 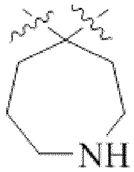, 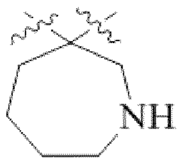, 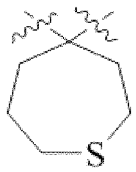 or 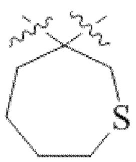;

each Q2 is independently selected from a group consisting of halogen, hydroxyl, amino, nitro, cyano, carbonyl, $C_{1-6}$ alkyl, halo $C_{1-6}$ alkyl, $C_{1-6}$ alkyl amino, $(C_{1-6}$ alkyl$)_2$ amino, $C_{1-6}$ alkoxy and halo $C_{1-6}$ alkoxy; and
$R^7$ is selected from a group consisting of H, $C_{1-6}$ alkyl, halo $C_{1-6}$ alkyl, hydroxy $C_{1-6}$ alkyl and amino $C_{1-6}$ alkyl.

In some of the above embodiments, in formula (IIIa) or (IIIb),

$R^1$ is selected from a group consisting of H, halogen, hydroxyl, amino, nitro, cyano, $C_{1-6}$ alkyl, halo $C_{1-6}$ alkyl, hydroxy $C_{1-6}$ alkyl, amino $C_{1-6}$ alkyl, $C_{1-6}$ alkoxy, halo $C_{1-6}$ alkoxy, hydroxy $C_{1-6}$ alkoxy and amino $C_{1-6}$ alkoxy;
$R^2$, $R^3$ and the carbon atom linked to them together form the following groups substituted optionally by 1-2 Q2:

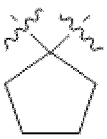, 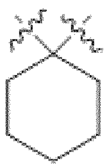, 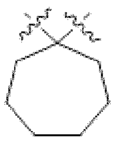, 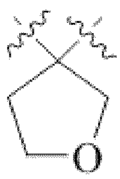, 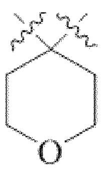, 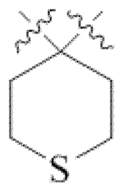, 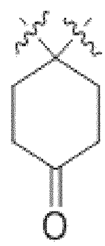, 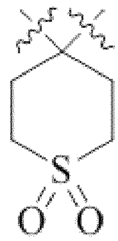

or

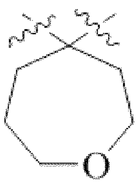;

each Q2 is independently selected from a group consisting of halogen, hydroxyl, amino, nitro, cyano, carbonyl, $C_{1-6}$ alkyl, halo $C_{1-6}$ alkyl, $C_{1-6}$ alkyl amino, $(C_{1-6}$ alkyl$)_2$ amino, $C_{1-6}$ alkoxy and halo $C_{1-6}$ alkoxy; and
$R^7$ is selected from a group consisting of H, $C_{1-6}$ alkyl, halo $C_{1-6}$ alkyl, hydroxy $C_{1-6}$ alkyl and amino $C_{1-6}$ alkyl.

In some embodiments, $R^1$ is selected from a group consisting of H, halogen, hydroxyl, amino, nitro, cyano, methyl, ethyl, propyl, isopropyl, monofluoromethyl, difluoromethyl, trifluoromethyl, hydroxymethyl, aminomethyl, methoxy, ethoxy, propoxy, isopropoxy, monofluoromethoxy, difluoromethoxy and trifluoromethoxy;

$R^2$ and $R^3$ are respectively independently selected from a group consisting of H, halogen, hydroxyl, amino, nitro, cyano, methyl, ethyl, propyl, isopropyl, trifluoromethyl, trifluoroethyl, trifluoropropyl, hydroxymethyl, hydroxyethyl, hydroxypropyl, aminomethyl, aminoethyl, aminopropyl, methoxy, ethoxy and propoxy, and cyclobutyl, cyclopentyl,

cyclohexyl, tetrahydrofuran, tetrahydrothienyl, tetrahydropyrrolidinyl, tetrahydropyrazolyl, tetrahydroimidazolidinyl, tetrahydropyranyl, tetrahydrothiopyranyl, piperidinyl, piperazinyl, hexahydropyrimidinyl or morpholinyl, which could be optionally substituted by 1-2 Q1; and each Q1 is independently selected from a group consisting of halogen, hydroxyl, amino, nitro, cyano, methyl, ethyl, propyl, isopropyl, trifluoromethyl, methoxy, ethoxy and trifluoromethoxy; or $R^2$, $R^3$ and the carbon atom linked to them together form the following groups substituted optionally by 1-2 Q2:

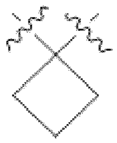, 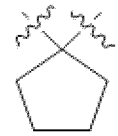, 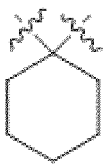, 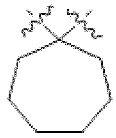, 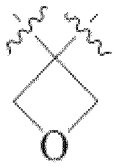, 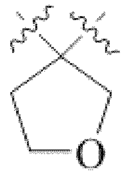, 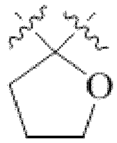,

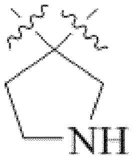, 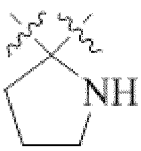, 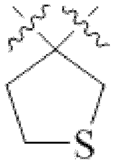, 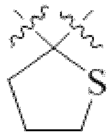, 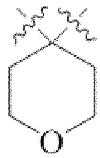, 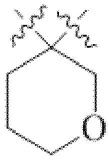, 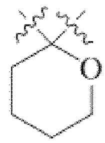, 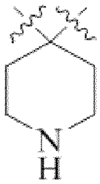,

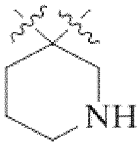, 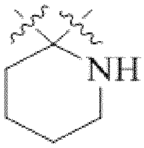, 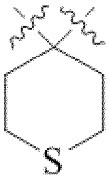, 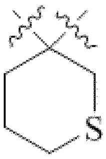, 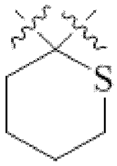, 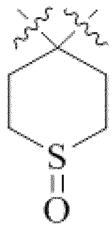, 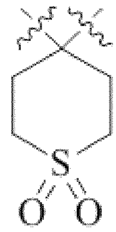, 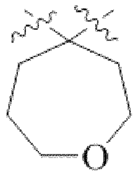,

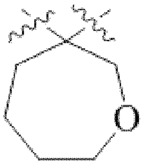, 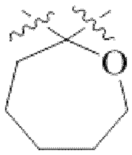, 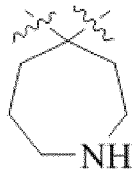, 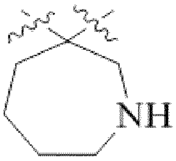, 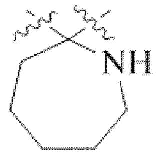, 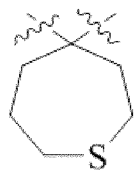, 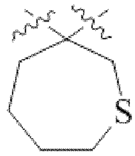

or

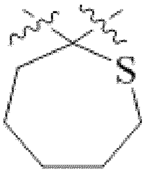;

each Q2 is independently selected from a group consisting of halogen, hydroxyl, amino, nitro, cyano, carbonyl, methyl, ethyl, propyl, isopropyl, trifluoromethyl, methylamino, ethylamino, dimethylamino, diethylamino, methoxy, ethoxy and trifluoromethoxy;

$R^4$ is H; $R^5$ and $R^6$ are respectively independently selected from a group consisting of H, halogen, hydroxyl, amino, nitro, cyano, methyl, ethyl, propyl, isopropyl, trifluoromethyl, methoxy, ethoxy and trifluoromethoxy; and

$R^7$ is selected from a group consisting of H, methyl, ethyl, propyl, isopropyl, trifluoromethyl, hydroxymethyl and aminomethyl.

In some embodiments, $R^1$ is selected from a group consisting of H, halogen, hydroxyl, amino, nitro, cyano, methyl, ethyl, propyl, isopropyl, monofluoromethyl, difluoromethyl, trifluoromethyl, hydroxymethyl, aminomethyl, methoxy, ethoxy, propoxy, isopropoxy, monofluoromethoxy, difluoromethoxy and trifluoromethoxy;

$R^2$, $R^3$ and the carbon atom linked to them together form the following groups substituted optionally by 1-2 Q2:

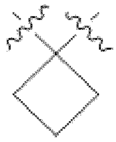 , 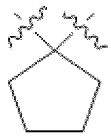 , 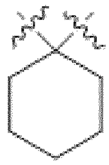 , 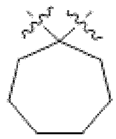 , 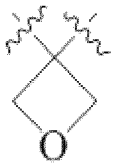 , 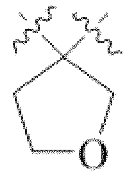 , 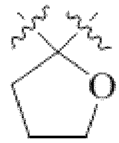 ,

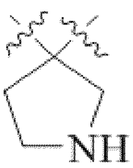 , 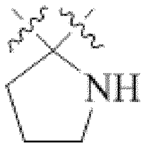 , 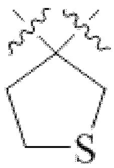 , 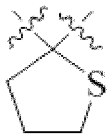 , 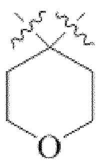 , 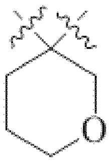 , 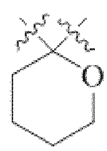 , 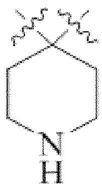 ,

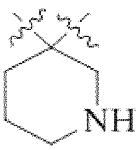 , 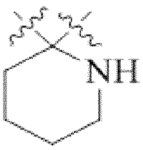 , 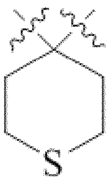 , 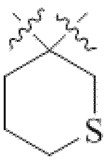 , 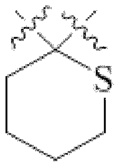 , 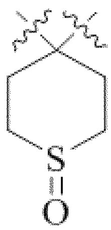 , 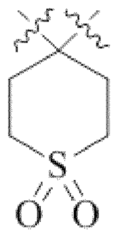 , 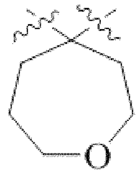 ,

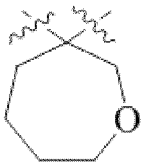 , 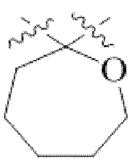 , 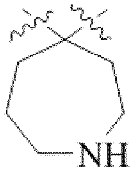 , 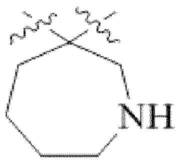 , 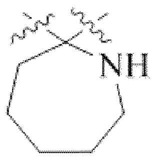 , 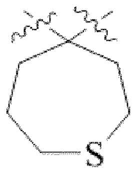 , 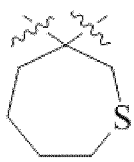

or

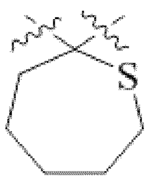 ;

each Q2 is independently selected from a group consisting of halogen, hydroxyl, amino, nitro, cyano, carbonyl, methyl, ethyl, propyl, isopropyl, trifluoromethyl, methylamino, ethylamino, dimethylamino, diethylamino, methoxy, ethoxy and trifluoromethoxy;

$R^4$ is H; $R^5$ and $R^6$ are respectively independently selected from a group consisting of H, halogen, hydroxyl, amino, nitro, cyano, methyl, ethyl, propyl, isopropyl, trifluoromethyl, methoxy, ethoxy and trifluoromethoxy; and

$R^7$ is selected from a group consisting of H, methyl, ethyl, propyl, isopropyl, trifluoromethyl, hydroxymethyl and aminomethyl.

In some embodiments, the compound of formula (I), the pharmaceutically acceptable salt thereof, or the isomer thereof further has a structure of formula (II),

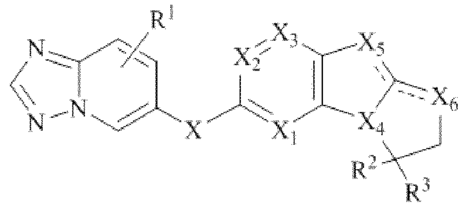
(II),

Wherein, $R^1$, $R^2$, $R^3$, $R^4$, $R^5$, $R^6$, $R^7$, X, $X_1$, $X_2$, $X_3$, $X_4$, $X_5$, $X_6$, Q1, and Q2 are defined as in any one of the above embodiments.

In some embodiments, the compound of formula (I), the pharmaceutically acceptable salt thereof, or the isomer thereof further has a structure of formula (Ia) or (Ib),

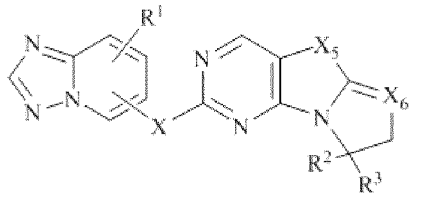
(Ia),

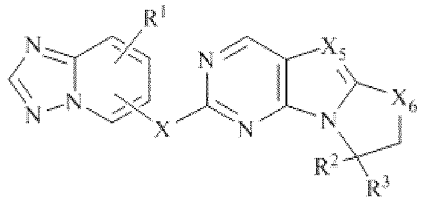
(Ib).

Wherein, $R^1$, $R^2$, $R^3$, $R^5$, $R^6$, $R^7$, X, $X_5$, $X_6$, Q1, and Q2 are defined as in any one of the above embodiments.

In some embodiments, the compound of formula (I), the pharmaceutically acceptable salt thereof, or the isomer thereof further has a structure of formula (Ic) or (Id),

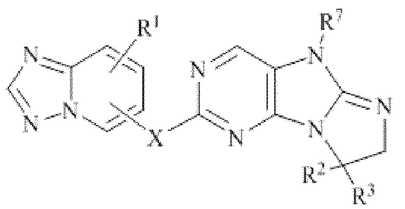
(Ic),

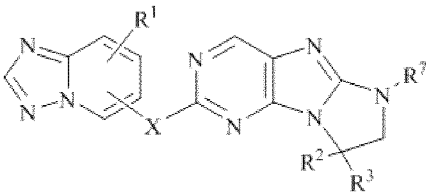
(Id).

Wherein, X, $R^1$, $R^2$, $R^3$, $R^7$, Q1, and Q2 are defined as in any one of the above embodiments.

The various technical solutions in the present disclosure may be combined mutually to form new technical solutions, and the new technical solutions formed are also included within the scope of the present disclosure.

In some embodiments, the aforementioned compound of formula (I), the pharmaceutically acceptable salt thereof, or the isomer thereof is selected from the following compounds:

| number | structure | number | structure |
|---|---|---|---|
| compound 1 | 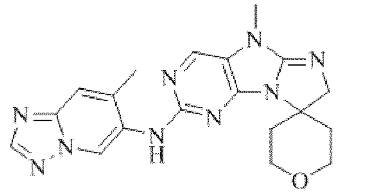 | compound 2 | 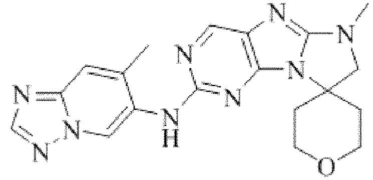 |
| compound 3 | 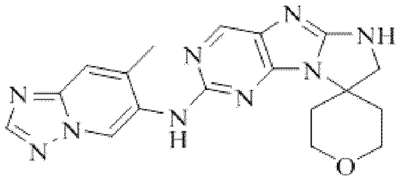 | compound 4 | 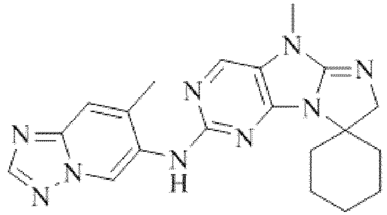 |
| compound 5 | 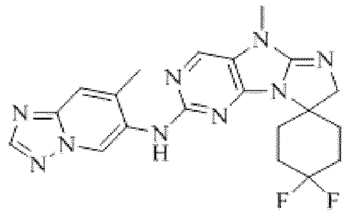 | compound 6 | 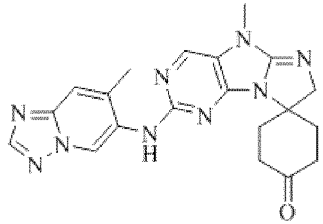 |
| compound 7 | 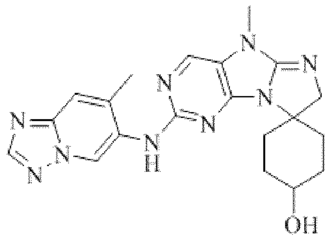 | compound 7-1 | 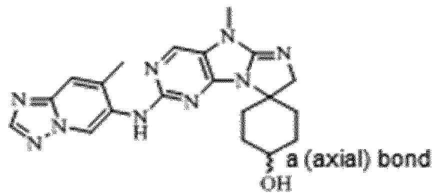 |

(continued)

| number | structure | number | structure |
|---|---|---|---|
| compound 7-2 | 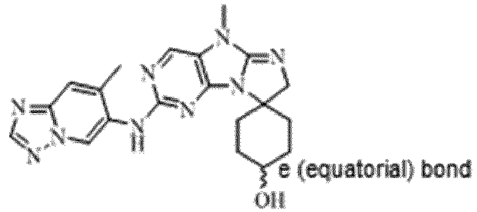 | compound 8 | 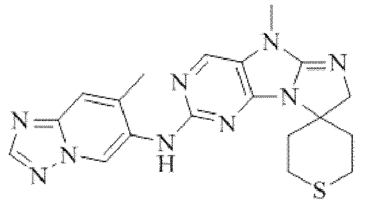 |
| compound 9 | 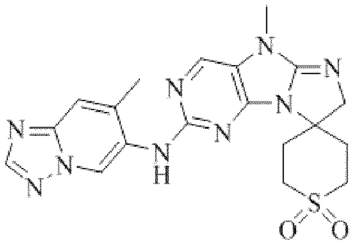 | compound 10 | 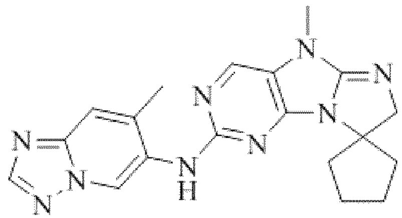 |
| compound 11 | 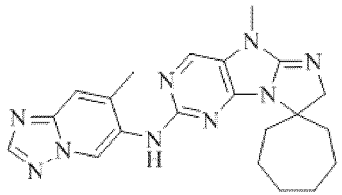 | compound 12 | 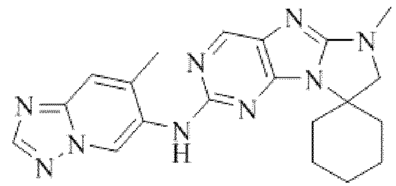 |
| compound 13 | 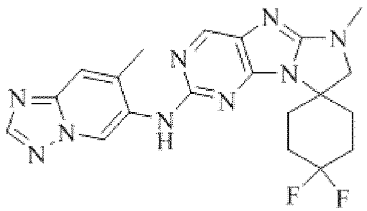 | compound 14 | 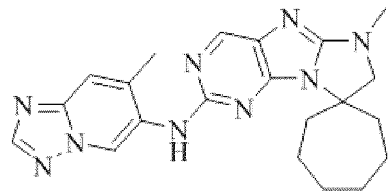 |

(continued)

| number | structure | number | structure |
|---|---|---|---|
| compound 15 | 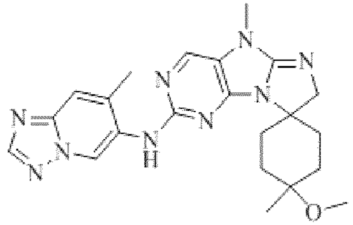 | compound 15-1 | 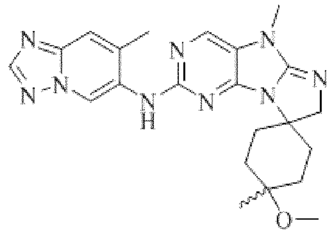 |
| compound 16 | 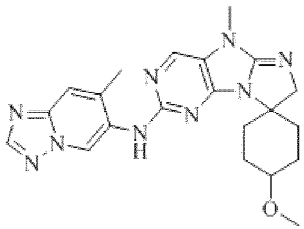 | compound 16-1 | 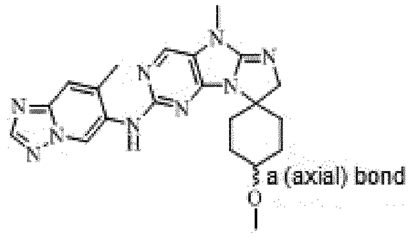 |
| compound 16-2 | 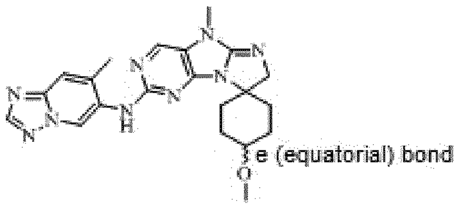 | compound 17 | 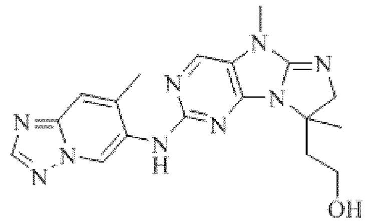 |
| compound 18 | 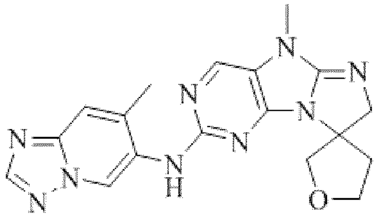 | compound 19 | 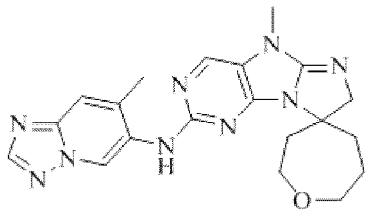 |

(continued)

| number | structure | number | structure |
|---|---|---|---|
| compound 19-1 | 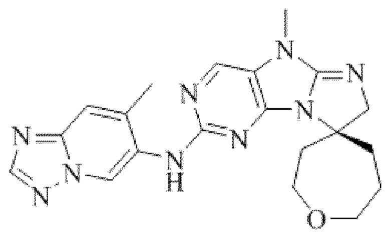 | compound 19-2 | 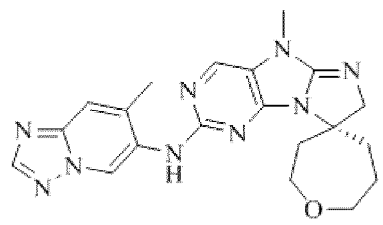 |
| compound 20 | 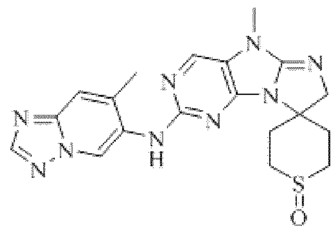 | | |

In another aspect, the present disclosure further provides an intermediate of formula (V),

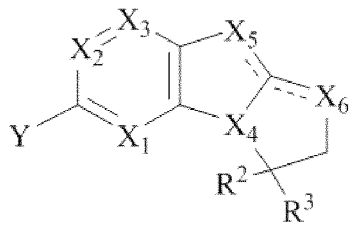
(V)

Wherein, $X_1$, $X_2$, $X_3$, $X_4$, $X_5$, $X_6$, $R^1$, $R^2$, $R^3$, $R^4$, $R^5$, $R^6$, $R^7$, Q1, Q2, and virtual bond --- are defined as in any one of the above embodiments; and Y is halogen, amino, hydroxyl, or sulfydryl.

In some embodiments, the intermediate of formula (V) further has a structure of formula (Va) or (Vb),

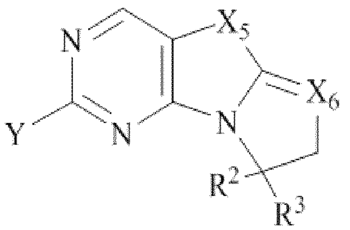
(Va),
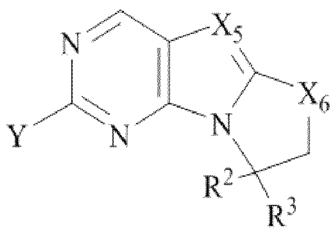
(Vb).

Wherein, $R^2$, $R^3$, $R^5$, $R^6$, $R^7$, $X_5$, $X_6$, Q1, and Q2 are defined as in any one of the above embodiments; and Y is halogen, amino, hydroxyl, or sulfydryl.

In some embodiments, the intermediate of formula (V) further has a structure of formula (Vc) or (Vd),

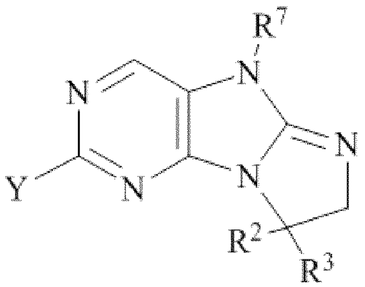
(Vc),
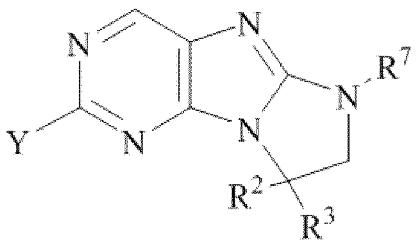
(Vd).

Wherein, $R^2$, $R^3$, $R^7$, Q1, and Q2 are defined in any one of the above embodiments; and Y is halogen, amino, hydroxyl, or sulfydryl.

In another aspect, the present disclosure further provides a pharmaceutical formulation containing the aforementioned compound of formula (I), formula (Ia), formula (Ib), formula (Ic), formula (Id), formula (II), formula (IIa), formula (IIb), formula (IIIa) or formula (IIIb), the pharmaceutically acceptable salt thereof or the isomer thereof, and one or more pharmaceutically acceptable excipients, and the pharmaceutical formulation may be any one pharmaceutically acceptable dosage form. The pharmaceutically acceptable excipient is a substance that is non-toxic, compatible with active ingredients, and otherwise biologically suitable for living organisms. The selection of specific excipients may depend on the administration mode used to treat specific patients or disease type and status.

In some embodiments, the above pharmaceutical formulation may be administered orally, parenterally, rectally, or pulmonically to a patient or a subject in need of such treatment. While used for oral administration, the pharmaceutical composition may be made into an oral formulation, for example, it may be made into a conventional oral solid formulation, such as tablets, capsules, pills, granules and the like; and it may also be made into an oral liquid formulation, such as oral solution, oral suspension, syrup and the like. While used for parenteral administration, the above pharmaceutical formulation may also be made into an injection, including injection solution, sterile powder for injection, and concentrated solution for injection. While used for rectal administration, the pharmaceutical composition may be made into a suppository and the like. While used for pulmonical administration, the pharmaceutical composition may be made into an inhalation preparation, an aerosol, a powder aerosol or a spray.

In another aspect, the present disclosure further relates to a use of the aforementioned compound of formula

(I), formula (Ia), formula (Ib), formula (Ic), formula (Id), formula (II), formula (IIa), formula (IIb), formula (IIIa) or formula (IIIb), the pharmaceutically acceptable salt thereof or the isomer thereof in preparation of a drug for preventing and/or treating a benign tumor or cancer and other diseases, and the cancer includes carcinoma in situ and metastatic cancer.

Further, the present disclosure further relates to a use of the pharmaceutical formulation containing the aforementioned compound of formula (I), formula (Ia), formula (Ib), formula (Ic), formula (Id), formula (II), formula (IIa), formula (IIb), formula (IIIa) or formula (IIIb), the pharmaceutically acceptable salt thereof or the isomer thereof in preparation of a drug for preventing and/or treating a benign tumor or cancer and other diseases, and the cancer includes carcinoma in situ and metastatic cancer.

In another aspect, the present disclosure further relates to a use of the aforementioned compound of formula (I), formula (Ia), formula (Ib), formula (Ic), formula (Id), formula (II), formula (IIa), formula (IIb), formula (IIIa) or formula (IIIb), the pharmaceutically acceptable salt thereof or the isomer thereof in preparation of a drug for preventing and/or treating a benign tumor or cancer and other diseases, the drug is used in combination with radiotherapy and/or one or more anti-cancer agents, and the cancer includes carcinoma in situ and metastatic cancer.

Further, the present disclosure further relates to a use of the pharmaceutical formulation containing the aforementioned compound of formula (I), formula (Ia), formula (Ib), formula (Ic), formula (Id), formula (II), formula (IIa), formula (IIb), formula (IIIa) or formula (IIIb), the pharmaceutically acceptable salt thereof or the isomer thereof in preparation of a drug for preventing and/or treating a benign tumor or cancer and other diseases, the drug is used in combination with radiotherapy and/or one or more anti-cancer agents, and the cancer includes carcinoma in situ and metastatic cancer.

In another aspect, the present disclosure further relates to a use of the aforementioned compound of formula (I), formula (Ia), formula (Ib), formula (Ic), formula (Id), formula (II), formula (IIa), formula (IIb), formula (IIIa) or formula (IIIb), the pharmaceutically acceptable salt thereof or the isomer thereof in preparation of a drug for making cancer cells sensitive to anticancer agents and/or radiotherapy.

Further, the present disclosure further relates to a use of the pharmaceutical formulation containing the aforementioned compound of formula (I), formula (Ia), formula (Ib), formula (Ic), formula (Id), formula (II), formula (IIa), formula (IIb), formula (IIIa) or formula (IIIb), the pharmaceutically acceptable salt thereof or the isomer thereof in preparation of a drug for making cancer cells sensitive to anticancer agents and/or radiotherapy.

In another aspect, the present disclosure further provides a pharmaceutical composition, which contains the aforementioned compound of formula (I), formula (Ia), formula (Ib), formula (Ic), formula (Id), formula (II), formula (IIa), formula (IIb), formula (IIIa) or formula (IIIb), the pharmaceutically acceptable salt thereof or the isomer thereof, and one or more second therapeutic active agent. The second therapeutic active agent is anticancer agents, including a mitosis inhibitor, an alkylating agent, an antimetabolite, a DNA intercalate agent, an anti-tumor antibiotic, a growth factor inhibitor, a signal transduction inhibitor, a cell cycle inhibitor, an enzyme inhibitor, a vitamin A-like receptor regulator, a proteasome inhibitor, a topoisomerase inhibitor, a biological response regulator, a hormone drug, an angiogenesis inhibitor, a cell growth inhibitor, a targeted antibody, an HMG-CoA reductase inhibitor and an isoprene based protein transferase inhibitor.

In some embodiments, the second therapeutic active agent may be a drug that relieves or reduces one or more side effects of the compound of the present disclosure while used to treat the subject's disease, or a drug that enhances the efficacy of the compound of the present disclosure.

In some embodiments, the pharmaceutical composition further includes one or more pharmaceutically acceptable excipients, and the excipient is described as previously.

In another aspect, the present disclosure further relates to a use of the pharmaceutical composition containing the aforementioned compound of formula (I), formula (Ia), formula (Ib), formula (Ic), formula (Id), formula (II), formula (IIa), formula (IIb), formula (IIIa) or formula (IIIb), the pharmaceutically acceptable salt thereof or the isomer thereof in preparation of a drug for preventing and/or treating a benign tumor or cancer and other diseases, and the cancer includes carcinoma in situ and metastatic cancer.

In another aspect, the present disclosure further relates to a use of the pharmaceutical composition containing the aforementioned compound of formula (I), formula (Ia), formula (Ib), formula (Ic), formula (Id), formula (II), formula (IIa), formula (IIb), formula (IIIa) or formula (IIIb), the pharmaceutically acceptable salt thereof or the isomer thereof in preparation of a drug for preventing and/or treating a benign tumor or cancer and other diseases, the drug may be used in combination with radiotherapy and/or one or more anti-cancer agents, and the cancer includes carcinoma in situ and metastatic cancer.

Further, the present disclosure further relates to a use of the pharmaceutical composition containing the aforementioned compound of formula (I), formula (Ia), formula (Ib), formula (Ic), formula (Id), formula (II), formula (IIa), formula (IIb), formula (IIIa) or formula (IIIb), the pharmaceutically acceptable salt thereof or the isomer thereof in preparation of a drug for making cancer cells sensitive to anticancer agents and/or radiotherapy.

In another aspect, the present disclosure further provides a method for treating a disease related to DNAPK overactivation, and the method includes administering an effective amount of the aforementioned compound of formula (I), formula (Ia), formula (Ib), formula (Ic), formula (Id), formula (II), formula (IIa), formula (IIb), formula (IIIa) or formula (IIIb), the pharmaceutically acceptable salt thereof or the isomer thereof, the aforementioned pharmaceutical formulation

or pharmaceutical composition to a patient in need; and the disease related to the DNAPK overactivation is benign tumor or cancer, and the cancer include carcinoma in situ and metastatic cancer.

Further, the present disclosure further provides a method for treating a disease related to DNAPK overactivation, and the method includes administering an effective amount of the aforementioned compound of formula (I), formula (Ia), formula (Ib), formula (Ic), formula (Id), formula (II), formula (IIa), formula (IIb), formula (IIIa) or formula (IIIb), the pharmaceutically acceptable salt thereof or the isomer thereof, the aforementioned pharmaceutical formulation or pharmaceutical composition to a patient before/after radiotherapy; and the disease related to the DNAPK overactivation is benign tumor or cancer, and the cancer include carcinoma in situ and metastatic cancer.

Further, the present disclosure further provides a method for treating a disease related to DNAPK overactivation, and the method includes administering an effective amount of the aforementioned compound of formula (I), formula (Ia), formula (Ib), formula (Ic), formula (Id), formula (II), formula (IIa), formula (IIb), formula (IIIa) or formula (IIIb), the pharmaceutically acceptable salt thereof or the isomer thereof, the aforementioned pharmaceutical formulation or pharmaceutical composition to a patient before/after chemotherapy; and the disease related to the DNAPK overactivation is benign tumor or cancer, and the cancer include carcinoma in situ and metastatic cancer.

In another aspect, the present disclosure further provides a method for enhancing sensitivity of a patient to anticancer agent or radiotherapy, and the method includes administering an effective amount of the aforementioned compound of formula (I), formula (Ia), formula (Ib), formula (Ic), formula (Id), formula (II), formula (IIa), formula (IIb), formula (IIIa) or formula (IIIb), the pharmaceutically acceptable salt thereof or the isomer thereof, and the aforementioned pharmaceutical formulation or pharmaceutical composition to a patient in need; and the anticancer agent is described below.

Further, the present disclosure further provides a method for enhancing sensitivity of a patient to anticancer agent or radiotherapy, and the method includes administering an effective amount of the aforementioned compound of formula (I), formula (Ia), formula (Ib), formula (Ic), formula (Id), formula (II), formula (IIa), formula (IIb), formula (IIIa) or formula (IIIb), the pharmaceutically acceptable salt thereof or the isomer thereof, and the aforementioned pharmaceutical formulation or pharmaceutical composition to a patient before/after radiotherapy; and the anticancer agent is described below.

Further, the present disclosure further provides a method for enhancing sensitivity of a patient to anticancer agent or radiotherapy, and the method includes administering an effective amount of the aforementioned compound of formula (I), formula (Ia), formula (Ib), formula (Ic), formula (Id), formula (II), formula (IIa), formula (IIb), formula (IIIa) or formula (IIIb), the pharmaceutically acceptable salt thereof or the isomer thereof, and the aforementioned pharmaceutical formulation or pharmaceutical composition to a patient before/after chemotherapy; and the anticancer agent is described below.

In another aspect, the present disclosure further provides a kit, containing:

(a) an effective amount of one or more of the aforementioned compound of formula (I), formula (Ia), formula (Ib), formula (Ic), formula (Id), formula (II), formula (IIa), formula (IIb), formula (IIIa) or formula (IIIb), the pharmaceutically acceptable salt thereof or the isomer thereof; and
(b) an effective amount of one or more of anticancer agents.

The "anticancer agent" in the present disclosure refers to an agent that has a certain therapeutic effect on tumors, including but not limited to a mitosis inhibitor, an alkylating agent, an antimetabolite, a DNA intercalate agent, an anti-tumor antibiotic, a growth factor inhibitor, a signal transduction inhibitor, a cell cycle inhibitor, an enzyme inhibitor, a vitamin A-like receptor regulator, a proteasome inhibitor, a topoisomerase inhibitor, a biological response regulator, a hormone drug, an angiogenesis inhibitor, a cell growth inhibitor, a targeted antibody, an HMG-CoA reductase inhibitor, an isoprene based protein transferase inhibitor and the like; and the tumor includes benign tumor and cancer. The "effective amount" refers to an amount of a drug that may prevent, alleviate, delay, inhibit, or cure the subject's disease. The size of administration dose is related to the drug administration mode, pharmacokinetics, severity of the disease, and individual physical signs (gender, weight, height, and age) of the subject.

In the present disclosure, unless otherwise stated, scientific and technical terms used herein have the meanings commonly understood by those skilled in the art. However, in order to better understand the present disclosure, the definitions of some terms are provided below. While the definition and explanation of the terms provided by the present disclosure do not conform to the meanings commonly understood by those skilled in the art, the definition and explanation of the terms provided by the present disclosure shall prevail.

The "halogen" in the present disclosure refers to a fluorine atom, a chlorine atom, a bromine atom, or an iodine atom.

The "$C_{1-6}$ alkyl" in the present disclosure refers to a straight chain or branched chain alkyl containing 1-6 carbon atoms, including, for example, "$C_{1-4}$ alkyl", "$C_{1-3}$ alkyl", "$C_{1-2}$ alkyl", "$C_{2-6}$ alkyl", "$C_{2-5}$ alkyl", "$C_{2-4}$ alkyl", "$C_{2-3}$ alkyl", "$C_{3-6}$ alkyl", "$C_{3-5}$ alkyl", "$C_{3-4}$ alkyl" and the like. Specific examples include but not limited to: methyl, ethyl, n-propyl,

isopropyl, n-butyl, isobutyl, sec-butyl, tert-butyl, n-pentyl, isopentyl, 2-methylbutyl, neopentyl, 1-ethylpropyl, n-hexyl, isohexyl, 3-methylpentyl, 2-methylpentyl, 1-methylpentyl, 3,3-dimethylbutyl, 2,2-dimethylbutyl, 1,1-dimethylbutyl, 1,2-dimethylbutyl, 1,3-dimethylbutyl, 2,3-dimethylbutyl, 2-ethylbutyl, 1,2-dimethylpropyl and the like. The "$C_{1-4}$ alkyl" in the present disclosure refers to a specific example of the $C_{1-6}$ alkyl containing 1-4 carbon atoms.

The "$C_{1-6}$ alkoxy" in the present disclosure refers to "$C_{1-6}$ alkyl-O-", and the "$C_{1-6}$ alkyl" is defined as previously. The "$C_{1-4}$ alkoxy" in the present disclosure refers to "$C_{1-4}$ alkyl-O-", and the "$C_{1-4}$ alkyl" is defined as previously.

The "$C_{1-6}$ alkylthio" in the present disclosure refers to "$C_{1-6}$ alkyl-S-", and the "$C_{1-6}$ alkyl" is defined as previously. The "$C_{1-4}$ alkylthio" in the present disclosure refers to "$C_{1-4}$ alkyl-S-", and the "$C_{1-4}$ alkyl" is defined as previously.

The "hydroxy $C_{1-6}$ alkyl, amino $C_{1-6}$ alkyl, and halo $C_{1-6}$ alkyl" in the present disclosure refer to one or more hydrogen atoms in the $C_{1-6}$ alkyl being respectively substituted by one or more hydroxyls, aminos, or halogens. The $C_{1-6}$ alkyl is defined as previously.

The "hydroxy $C_{1-6}$ alkoxy, amino $C_{1-6}$ alkoxy, and halo $C_{1-6}$ alkoxy" in the present disclosure refer to one or more hydrogen atoms in the "$C_{1-6}$ alkoxy" being respectively substituted by one or more hydroxyls, aminos, or halogens.

The "hydroxy $C_{1-6}$ alkylthio, amino $C_{1-6}$ alkylthio, and halo $C_{1-6}$ alkylthio" in the present disclosure refer to one or more hydrogen atoms in the "$C_{1-6}$ alkylthio" being respectively substituted by one or more hydroxyls, aminos, or halogens.

The "$C_{1-6}$ alkyl amino and di ($C_{1-6}$ alkyl) amino" in the present disclosure refer to $C_{1-6}$ alkyl-NH-, and

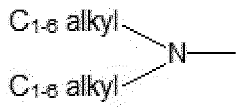

respectively.

The "3-8 membered cycloalkyl" in the present disclosure refers to a saturated or partially saturated monocyclic cyclic group with 3-8 ring atoms and without aromaticity. The "3-8 membered cycloalkyl" in the present disclosure includes "3-8 membered saturated cycloalkyl" and "3-8 membered partially saturated cycloalkyl", such as "3-6 membered cycloalkyl", "3-6 membered saturated cycloalkyl", "5-7 membered cycloalkyl", "5-7 membered saturated cycloalkyl", "5-6-membered cycloalkyl" "5-6 membered saturated cycloalkyl" and the like. Its examples include but not limited to: cyclopropyl, cyclobutyl, cyclopentyl, cyclohexyl, cycloheptyl or cyclohexenyl and the like.

The "3-8 membered heterocyclic group" in the present disclosure refers to a saturated or partially saturated monocyclic cyclic group without aromaticity that contains at least one (for example, one, two, three, four or five) heteroatom and has 3-8 ring atoms, the heteroatom is a nitrogen atom, an oxygen atom, and/or a sulfur atom, and optionally, the ring atom (such as a carbon atom, a nitrogen atom, or a sulfur atom) in the ring structure may be oxygenated. The "3-8 membered heterocyclic group" in the present disclosure includes "3-8 membered saturated heterocyclic group" and "3-8 membered partially saturated heterocyclic group". The "3-8 membered heterocyclic group" is, for example, "3-6 membered heterocyclic group", "3-6 membered saturated heterocyclic group", "3-7 membered heterocyclic group", "3-7 membered saturated heterocyclic group", "5-7 membered heterocyclic group", "5-7 membered saturated heterocyclic group", "5-6 membered heterocyclic group", "5-6 membered saturated heterocyclic group" and the like. Its specific examples include but not limited to: azacyclopropyl, 2H-azacyclopropyl, diazacyclopropyl, 3H-diazacyclopropenyl, azacyclobutyl, oxacyclopropyl, oxacyclobutyl, 1,4-dioxacyclohexyl, 1,3-dioxacyclohexyl, 1,3-dioxacyclopentyl, 1,4-dioxacyclohexdienyl, tetrahydrofuranyl, dihydropyrrolyl, tetrahydropyrrolidinyl, tetrahydropyrazolidinyl, tetrahydroimidazolidinyl, 4,5-dihydroimidazolyl, pyrazolyl, 4,5-dihydropyrazolyl, 2,5-dihydrothiophenyl, tetrahydrothienyl, 4,5-dihydrothiazolyl, thiazolidinyl, tetrahydropyranyl, tetrahydrothiopyranyl, 1,1-dioxytetrahydrothiopyranyl, piperidinyl, tetrahydropyridinyl, piperidinone, tetrahydropyridinone, dihydropiperidone, piperazinyl, hexahydropyrimidinyl, morpholinyl and the like.

The "C ($R^4$) ", "CH ($R^5$) ", "C ($R^6$) ", and "N ($R^7$)" in the present disclosure respectively refer to the following structures:

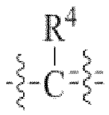, 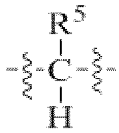, 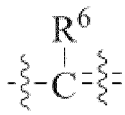, 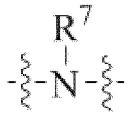.

While "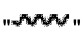" in the present disclosure appears in a group, for example,

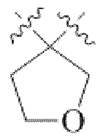,

"" indicates a linkage position between this group and the adjacent group; and while it appears in a position of a chemical bond in the chemical structure, especially in a position of 6 membered saturated ring substituent, for example,

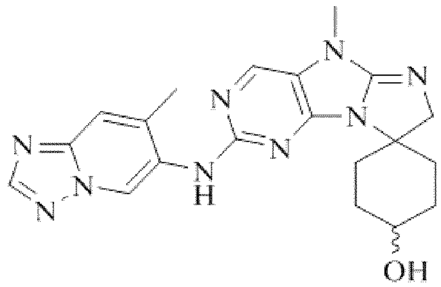,

"" represents a chemical bond, and is specifically singly located on a bond a (axial bond) or a bond e (equatorial bond) of a chair-type 6 membered saturated ring.

The "each $R^4$" in the present disclosure means that while at least two of $X_1$-$X_4$ are selected from "C ($R^4$) ", and each $R^4$ in a plurality of $R^4$ is independently selected from the groups described in the above technical schemes.

The "each $R^5$" in the present disclosure means that while $X^5$ and $X^6$ are both CH ($R^5$), each $R^5$ in a plurality of $R^5$ is independently selected from the groups described in the above technical schemes.

The "each $R^7$" in the present disclosure means that while $X^5$ and $X^6$ are both N ($R^7$), each $R^7$ in a plurality of $R^7$ is independently selected from the groups described in the above technical schemes.

The "optional substituent substitution" in the present disclosure refers to two situations that one or more hydrogen atoms on the substituted group are "substituted" by one or more substituents or "unsubstituted".

The "chemotherapy" in the present disclosure is an abbreviation of chemical drug therapy, and achieves the purpose of treatment mainly by using chemical therapy drugs to kill the cancer cells.

The "radiotherapy" in the present disclosure refers to a tumor treatment method, namely tumor radiotherapy, and mainly uses radiation for local tumor treatment. The "radiation" includes $\alpha$, $\beta$, $\gamma$ rays generated by radioactive isotopes and X-rays, electron beams, proton beams, and other particle beams generated by various X-ray therapy machines or accelerators.

The "pharmaceutically acceptable salt" in the present disclosure refers to a salt formed by an acidic functional group (such as -COOH, -OH, and $-SO_3H$) existing in the compound and an appropriate inorganic or organic cation (base), including a salt formed with alkali metal or alkaline earth metal, an ammonium salt, and a salt formed with a nitrogen-containing organic base; and a salt formed by a basic functional group (such as $-NH_2$) existing in the compound and an appropriate inorganic or organic anion (acid), including a salt formed with an inorganic acid or an organic acid (such as a carboxylic acid).

The "isomer" of the present disclosure means that the compound of the present disclosure contains one or more asymmetric centers, so it may be a raceme and racemic mixture, a single enantiomer, a diastereomer mixture, and a single diastereomer. The compound of the present disclosure may have the asymmetric centers, and such asymmetric centers each independently generate two optical isomers. The scope of the present disclosure includes all possible optical isomers and their mixtures. If the compound in the present disclosure contains an olefin double bond, unless otherwise specified, it includes a cis-isomer and a trans-isomer. The compound of the present disclosure may exist in the form of tautomer (one of functional group isomers), and it has different hydrogen linkage points by one or more double bond shifts, for example, ketone and its enol form are keto-enol tautomers. The compound of the present disclosure contains a spiral ring structure, and substituents on the ring may exist on both sides of the ring due to the influence of the three-dimensional spatial structure of the ring, as to form relative cis and trans-isomers. Each tautomer and the mixture thereof are all included in the scope of the present disclosure. The enantiomer, diastereomer, raceme, mesomer, cis-trans isomer, tautomer, geometric isomer, epimer and the mixtures thereof and the like of all compounds are included in the scope of the present disclosure.

The compound of the present disclosure may be prepared in the form of individual enantiomer by enantiospecific

synthesis or resolution from the enantiomer mixture. Conventional resolution technologies include the use of various well-known chromatographic methods to split the enantiomer mixture of a starting substance or a final product.

While the stereochemistry of the compound disclosed is named or described by the structure, the named or described stereisomer is at least 60%, 70%, 80%, 90%, 99% or 99.9% pure by weight relative to other stereisomers. While the single isomer is named or described by the structure, the described or named enantiomer is at least 60%, 70%, 80%, 90%, 99%, or 99.9% pure by weight. The weight% of optical purity is a ratio of the weight of enantiomer to the weight of enantiomer plus its optical isomers.

Beneficial Effects of the Invention

1. The compound of the present disclosure, the pharmaceutically acceptable salt thereof, or the isomer thereof has the excellent DNA-PK inhibitory effects, and it has the good pharmacokinetic properties in the living organisms (such as a mouse, a rat, a dog, a monkey, and a human), the effect is long-lasting, and the bioavailability is high. It may enhance the sensitivity of the cancer cells (such as a lung cancer cell A549, a liver cancer cell huh-7, breast cancer cells MDA-MB-231 and MCF-7, and an ovarian cancer cell SKOV3) to radiotherapy and/or one or more anticancer agents (such as a chemotherapy drug).

2. The compound of the present disclosure, the pharmaceutically acceptable salt thereof or the isomer thereof has the better therapeutic effect on the benign tumor and cancer, and the stability of liver microsomes in a plurality of species (such as a human source, a mouse source, a monkey source and a dog source) is high.

3. The preparation process of compound in the present disclosure is simple, the drug purity is high, the quality is stable, and it is easy to perform large-scale industrial production.

4. The compound of the present disclosure has the significant anti-tumor effects in vivo (such as a CDX model in a nude mouse: an NCI-H1048 model and an MDA-MB-231 model), and may significantly improve the tumor inhibition rate of radiotherapy and/or one or more anticancer agents (such as the chemotherapy drug).

## Detailed Description of the Embodiments

Technical solutions of the present disclosure are described below in combination with specific embodiments, and the above content of the present disclosure is further described in detail. However, this should not be understood as limiting the scope of the above subject matter of the present disclosure to the following embodiments. All technologies achieved based on the above content of the present disclosure belong to the scope of the present disclosure.

Abbreviation:

BrettPhos Pd G3: methanesulfonic acid (2-dicyclohexylphosphonyl-3,6-dimethoxy-2',4',6'-triisopropyl-1,1'-biphenyl) (2-amino-1,1'-biphenyl-2-yl) palladium (II); DMA: N,N-dimethylacetamide; DCM: dichloromethane; MeOH: methanol; PE: petroleum ether; EA: ethyl acetate; THF: tetrahydrofuran; DIEA: N,N-diisopropylethylamine; S-CDI: N,N'-thiocarbonyldiimidazole; DMAP: 4-dimethylaminopyridine.

### Preparation example 1: Preparation of 2-chloro-2',3',5',6,6',7-hexahydrospiro[imidazo[1,2-e]purine-8,4'-pyran]

### 1. Preparation of 4-((2-chloro-5-nitropyrimidin-4-yl) amino) tetrahydro-2H-pyran-4-carbonitrile

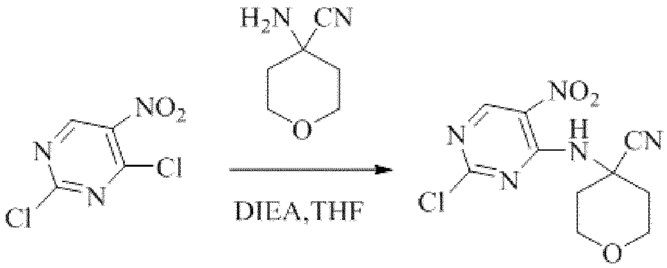


2,4-dichloro-5-nitropyrimidine (7.76 g, 40.0 mmol) and DIEA (10.4 g, 80.5 mmol) were added to THF (150 mL), 4-aminotetrahydro-2H-pyran-4-nitrile (5.05 g, 40.0 mmol) was added at -20°C, and then it was reacted for 2 hours at 25 °C. The system was cooled to 20 °C, concentrated and purified by a silica gel column chromatography (PE: EA=2:1), to obtain a product (10.2 g, yield: 89.9%).

**2. Preparation of 4-((5-amino-2-chloropyrimidin-4-yl) amino) tetrahydro-2H-pyran-4-carbonitrile**

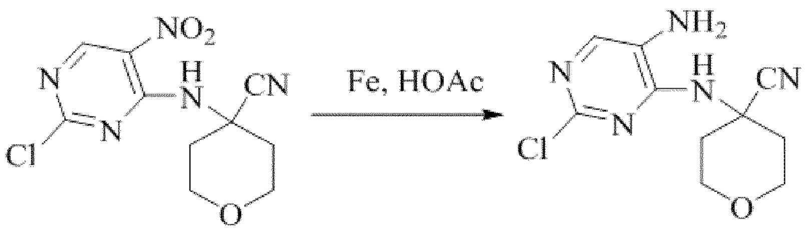


4-((2-chloro-5-nitropyrimidin-4-yl)amino)tetrahydro-2H-pyran-4-carbonitrile (9.8 g, 34.5 mmol) and iron powder (7.7 g, 137.5 mmol) were added to a mixture of HOAc (20 mL), ethanol (20 mL) and water (20 mL), and then it was reacted at 70°C for 1 hour. The system was cooled to 25 °C, and suction-filtered under a reduced pressure, a filter cake was washed with (DCM:MeOH=10:1) (20 mL), and a filtrate obtained was spin-dried and purified by a silica gel column chromatography (DCM:MeOH=15:1), to obtain a product (8.0 g, yield: 91.4%).

**3. Preparation of 4-(2-chloro-8-thio-7,8-dihydro-9H-purine-9-yl) tetrahydro-2H-pyran-4-carbonitrile**

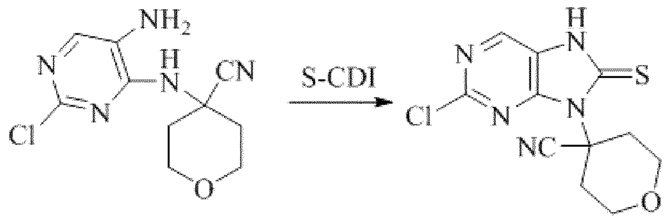


4-((5-amino-2-chloropyrimidin-4-yl) amino) tetrahydro-2H-pyran-4-carbonitrile (4.7 g, 18.5 mmol), imidazole (2.5 g, 36.7 mmol), and S-CDI (6.5 g, 36.5 mmol) were added to DCM (90 mL), and then it was reacted at 25 °C for 1 hour. PH of the system was adjusted to 5 with 2N HCl (40 mL), and a solid was precipitated from the system, then it was suction-filtered under a reduced pressure, and a filter cake was dried to obtain a product (4.5 g, yield: 82.3%).

**4. Preparation of 2-chloro-2',3',5',6,6',7-hexahydrospiro[imidazo[1,2-e]purine-8,4'-pyran]**

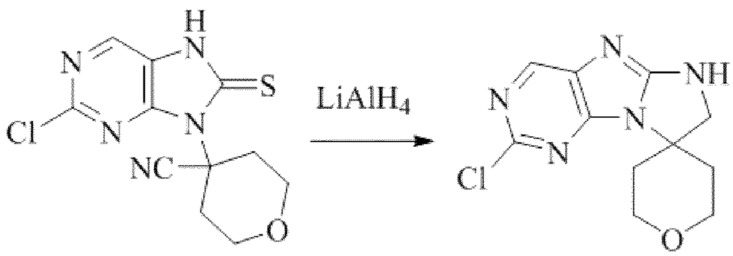


4-(2-chloro-8-thio-7,8-dihydro-9H-purine-9-yl) tetrahydro-2H-pyran-4-carbonitrile (4.0 g, 13.5 mmol) was added to THF (80 mL), then lithium aluminium hydride (1.5 g, 39.5 mmol) was added in batches at 40 °C, and it was reacted at 40 °C for 0.5 hours. The system was cooled to 20 °C, and slowly quenched by adding water (2 mL), and then (DCM:MeOH=10:1) (50 mL) was added and stirred for 0.5 hours. It was suction-filtered under a reduced pressure, a filter cake was washed with (DCM:MeOH=10:1) (50 mL), and a filtrate obtained was concentrated and purified by a silica gel column chromatography (DCM:MeOH=20:1), to obtain a product (1.3 g, yield: 36.2%).

**Example 1: Preparation of 5-methyl-N-(7-methyl-[1,2,4]triazolo[1,5-a]pyridin-6-yl)-2',3',5,5',6',7-hexahydrospiro[imidazo[ 1,2-e]purine-8,4'-pyran]-2-amine (Compound 1)**

**1. Preparation of 2-chloro-5-methyl-2',3',5,5',6',7-hexahydrospiro[imidazo [1,2-e]purine-8,4'-pyran] (Compound 1a)**

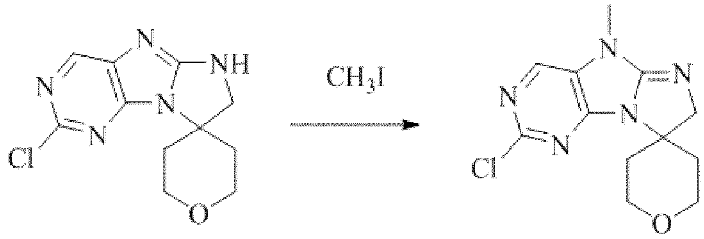


2-chloro-2',3',5',6,6',7-hexahydrospiro[imidazolo[1,2-e]purine-8,4'-pyran] (266 mg, 1 mmol) and iodomethane (2.85 g, 20.0 mmol) were added to acetonitrile (10 mL), and then the system was reacted at 105 °C under microwave for 1 hour. Finally, the system was concentrated and purified by a silica gel column chromatography (DCM:MeOH=20:1), to obtain a product (130 mg, yield: 46.5%).

$^1$HNMR (400MHz,d-DMSO):δ 7.91 (s,1H), 4.10 (s,2H), 3.98-3.92 (m,2H), 3.44-3.40 (m,2H), 3.27 (s,3H), 2.31-2.23 (m,2H), 1.81-1.74 (m,2H).

1D NOESY (400 MHz, d-DMSO): excitation peak (upward): 7.914 ppm; and response peak (downward): 3.271 ppm.

**2. Preparation of 5-methyl-N-(7-methyl-[1,2,4]triazolo[1,5-a]pyridin-6-yl)-2',3',5,5',6',7-hexahydrospiro[imidazo[ 1,2-e]purine-8,4'-pyran]-2-amine**

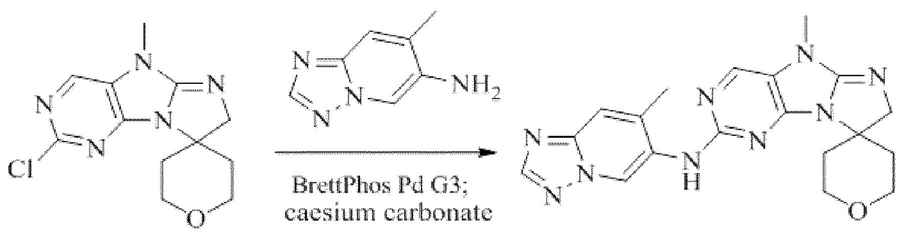


2-chloro-5-methyl-2',3',5,5',6',7-hexahydrospiro[imidazo[1,2-e]purine-8,4'- pyran] (110 mg, 0.39 mmol), 7-methyl- [1,2,4]triazolo[1,5-a]pyridine-6-amine (64 mg, 0.43 mmol), caesium carbonate (254 mg, 0.78 mmol) and BrettPhos Pd G3 (35 mg, 0.039 mmol) were dissolved in 1,4-dioxane (15 mL), and then the system was reacted for 2 hours at 105 °C under an $N_2$ environment. The system was cooled to 20°C, concentrated and purified by a silica gel column chromatography (DCM:MeOH=10:1), to obtain a product (112 mg, yield: 73.4%).

Molecular formula: $C_{19}H_{21}N_9O$

Molecular weight: 391.4

LC-MS (M/e): 392.2 ($M+H^+$)

$^1$HNMR (400MHz,d-DMSO):δ 9.13 (s,1H), 8.45 (s,1H), 8.36 (s, 1H), 7.72-7.68 (m, 2H), 4.08 (s, 2H), 3.97-3.92 (m, 2H), 3.44-3.33 (m, 2H), 3.23 (s, 3H), 2.36 (s, 3H), 2.35-2.31 (m, 2H), 1.80-1.72 (m, 2H).

**Example 2: Preparation of 6-methyl-N-(7-methyl-[1,2,4]triazolo[1,5-a]pyridin-6-yl)-2',3',5',6,6',7-hexahydrospiro[imidazo[ 1,2-e]purine-8,4'-pyran]-2-amine (Compound 2)**

**1. Preparation of 2-chloro-6-methyl-2',3',5',6,6',7-hexahydrospiro[imidazo [1,2-e]purine-8,4'-pyran] (Compound 2a)**

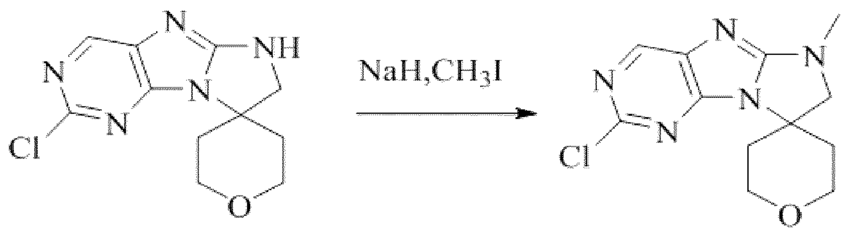


2-chloro-2',3',5',6,6',7-hexahydrospiro[imidazo[1,2-e]purine-8,4'-pyran] (100 mg, 0.38 mmol) was added to THF (12 mL), then NaH (60%, 31 mg, 0.78 mmol) was added to the system at 0°C, it was reacted for 20 minutes, then iodomethane (271 mg, 1.9 mmol) was added, and it was reacted for 1 hour at 20 °C. Finally, the system was concentrated and purified by a silica gel column chromatography (DCM:MeOH=20:1), to obtain a product (100 mg, yield: 94.1%).

$^1$HNMR (400MHz, d-DMSO): 8.27 (s, 1H), 4.05-3.97 (m, 4H), 3.52-3.42 (m, 2H), 3.01 (s, 3H), 2.35-2.25 (m, 2H), 1.96-1.88 (m, 2H).

1D NOESY (400 MHz, d-DMSO): excitation peak (upward): 3.014 ppm; and response peak (downward): 4.012 ppm

**2. Preparation of 6-methyl-N-(7-methyl-[1,2,4]-triazolo[1,5-a]pyridin-6-yl)-2',3',5',6,6',7-hexahydrospiro[imidazo [1,2-e]purine-8,4'-pyran]-2-amine**

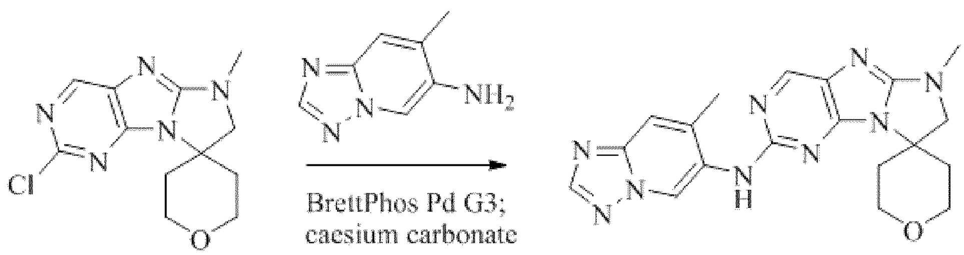


2-chloro-6-methyl-2',3',5',6,6',7-hexahydrospiro[imidazo[1,2-e]purine-8,4'-pyran] (90 mg, 0.32 mmol), 7-methyl-[1,2,4]triazolo[1,5-a]pyridine-6-amine (48 mg, 0.32 mmol), caesium carbonate (208.5 mg, 0.64 mmol) and BrettPhos Pd G3 (29 mg, 0.032 mmol) were dissolved in 1,4-dioxane (15 mL), and then the system was reacted for 2 hours at 105 °C under an $N_2$ environment. The system was cooled to 20 °C, concentrated and purified by a silica gel column chromatography (DCM:MeOH=15:1), to obtain a product (40 mg, yield: 32.0%).

Molecular formula: $C_{19}H_{21}N_9O$

Molecular weight: 391.4

LC-MS (M/e): 392.2 ($M+H^+$)

$^1$HNMR (400MHz,CDCl3):δ 9.79 (s, 1H), 8.34 (s, 1H), 8.25 (s, 1H), 7.57 (s, 1H), 6.64 (s, 1H), 4.28-4.22 (m, 2H), 3.88 (s, 2H), 3.63-3.53 (m, 2H), 3.11 (s, 3H), 2.69-2.61 (m, 2H), 2.52 (s, 3H), 1.93-1.87 (m, 2H).

**Example 3: Preparation of N-(7-methyl-[1,2,4]-triazolo[1,5-a]pyridin-6-yl)-2',3',5',6,6',7-hexahydrospiro[imidazo[1,2-e]pur ine-8,4'-pyran]-2-amine (Compound 3)**

**1. Preparation of tert-butyl 2-chloro-2',3',5',6'-tetrahydrospiro[imidazo [1,2-e]purine-8,4'-pyran]-6(7H)- carboxylate**

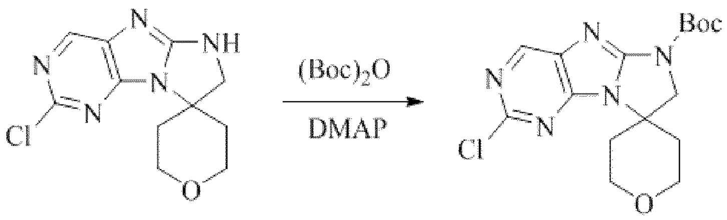


2-chloro-2',3',5',6,6',7-hexahydrospiro[imidazo[1,2-e]purine-8,4'-pyran] (200 mg, 0.75 mmol), $Boc_2O$ (328 mg, 1.5 mmol), and DMAP (184 mg, 1.5 mmol) were added to THF (15 mL), and then it was reacted at 25 °C for 2 hours. Finally, the system was concentrated and purified by a silica gel column chromatography (DCM:MeOH=35:1), to obtain a product (250 mg, yield: 91.1%).

**2. Preparation of tert-butyl 2-((7-methyl-[1,2,4]-triazolo[1,5-a]pyridin-6-yl) amino)-2',3',5',6'-tetrahydrospiro[imidazo[1,2-e]purine-8,4'-pyran]-6(7H)-carboxylate**

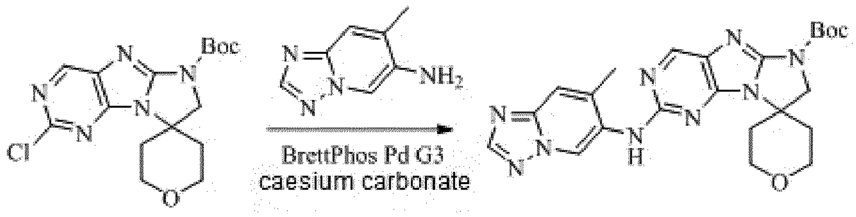


Tert-butyl 2-chloro-2',3',5',6'-tetrahydrospiro[imidazo[1,2-e]purine-8,4'-pyran]-6 (7H)-carboxylate (220 mg, 0.60 mmol), 7-methyl-[1,2,4]triazolo[1,5-a]pyridin-6-amine (98 mg, 0.66 mmol), caesium carbonate (391 mg, 1.2 mmol) and BrettPhos Pd G3 (55 mg, 0.061 mmol) were dissolved in 1,4-dioxane (15 mL), and then the system was reacted for 3 hours at 105 °C under an $N_2$ environment. The system was cooled to 20°C, concentrated and purified by a silica gel column chromatography (DCM:MeOH=30:1), to obtain a product (260 mg, yield: 90.8%).

**3. Preparation of N-(7-methyl-[1,2,4]triazolo[1,5-a]pyridin-6-yl)-2',3',5',6,6',7-hexahydrospiro[imidazo[1,2-e]puri ne-8,4'-pyran] -2-amine**

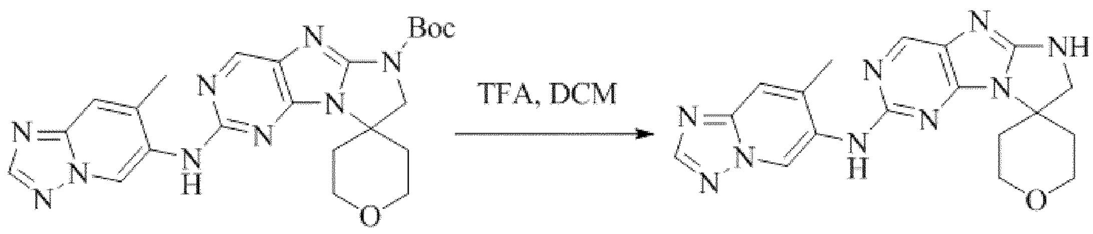


Tert-butyl 2-((7-methyl-[1,2,4]-triazolo[1,5-a]pyridin-6-yl)amino)-2',3',5',6'-tetrahydrospiro[imidazo[1,2-e]purine -8,4'-pyran]-6(7H)-carboxylate (230 mg, 0.48 mmol) was added to DCM (6 mL), then TFA (3 mL) was added, and the system was reacted at 25 °C for 4 hours. Then, pH of a crude product obtained from system concentration was adjusted to alkaline by using saturated sodium bicarbonate solution (1 mL), and it was concentrated and purified by a silica gel column chromatography (DCM:MeOH=15:1), to obtain a product (124 mg, yield: 68.5%).

Molecular formula: $C_{18}H_{19}N_9O$

Molecular weight: 377.4

LC-MS (M/e): 378.2 ($M+H^+$)

$^1$HNMR (400MHz, d-DMSO):δ 9.19 (s,1H), 8.75 (s,1H),8.39 (s,1H), 8.10 (s,1H), 8.03 (s,1H), 7.72 (s,1H), 4.04-3.94 (m,4H), 3.58-3.53 (m,2H), 3.43-3.33 (m,5H), 1.91-1.87 (m,2H).

**Example 4: Preparation of 5'-methyl-N-(7-methyl-[1,2,4]triazolo[1,5-a]pyridin-6-yl)-5',7'-dihydrospiro[cyclohexane-1,8'-imidazo[1,2-e]purine]-2'-amine (Compound 4)**

**1. Preparation of 1-aminocyclohexane-1-nitrile**

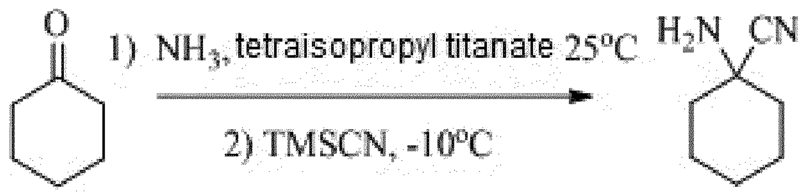


Cyclohexanone (2.7 g, 27.5 mmol) was dissolved in ammonia tetrahydrofuran solution (40 ml), tetraisopropyl titanate (6.0 mL) was added, it was stirred for 6 hours at 25°C, the temperature was reduced to -10°C and TMSCN (2.7 g, 27.5 mmol) was added, the temperature was raised to 0 °C and it was stirred for 16 hours, a filtrate was extracted with DCM (100 mL), and an organic phase was spin-dried to obtain a product (3.1 g) which was directly use in the next reaction.

**2. Preparation of 1-((2-chloro-5-nitropyrimidin-4-yl) amino) cyclohexane-1-carbonitrile**

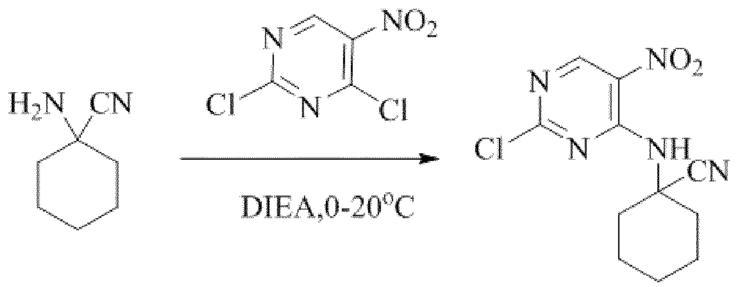


2,4-dichloro-5-nitropyrimidine (4.5 g, 23.3 mmol) was dissolved in THF (100 mL), it was stirred at °C, 1-aminocyclohexane-1-carbonitrile (2.9 g, 23.3 mmol) and DIEA (6.0 g, 46.6 mmol) were added, the temperature was raised to 20°C and it was continuously stirred for 1 hour. It was spin-dried and purified by a silica gel column chromatography (PE/EA=8/1), to obtain a product (1.6 g, yield: 24.3%).

**3. Preparation of 1-((5-amino-2-chloropyrimidin-4-yl) amino) cyclohexane-1-carbonitrile**

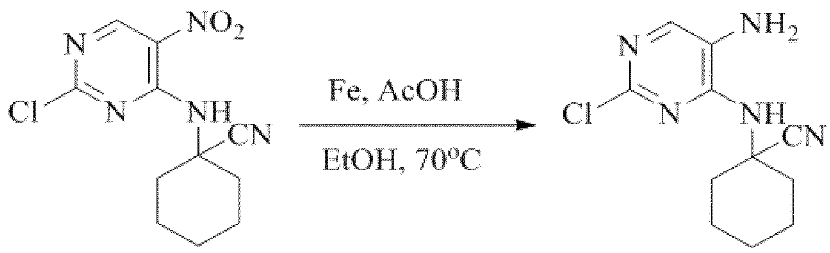


1-((2-chloro-5-nitropyrimidin-4-yl) amino) cyclohexane-1-carbonitrile (1.6 g, 5.7 mmol) was dissolved in EtOH (10 ml), iron powder (1.3 g, 22.8 mmol), glacial acetic acid (10 mL), and water (10 mL) were added, the temperature was raised to 70°C and it was stirred for 1 hour. It was filtered, water (50 mL) was added, it was adjusted to pH>7 with NaOH aqueous solution, and it was extracted with DCM (100 mL). An organic phase was spin-dried and purified by a silica gel column chromatography (DCM/MeOH=10/1), to obtain a product (800 mg, yield: 56.0%).

**4. Preparation of 1-(2-chloro-8-thio-7,8-dihydro-9H-purine-9-yl)cyclohexane-1-carbonitrile**

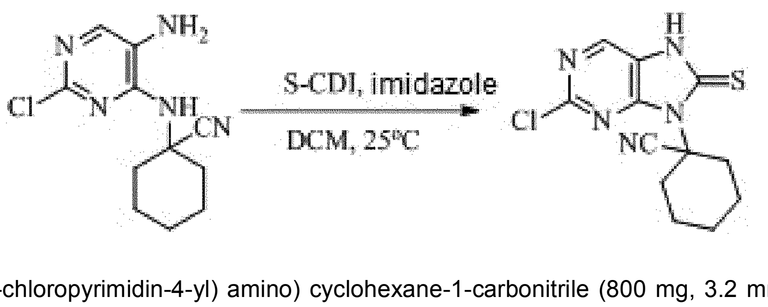


1-((5-amino-2-chloropyrimidin-4-yl) amino) cyclohexane-1-carbonitrile (800 mg, 3.2 mmol) was dissolved in DCM (30 mL), imidazole (361 mg, 5.3 mmol) and S-CDI (944 mg, 5.3 mmol) were added, it was stirred at 25°C for 1 hour, and filtered, to obtain a product (650 mg, yield: 69.6%).

**5. Preparation of 2'-chloro-5',7'-dihydrospiro[cyclohexane-1,8'-imidazo[1,2-e]purine]**

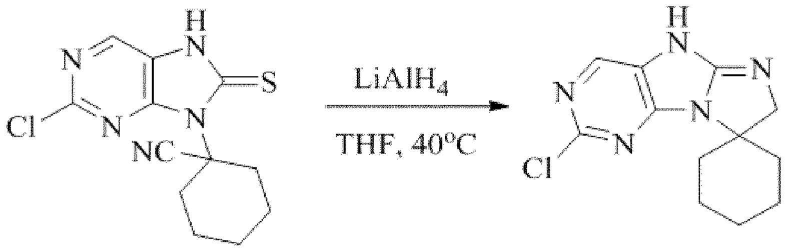


1-(2-chloro-8-thio-7,8-dihydro-9H-purine-9-yl) cyclohexane-1-carbonitrile (620 mg, 2.1 mmol) was dissolved in THF (20 ml), $LiAlH_4$ (340 mg, 6.3 mmol) was added, it was stirred at 40°C for 15 minutes, water was added for quenching the reaction, it was spin-dried and purified by a silica gel column chromatography (DCM/MeOH=20/1), to obtain a product (200 mg, yield: 35.9%).

**6. Preparation of 2'-chloro-5'-methyl-5',7'-dihydrospiro[cyclohexane-1,8'-imidazo [1,2-e]purine]**

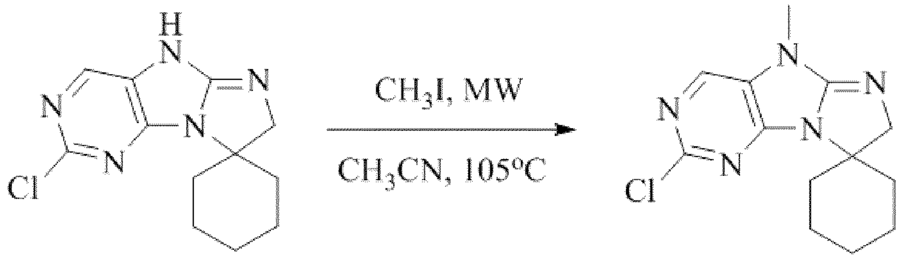


2'-chloro-5',7'-dihydrospiro[cyclohexane-1,8'-imidazo[1,2-e]purine] (150 mg, 0.57 mmol) was dissolved in acetonitrile (10 mL), iodomethane (1.6 g, 11.4 mmol) was added, it was stirred at 105 °C for 1.5 h under microwave, it was spin-dried and purified by a silica gel column chromatography (DCM/MeOH=94/6), to obtain the product (90 mg, yield: 57.0%).

**7. Preparation of 5'-methyl-N-(7-methyl-[1,2,4]-triazolo[1,5-a]pyridin-6-yl)-5',7'-dihydrospiro[cyclohexane-1,8'-i midazo[1,2-e]purine]-2'-amine**

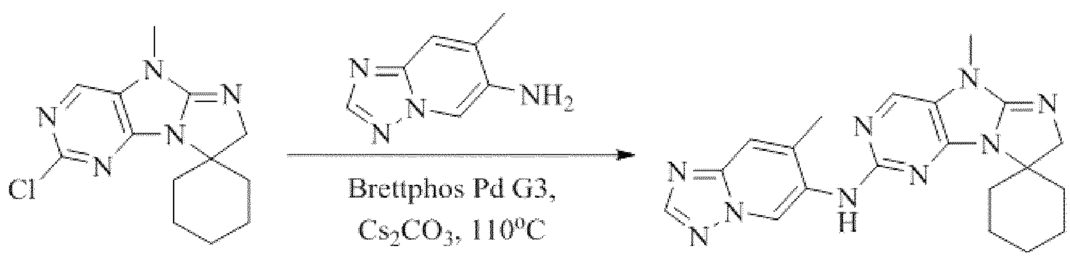


(2'-chloro-5'-methyl-5',7'-dihydrospiro[cyclohexane-1,8'-imidazo[1,2-e]purine] (90 mg, 0.32 mmol) was dissolved in dioxane (30 mL), 7-methyl-[1,2,4]triazolo[1,5-a]pyridine-6-amine (52 mg, 0.35 mmol), Brettphos Pd G3 (29 mg, 0.032 mmol), and $Cs_2CO_3$ (209 mg, 0.64 mmol) were added, and it was stirred under nitrogen protection at 110 °C for 24 hours. The solvent was spin-dried and it was purified by a silica gel column chromatography (DCM/MeOH=20/1), to obtain a product (45 mg, yield: 35.7%).

Molecular formula: $C_{20}H_{23}N_9$

Molecular weight: 389.21

LC-MS (M/e): 390.2 (M+H+)

$^1$H-NMR (400MHz,$CDCl_3$) δ:9.14 (s,1H), 8.53 (s,1H), 8.38 (s,1H), 7.80 (s,1H), 7.70 (s,1H), 3.98 (s,2H), 3.27 (s,3H), 2.39 (s,3H), 2.19-2.09 (m,2H), 1.82-1.76 (m,4H), 1.67-1.58 (m,1H), 1.34-1.14 (m,3H).

**Example 5: Preparation of 4,4-difluoro-5'-methyl-N-(7-methyl-[1,2,4]-triazolo[1,5-a]pyridin-6-yl)-5',7'-dihydrospiro[cyclo hexane-1,8'-imidazo[1,2-e]purine]-2'-amine (Compound 5)**

**1. Preparation of 2'-chloro-4,4-difluoro-5'-methyl-5',7'-dihydrospiro[cyclohexane-1,8'- imidazo[1,2-e]purine]**

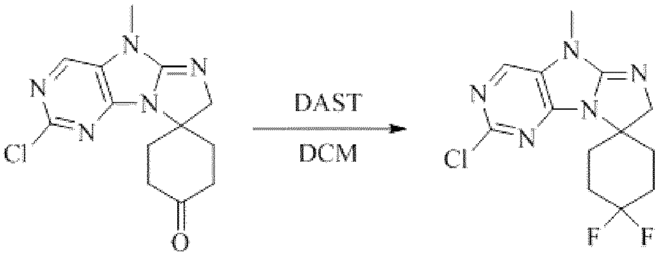


2'-chloro-5'-methyl-5',7'-dihydrospiro[cyclohexane-1,8'-imidazo[1,2-e]purine]-4-one (100.0 mg, 0.34 mmol) was dissolved in DCM (5.0 mL), DAST (109.6 mg, 0.68 mmol) was added, it was stirred at 16°C for 2 hours, the solvent was spin-dried, and it was purified by a silica gel column chromatography (DCM/MeOH=20/1), to obtain a product (60.0 mg, yield: 55.8%).

**2. Preparation of 4,4-difluoro-5'-methyl-N-(7-methyl-[1,2,4]-triazolo[1,5-a]pyridin-6-yl)-5',7'-dihydrospiro[cyclo hexane-1,8'-imidazo[1,2-e]purine]-2'-amine**

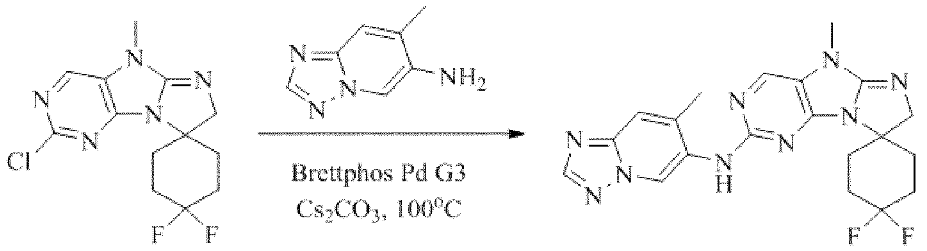


2'-chloro-4,4-difluoro-5'-methyl-5',7'-dihydrospiro[cyclohexane-1,8'-imidazo[1,2-e]purine] (50.0 mg, 0.16 mmol) was dissolved in dioxane (3 mL), 7-methyl-[1,2,4]triazolo[1,5-a]pyridin-6-amine (23.7 mg, 0.16 mmol), Brettphos Pd G3 (18.2 mg, 0.02 mmol), and $Cs_2CO_3$ (104.3 mg, 0.32 mmol) were added, and it was stirred under nitrogen protection at 100°C for 2 hours. The solvent was spin-dried, and it was prepared and purified under a high pressure (acetonitrile/water), to obtain a product (4.5 mg, yield: 6.6%).

Molecular formula: $C_{20}H_{21}F_2N_9$

Molecular weight: 425.4

LC-MS (M/e): 426.2 (M+H+)

$^1$H-NMR (400MHz,$CDCl_3$) δ: 9.68 (s,1H), 8.27 (s,1H), 7.60 (s,1H), 7.57 (s,1H), 6.55 (s,1H), 4.15 (s,2H), 3.36 (s,3H), 2.53-2.34 (m,7H), 2.06-1.93 (m,4H).

**Example 6: Preparation of 5'-methyl-2'-((7-methyl-[1,2,4]-triazolo[1,5-a]pyridin-6-yl) amino)-5',7'-dihydrospiro[cyclohexane-1,8'-imidazo[1,2-e]purine]-4-one (Compound 6)**

**1. Preparation of 8-amino-1,4-dioxaspiro[4.5]decane-8-carbonitrile**

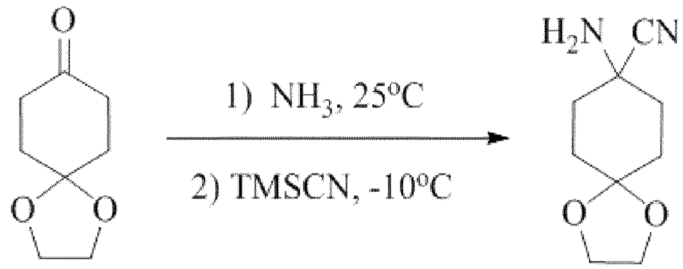


1,4-dioxaspiro[4.5]decane-8-ketone (23.0 g, 147.2 mmol) was dissolved in methanol (40 ml), 7 M ammonia/methanol (115.7 mL, 809.6 mmol) was added, it was stirred at 25°C for 4 hours, TMSCN (14.6 g, 147.2 mmol) was added, and it was stirred at-10°C for 12 hours, and spin-dried to obtain a product (26.0 g, yield: 97.0%).

**2. Preparation of 8-((2-chloro-5-nitropyrimidin-4-yl) amino)-1,4-dioxaspiro[4.5]decane-8-carbonitrile**

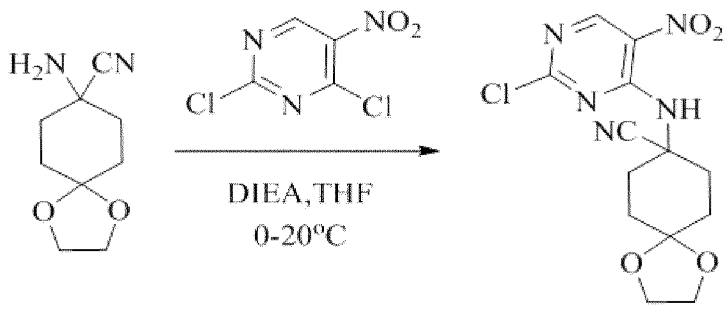


2,4-dichloro-5-nitropyrimidine (25.2 g, 129.8 mmol) was dissolved in THF (400 mL), it was stirred at 0°C, 8-amino-1,4-dioxaspiro[4.5]decane-8-carbonitrile (26.0 g, 142.8 mmol) and DIEA (33.6 g, 259.6 mmol) were added, it was stirred at 20°C for 2 hours, it was spin-dried and purified by a silica gel column chromatography (PE/EA=8/1~3/1), to obtain a product (40.0 g, yield: 90.7%).

**3. Preparation of 8-((5-amino-2-chloropyrimidin-4-yl) amino)-1,4-dioxaspiro[4.5]decane-8-carbonitrile**

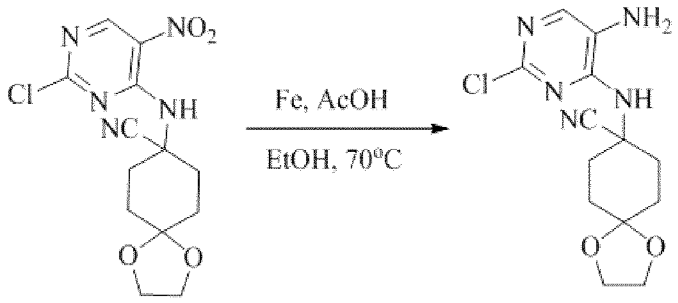


8-((2-chloro-5-nitropyrimidin-4-yl) amino)-1,4-dioxaspiro[4.5]decane-8-carbonitrile (38.0 g, 111.8 mmol) was dissolved in EtOH (300 ml), iron powder (25.0 g, and 447.2 mmol), glacial acetic acid (300 mL), and water (300 mL)

were added, the temperature was raised to 70°C, and it was stirred for 1 hour. It was filtered, adjusted to pH>7 by adding sodium carbonate aqueous solution, and extracted with EA (600 mL). An organic phase was spin-dried and purified by a silica gel column chromatography (DCM/MeOH=80/1~40/1), to obtain a product (26 g, yield: 74.9%).

**4. Preparation of 8-(2-chloro-8-thio-7,8-dihydro-9H-purine-9-yl)-1,4-dioxaspiro[4.5]decane-8-carbonitrile**

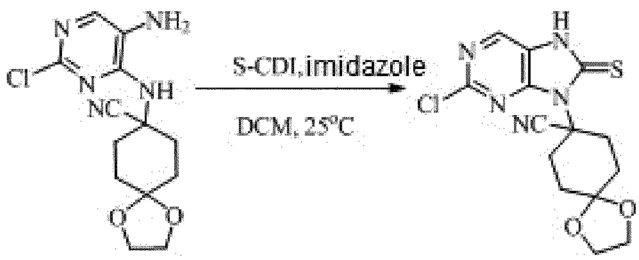


8-((5-amino-2-chloropyrimidin-4-yl) amino)-1,4-dioxaspiro[4.5]decane-8-carbonitrile (25 g, 80.6 mmol) was dissolved in DCM (400 mL), imidazole (11 g, 161.2 mmol) and S-CDI (28.8 g, 161.2 mmol) were added, it was stirred at 25°C for 6 hours, and filtered to obtain a product (16 g, yield: 56.3%).

**5. Preparation of 2-chloro-5,7-dihydrodispiro[imidazo[1,2-e]purine-8,1'-cyclohexane-4',2"-[1,3]dioxolane]**

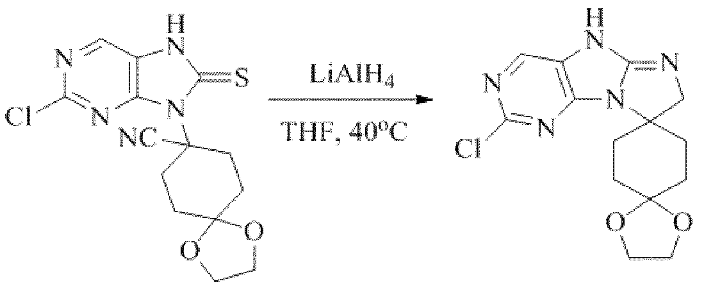


8-(2-chloro-8-thio-7,8-dihydro-9H-purine-9-yl)-1,4-dioxaspiro[4.5]decane-8-carbonitrile (10 g, 28.4 mmol) was dissolved in THF (100 ml), $LiAlH_4$ (3.2 g, 85.2 mmol) was added, it was stirred at 40°C for 1 hour, water was added for quenching the reaction, and it was spin-dried and purified by a silica gel column chromatography (DCM/MeOH=60/1~20/1), to obtain a product (5.6 g, yield: 61.3%).

**6. Preparation of 2-chloro-5-methyl-5,7-dihydrodispiro[imidazo[1,2-e]purine-8,1'-cyclohexane-4',2"-[1,3]dioxolane]**

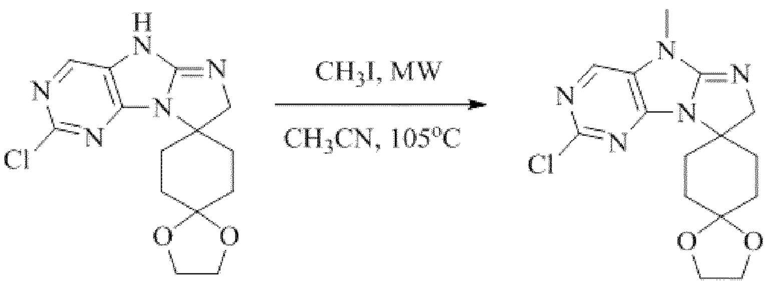


2-chloro-5,7-dihydrodispiro[imidazo[1,2-e]purine-8,1'-cyclohexane-4',2"-[1,3]dioxolane] (5 g, 15.5 mmol) was dissolved in acetonitrile (70 mL), iodomethane (66 g, 465 mmol) was added, it was stirred under microwave at 105 °C for 1 h, and filtered to obtain a product (3.8 g, yield: 73.1%).

**7. Preparation of 2'-chloro-5'-methyl-5',7'-dihydrospiro[cyclohexane-1,8'-imidazo[1,2-e]purine]-4-one**

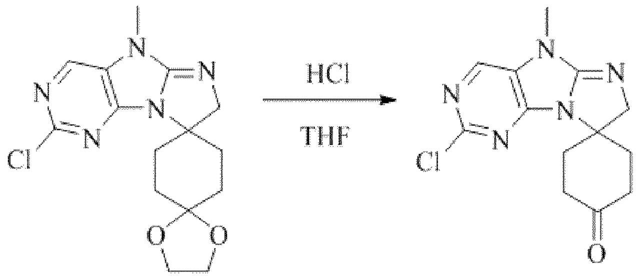


2-chloro-5-methyl-5,7-dihydrodispiro[imidazo[1,2-e]purine-8,1'-cyclohexane-4',2"-[1,3]diox olane] (3.5 g, 10.4 mmol) was dissolved in THF (76 mL), a concentrated hydrochloric acid (20 ml) was added, it was stirred at 16°C for 6 hours, it was added to sodium carbonate aqueous solution so that pH was kept at 8-9, and it was extracted with EA, to obtain a product (1.2 g, yield: 40%).

**8. Preparation of 5'-methyl-2'-((7-methyl-[1,2,4]-triazolo[1,5-a]pyridin-6-yl) amino)-5',7'-dihydrospiro[cyclohexane-1,8'-imidazo [1,2-e]purine]-4-one**

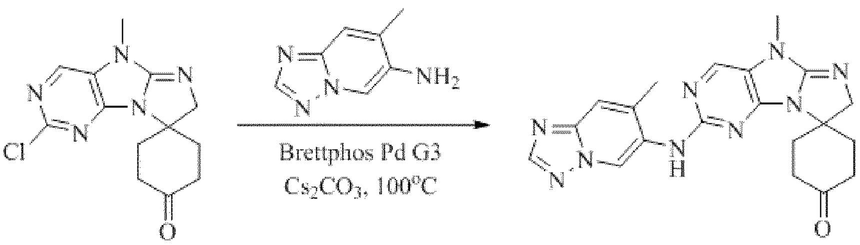


2'-chloro-5'-methyl-5',7'-dihydrospiro[cyclohexane-1,8'-imidazo[1,2-e]purine]-4-one (50 mg, 0.17 mmol) was dissolved in dioxane (3 mL), 7-methyl-[1,2,4]triazolo[1,5-a]pyridin-6-amine (25.2 mg, 0.17 mmol), Brettphos Pd G3 (15.4 mg, 0.017 mmol), and $Cs_2CO_3$ (110.8 mg, 0.34 mmol) were added, and it was stirred under nitrogen protection at 100°C for 6 hours. The solvent was spin-dried, and it was purified by a silica gel column chromatography (DCM/MeOH=40/1~15/1), to obtain a product (2.5 mg, yield: 3.6%).

Molecular formula: $C_{20}H_{21}N_9O$

Molecular weight: 403.5

LC-MS (M/e): 404.2 (M+H$^+$)

$^1$H-NMR (400MHz,$CDCl_3$) δ: 9.03 (s,1H), 8.54 (s,1H), 8.36 (s,1H), 7.81 (s,1H), 7.66 (s,1H), 4.14 (s,2H), 3.39 (s,3H), 2.63-2.58 (m,2H), 2.50-2.45 (m,2H), 2.34 (s,3H), 2.14-2.01 (m,2H), 1.99-1.91 (m,2H).

**Example 7: Preparation of 5'-methyl-2'-((7-methyl-[1,2,4]-triazolo[1,5-a]pyridin-6-yl) amino)-5',7'-dihydrospiro[cyclohexane-1,8'-imidazo[1,2-e]purine]-4-ol (Compounds 7-1 and 7-2)**

**1. Preparation of 2'-chloro-5'-methyl-5', 7'-dihydrospiro[cyclohexane-1,8'-imidazo[1,2-e]purine]-4-ol**

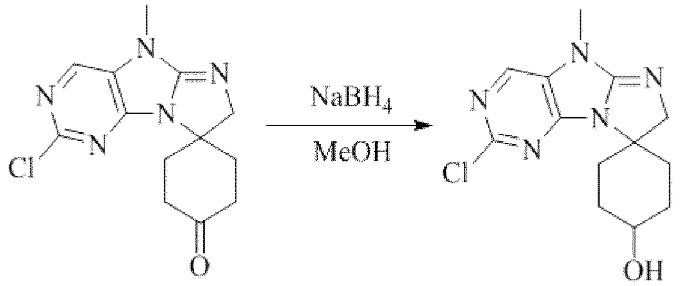


2'-chloro-5'-methyl-5',7'-dihydrospiro[cyclohexane-1,8'-imidazo[1,2-e]purine]-4-one (800.0 mg, and 2.7 mmol) was dissolved in MeOH (10.0 mL), sodium borohydride (43.2 mg, and 5.4 mmol) was added, it was stirred at 16°C for 1 hour, the solvent was spin-dried, and it was purified by a silica gel column chromatography (DCM/MeOH=40/1~20/1), to obtain a product (350.0 mg, and yield: 43.5%).

**2. Preparation of 5'-methyl-2'-((7-methyl-[1,2,4]-triazolo[1,5-a]pyridin-6-yl) amino)-5',7'-dihydrospiro[cyclohexane-1,8'-imidazo[1,2-e]purine]-4-ol**

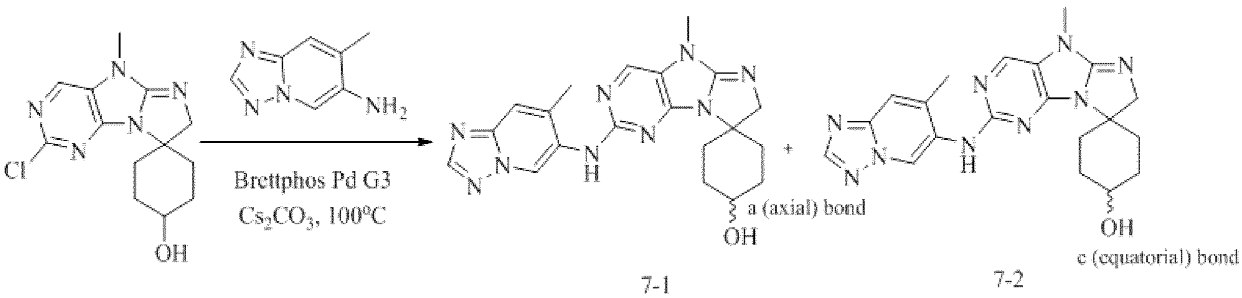


7-1 7-2

2'-chloro-5'-methyl-5',7'-dihydrospiro[cyclohexane-1,8'-imidazo[1,2-e]purine]-4-ol (59.0 mg, 0.2 mmol) was dissolved in dioxane (3 mL), 7-methyl-[1,2,4]triazolo[1,5-a]pyridine-6-amine (29.6 mg, 0.2 mmol), Brettphos Pd G3 (18.2 mg, 0.02 mmol), and $Cs_2CO_3$ (130.3 mg, 0.4 mmol) were added, and it was stirred under nitrogen protection at 100 °C for 2 hours, and was purified by prepared TLC (DCM/MeOH=8/1), to obtain compounds 7-1 and 7-2.

Molecular formula: $C_{20}H_{23}N_9O$

Molecular weight: 405.5

LC-MS (M/e): 406.3 ($M+H^+$)

The retention time, yield, and hydrogen spectrum of the two compounds obtained were as follows:

1) HPLC retention time RT: 6.6 min (high polarity); 14.0 mg, and yield: 17.2%;
$^1$H-NMR (400MHz,$CDCl_3$) δ:10.01 (s,1H), 8.24 (s,1H), 7.60 (s,1H), 7.55 (s,1H), 6.73 (s,1H), 4.15 (s,2H), 3.51 (s,3H), 2.85-2.78 (m,2H), 2.78 (s,3H), 2.11-2.03 (m,2H), 1.77-1.66 (m,4H).

2) HPLC retention time RT: 6.7 min (low polarity); 15.6 mg, and yield: 19.2%;
$^1$H-NMR (400MHz,$CDCl_3$) δ: 9.77 (s,1H), 8.27 (s,1H), 7.58 (s,1H), 7.28 (s,1H), 6.60 (s,1H), 4.18 (s,2H), 3.93 (m,1H), 3.88 (s,1H), 4.18 (s,3H), 2.43 (s,3H), 2.26-2.10 (m,2H), 2.02-1.95 (m,2H), 1.90-1.85 (m,2H), 1.52-1.49 (m,4H).

**Example 8: Preparation of 5-methyl-N-(7-methyl-[1,2,4]triazolo[1,5-a]pyridin-6-yl)-2',3',5,5',6',7-hexahydrospiro[imidazo[ 1,2-e]purine-8,4'-thiopyran]-2-amine (Compound 8)**

**1. Preparation of 4-aminotetrahydro-2H-thiopyran-4-carbonitrile**

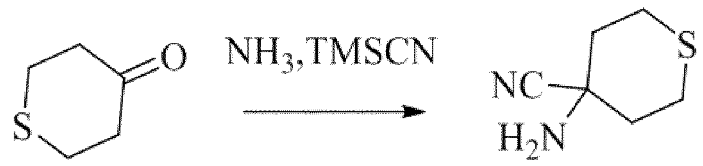


Tetrahydro-4H-thiopyran-4-one (2.4 g, 20.7 mmol) was added to 7 M ammonia methanol solution (10 mL, 70.0 mmol), it was reacted at 0°C for 4 hours, then TMSCN (2.05 g, 20.7 mmol) was added at 0°C, and it was reacted at 20 °C for 4 hours. The system was concentrated to obtain a crude product (2.9 g).

**2. Preparation of 4-((2-chloro-5-nitropyrimidin-4-yl) amino) tetrahydro-2H-thiopyran-4-carbonitrile**

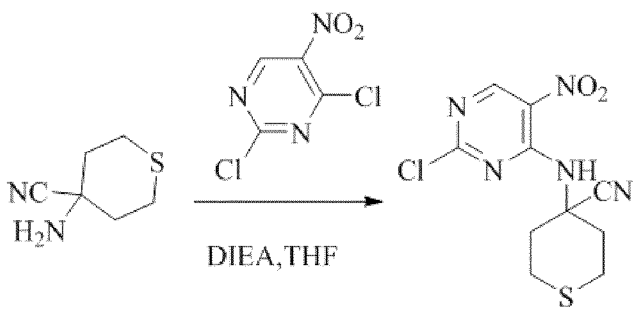


2,4-dichloro-5-nitropyrimidine (3.7 g, 19.3 mmol) and DIEA (5.0 g, 38.7 mmol) were added to THF (60 mL), 4-aminotetrahydro-2H-thiopyran-4-carbonitrile (2.75 g of a crude product) was added at -20°C, and then it was reacted at 25°C for 4 hours. The system was concentrated and purified by a silica gel column chromatography (PE: EA=3:1) to obtain a target compound (4.0 g, yield: 69.2%).

**3. Preparation of 4-((5-amino-2-chloropyrimidin-4-yl) amino) tetrahydro-2H-thiopyran-4-carbonitrile**

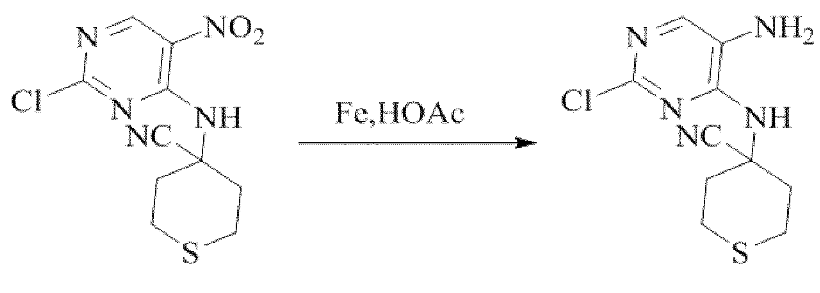


4-((2-chloro-5-nitropyrimidin-4-yl) amino) tetrahydro-2H-thiopyran-4-carbonitrile (3.6 g, 12.0 mmol) and iron powder (2.7 g, 48.2 mmol) were added to a mixed system of HOAc (20 mL), ethanol (20 mL) and water (20 mL), and then it was reacted at 75°C for 1 hour. The system was cooled to 15°C, it was suction-filtered under a reduced pressure, a filter cake was washed with (DCM:MeOH=10:1) (20 mL), and a filtrate obtained was spin-dried and purified by a silica gel column chromatography (DCM: MeOH=40:1), to obtain a product (1.3 g, yield: 40.2%).

**4. Preparation of 4-(2-chloro-8-thio-7,8-dihydro-9H-purine-9-yl) tetrahydro-2H-thiopyran-4-carbonitrile**

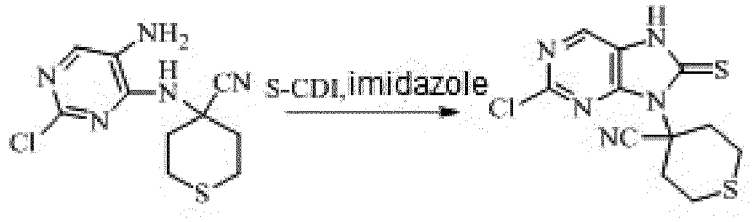


4-((5-amino-2-chloropyrimidin-4-yl)amino)tetrahydro-2H-thiopyran-4-carbonitrile (1.1 g, 4.1 mmol), imidazole (560 mg, 8.2 mmol), and S-CDI (1.45 g, 8.1 mmol) were added to DCM (30 mL), and then the system was reacted at 15°C for 2 hours, pH of the system was adjusted to 6 with 1N HCl, a solid was precipitated from the system, then it was suction-filtered under a reduced pressure, and a filter cake was dried to obtain a target compound (900 mg, yield: 70.4%).

**5. Preparation of 2-chloro-2',3',5',6,6',7-hexahydrospiro[imidazo[1,2-e]purine-8,4'-thiopyran]**

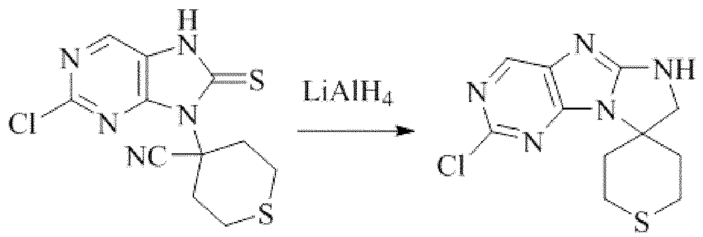


4-(2-chloro-8-thio-7,8-dihydro-9H-purine-9-yl)tetrahydro-2H-thiopyran-4-carbonitrile (800 mg, 2.6 mmol) was added to THF (30 mL), then lithium aluminum hydride (293 mg, 7.7 mmol) was added in batches at 40 °C, and it was reacted at 40°C for 1 hour. The system was cooled to 15°C, water (0.5 mL) was slowly added to quench the reaction, and then (DCM: MeOH=10:1) (40 mL) was added and it was stirred for 0.5 hours. It was suction-filtered under a reduced pressure, a filter cake was washed with (DCM: MeOH=10:1) (50 mL), and a filtrate obtained was concentrated and purified by a silica gel column chromatography (DCM:MeOH=35:1), to obtain a product (150 mg, yield: 20.5%).

**6. Preparation of 2-chloro-5-methyl-2',3',5,5',6',7-hexahydrospiro[imidazo[1,2-e]purine-8,4'- thiopyran]**

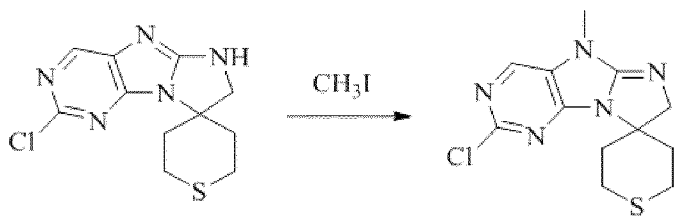


2-chloro-2',3',5',6,6',7-hexahydrospiro[imidazo[1,2-e]purine-8,4'-thiopyran] (140 mg, 0.50 mmol) was added to acetonitrile (5 mL), then iodomethane (1 mL) was added to the system, and it was reacted for 1 h under microwave at 105 °C. Finally, the system was concentrated and saturated sodium bicarbonate solution (0.5 mL) was added. A silica gel column chromatography (DCM:MeOH=35:1) is performed, to obtain a product (85 mg, yield: 57.5%).

**7. Preparation of 5-methyl-N-(7-methyl-[1,2,4]triazolo[1,5-a]pyridin-6-yl)-2',3', 5,5',6',7-hexahydrospiro[imidazo[1,2-e]purine-8,4'-thiopyran]-2-amine**

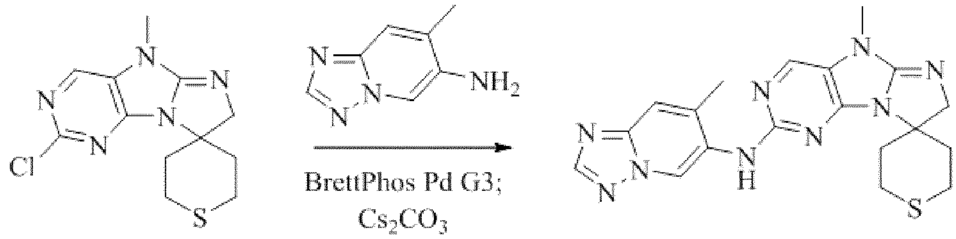


2-chloro-5-methyl-2',3',5,5',6',7-hexahydrospiro[imidazo[1,2-e]purine-8,4'-thiopyran] (75 mg, 0.25 mmol), 7-methyl-[1,2,4]triazolo[1,5-a]pyridin-6-amine (41 mg, 0.28 mmol), caesium carbonate (163 mg, 0.50 mmol) and BrettPhos Pd G3 (23 mg, and 0.025 mmol) were dissolved in 1,4-dioxane (10 mL), and then the system was reacted for 4 hours at 105 °C under an $N_2$ environment. The system was concentrated, and purified by a silica gel column chromatography (DCM:MeOH=20:1), to obtain a product (65 mg, yield: 63.8%).

Molecular formula: $C_{19}H_{21}N_9S$

Molecular weight: 407.5

LC-MS (M/e): 408.2 ($M+H^+$)

$^1$HNMR (400MHz,$CDCl_3$):δ 9.80 (s,1H), 8.27 (s,1H), 7.61-7.55 (m,2H), 6.58 (s,1H), 4.10 (s,2H), 3.26 (s,3H), 2.95-2.88 (m,2H), 2.79-2.70 (m,2H), 2.69-2.59 (m,2H), 2.52 (s,3H), 2.18-2.12 (m,2H).

**Example 9: Preparation of 5-methyl-2-((7-methyl-[1,2,4]-triazolo[1,5-a]pyridin-6-yl) amino)-2',3',5,5',6',7-hexahydrospiro[imidazo[1,2-e]purine-8,4'-thiopyran]1',1'-dioxide (Compound 9)**

**1. Preparation of 2-chloro-5-methyl-2',3',5,5',6',7-hexahydrospiro[imidazo[1,2-e]purine-8,4'-thiopyran]1,1' -dioxide**

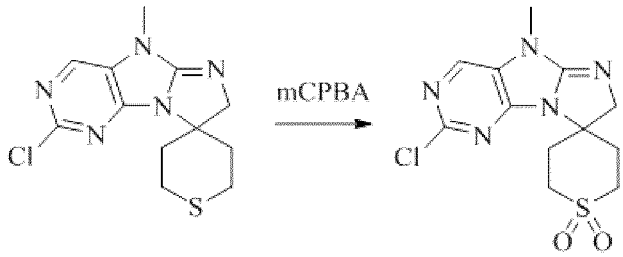


2-chloro-5-methyl-2',3',5',6',7-hexahydrospiro[imidazo[1,2-e]purine-8,4'-thiopyran] (110 mg, 0.37 mmol) was added to DCM (10 mL), then mCPBA (80%, 240 mg, 1.11 mmol) was added to the system, and it was reacted at 20°C for 2 hours. Saturated sodium bicarbonate solution (5 mL) was added to the system for quenching the reaction and it was extracted and liquid-separated. An organic phase was purified by a preparation large plate (DCM:MeOH=15:1), to obtain a product (25 mg, yield: 20.6%).

**2. Preparation of 5-methyl-2-((7-methyl-[1,2,4]-triazolo[1,5-a]pyridin-6-yl) amino)-2',3',5,5',6',7-hexahydrospiro[imidazo[1,2-e]purine-8,4'-thiopyran]1', 1'- dioxide**

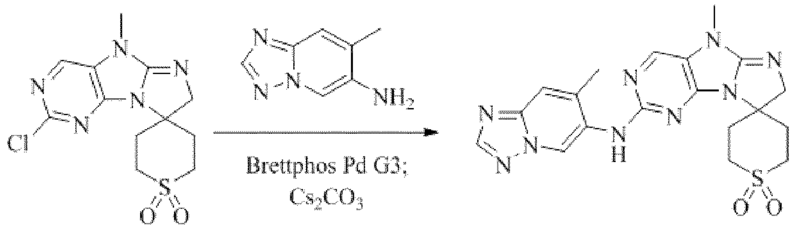


2-chloro-5-methyl-2',3',5,5',6',7-hexahydrospiro[imidazo[1,2-e]purine-8,4'-thiopyran]1',1'-di oxide (20 mg, 0.061 mmol), 7-methyl-[1,2,4]triazolo[1,5-a]pyridin-6-amine (11 mg, 0.074 mmol), caesium carbonate (40 mg, 0.12 mmol) and BrettPhos Pd G3 (6 mg, 0.0066 mmol) were dissolved in 1,4-dioxane (8 mL), and then the system was reacted under $N_2$ environment at 110°C for 6 hours. The system was concentrated, and purified by a silica gel column chromatography (DCM:MeOH=15:1), to obtain a product (6 mg, yield: 22.4%).

Molecular formula: $C_{19}H_{21}N_9O_2S$

Molecular weight: 439.5

LC-MS (M/e): 440.2 ($M+H^+$)

$^1$HNMR (400MHz,$CDCl_3$):δ 9.65 (s,1H), 8.29 (s,1H), 7.76-7.51 (m,2H), 6.68 (s,1H), 4.17 (s,2H), 4.12-4.02 (m,2H), 3.51 (s,3H), 3.16-3.08 (m,2H), 2.66-2.58 (m,2H), 2.56-2.45 (m,5H).

**Example 10: Preparation of 4-methoxy-4,5'-dimethyl-N-(7-methyl-[1,2,4]triazolo[1,5-a]pyridin-6-yl)-5',7'-dihydrospiro[cycl ohexane-1,8'-imidazo[1,2-e]purin]-2'-amine (Compound 15-1)**

**1. Preparation of 8-methyl-1,4-dioxaspiro[4.5]decan-8-ol**

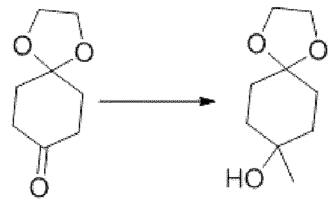

1,4-dioxaspiro[4.5]decan-8-one (25.0 g, 0.16 mol) was dissolved in THF (300 mL), the system was cooled to 0°C and then ether solution of 3M methyl magnesium bromide (100.0 mL, and 0.30 mol) was dropwise added. After dropping, the temperature was raised to 25°C and it was reacted for 5.0 hours. After that, water was added to quench the reaction, and it was concentrated and purified by a column chromatography (EA: PE=50%), to obtain a target compound (13.0 g, yield: 47.1 %).

**2. Preparation of 8-methoxy-8-methyl-1,4-dioxaspiro[4.5]decane**

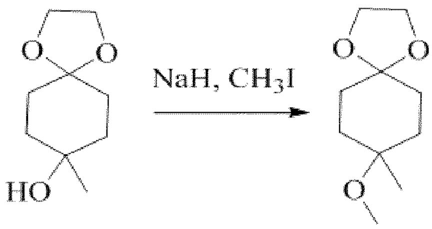

8-methyl-1,4-dioxaspiro[4.5]decan-8-ol (12.0 g, 69.7 mmol) was dissolved in THF (100 mL). 60% NaH (14.0 g, 350.0 mmol) was added to the system, and after adding, it was reacted at 10°C for 2 hours, and then $CH_3I$ (50.0 g, and 352.0 mmol) was added and it was continuously reacted for 5.0 hours. After that, water was added to quench the reaction, and it was concentrated and purified by a column chromatography (EA: PE=15%) to obtain a target compound (10.0 g, yield: 76.9%).

**3. Preparation of 4-methoxy-4-methylcyclohexan-1-one**

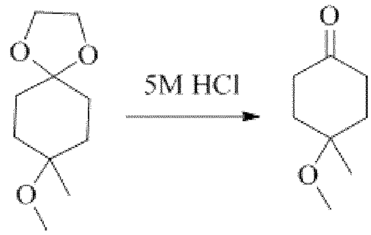


8-methoxy-8-methyl-1,4-dioxaspiro[4.5]decane (10.0 g, 53.7 mmol) was dissolved in THF (100 mL), 5M HCl solution was added at 25°C and it was reacted for 2.0 hours. It was neutralized with sodium carbonate solution until pH is 7, and then it was extracted, concentrated and purified by a column chromatography (EA: PE=20%) to obtain a target compound (6.3 g, yield: 82.9%).

**4. Preparation of 1-amino-4-methoxy-4-methylcyclohexane-1-carbonitrile**

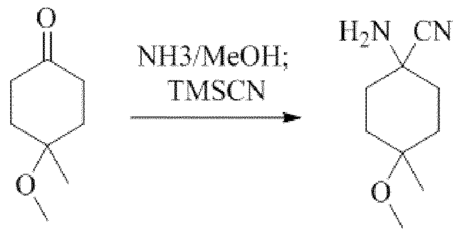


4-methoxy-4-methylcyclohexan-1-one (6.3 g, 44.3 mmol) was dissolved in 7M ammonia methanol solution (40.0 mL), it was stirred at 25°C for 3.0 hours, then trimethylsilyl cyanide (45.0 g, and 45.4 mmol) was dropwise added, it was continuously stirred for 5.0 h, and then it was concentrated and directly used for the next reaction.

**5. Preparation of 2'-chloro-4-methoxy-4,5'-dimethyl-5',7'-dihydrospiro [cyclohexane-1,8'-imidazo[1,2-e]purine]**

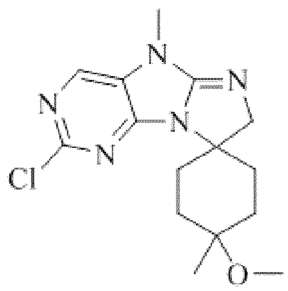

1-amino-4-methoxy-4-methylcyclohexan-1-carbonitrile was used as a starting material, and the preparation process from Step 2 to Step 6 of Example 4 (Compound 4) was referenced, to prepare 2'-chloro-4-methoxy-4,5'-dimethyl-5',7'-dihydrospiro[cyclohexane-1,8'-imidazo[1,2-e]purine].

**6. Preparation of 4-methoxy-4,5'-dimethyl-N-(7-methyl-[1,2,4]triazolo[1,5-a]pyridin-6-yl)-5',7'-dihydrospiro[cyclohexane-1,8'-imidazo[1,2-e]purin]-2'-amine**

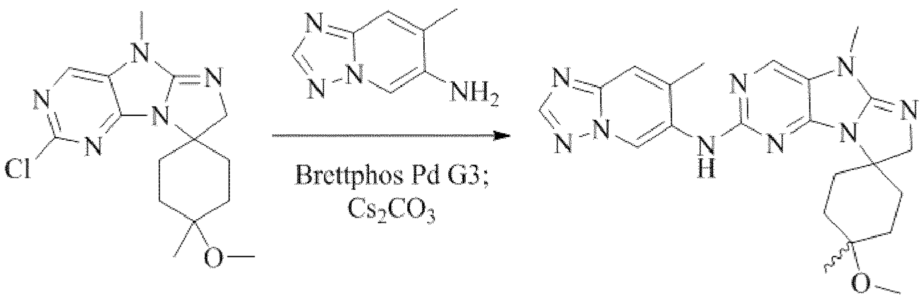


2'-chloro-4-methoxy-4,5'-dimethyl-5',7'-dihydrospiro[cyclohexane-1,8'-imidazo[1,2-e]purine] (100.0 mg, 0.31 mmol), 7-methyl-[1,2,4]triazolo[1,5-a]pyridin-6-amine (51.0 mg, 0.34 mmol), $Cs_2CO_3$ (210.0 mg, 0.64 mmol) and BrettPhos Pd G3 (60.0 mg, 0.07 mmol) were dissolved in dioxane (10.0 mL) and the system was reacted for 10.0 hours at 100°C under $N_2$. After that, it was concentrated and purified by a column chromatography (DCM:MeOH=10:1) to obtain a target product (13.0 mg, yield: 9.7%).

Molecular formula: $C_{22}H_{27}N_9O$

Molecular weight: 433.5

LC-MS (M/e): 434.0 ($M+H^+$)

$^1$H-NMR (400MHz,DMSO-d6) $\delta$: 9.71 (s,1H), 8.26 (s,1H), 7.5 (m,2H), 6.61 (s,1H), 4.11 (s,2H), 3.45 (s,3H), 3.35 (s,3H), 2.73-2.62 (t,2H), 2.51 (s,3H), 2.11-1.921 (m,2H), 1.53-1.42 (m,2H),1.25 (s,3H), 0.91-0.81 (m,2H).
HPLC retention time (RT): 3.879 min.

**Example 11: Preparation of 4-methoxy-5'-methyl-N-(7-methyl-[1,2,4]triazolo[1,5-a]pyridin-6-yl)-5',7'-dihydrospiro[cyclohe xane-1,8'-imidazo[1,2-e]purin]-2'-amine (Compounds 16-1 and 16-2)**

**1. Preparation of 2'-chloro-4-methoxy-5'-methyl-5',7'-dihydrospiro[cyclohexane-1,8'-imidazo[1,2-e]purine]**

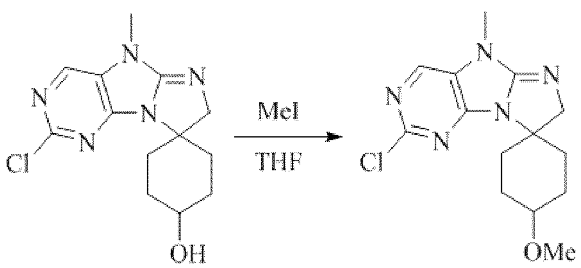


2'-chloro-5'-methyl-5',7'-dihydrospiro[cyclohexane-1,8'-imidazo[1,2-e]purin]-4-ol (190.0 mg, 0.65 mmol) was dissolved in THF (6.0 mL), 60% NaH (78.0 mg, 1.95 mmol) was added, it was stirred at 16°C for 15 minutes, iodomethane (306.5 mg, 2.2 mmol) was added, it was stirred at 60°C for 1 hour, the solvent was spin-dried and it was purified by a silica gel column chromatography (DCM/MeOH=60/1-20/1), to obtain a product (90.0 mg, yield: 45.2%).

**2. Preparation of 4-methoxy-5'-methyl-N-(7-methyl-[1,2,4]triazolo[1,5-a]pyridin-6-yl)-5',7'-dihydrospiro[cyclohexane-1,8'-imidazo[1,2-e]purin]-2'-amine**

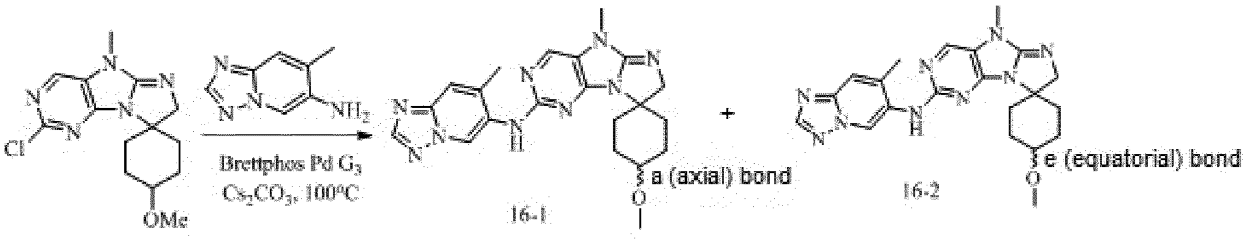


2'-chloro-4-methoxy-5'-methyl-5',7'-dihydrospiro[cyclohexane-1,8'-imidazo[1,2-e]purine] (70.0 mg, 0.23 mmol) was dissolved in dioxane (4 mL), 7-methyl-[1,2,4]triazolo[1,5-a]pyridin-6-amine (34.1 mg, 0.23 mmol), Brettphos Pd G3 (18.2 mg, 0.02 mmol), and $Cs_2CO_3$ (150.0 mg, 0.46 mmol) were added, and it was stirred for 2 hours under nitrogen protection at 100°C. It was purified by prepared TLC (DCM/MeOH=8/1), to obtain products 16-1 and 16-2.

Molecular formula: $C_{21}H_{25}N_9O$

Molecular weight: 419.5

LC-MS (M/e): 420.3 ($M+H^+$)

The retention time, yield, and hydrogen spectrum of the two products obtained were respectively as follows:

1) HPLC retention time (RT): 9.35 min (high polarity); 16.8 mg, and yield: 17.6%;
$^1$H-NMR (400MHz, $CDCl_3$) $\delta$:9.73 (s,1H), 8.26 (s,1H), 7.57 (s,2H), 6.66 (s,1H), 4.15 (s,2H), 3.77-3.53 (m,1H), 3.49 (m,3H), 3.46 (s,3H), 2.59-2.55 (m,2H), 2.52 (s,3H), 2.05-2.02 (m,2H), 1.69-1.61 (m,2H), 1.59-1.51 (m,2H).

2) HPLC retention time (RT): 9.49 min (low polarity); 18.8 mg, and yield: 19.7%;
$^1$H-NMR (400MHz, CDCl3) $\delta$:9.77 (s,1H), 8.27 (s,1H), 7.57 (s,2H), 6.56 (s,1H), 4.15 (s,2H), 3.41 (s,3H), 3.35 (s,3H), 2.51 (s,3H), 2.40-2.29 (m,2H), 2.28-2.15 (m,2H), 1.99-1.90 (m,2H), 1.45-1.35 (m,2H).

**Example 12: Preparation of 2-(5,8-dimethyl-2-((7-methyl-[1,2,4]-triazolo[1,5-a]pyridin-6-yl) amino)-7,8-dihydro-5H-imidazo[1,2-e]purin-8-yl) ethan-1-ol (Compound 17)**

**1. Preparation of 4-(benzyloxy) butan-2-one**

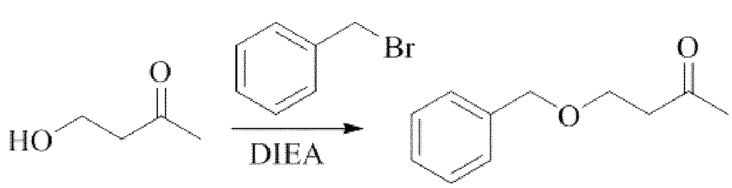


4-hydroxybutan-2-one (5.3 g, 60 mmol) was dissolved in benzyl bromide (15.4 g, 90 mmol), DIEA (15.8 mL, 96 mmol) was added, the temperature was raised to 150°C and it was stirred for 2 hours, it was diluted with ethyl acetate, washed with 1M hydrochloric acid, washed with saturated salt water, and dried with anhydrous sodium sulfate. It was filtered, concentrated and purified by a column chromatography (ethyl acetate/petroleum ether=0-40%) to obtain a product (10 g, yield: 93%).

**2. Preparation of 2-amino-4-(benzyloxy)-2-methylbutanenitrile**

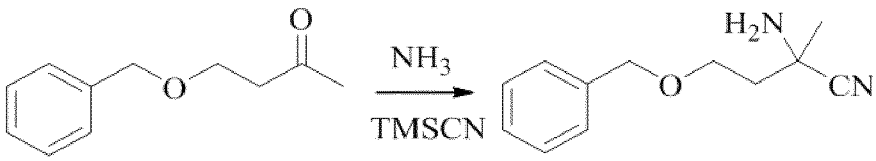


4-(benzyloxy) butan-2-one (10 g, 56 mmol) was dissolved in tetrahydrofuran solution of ammonia (27 mL), it was stirred at 0 °C for 4 hours, TMSCN (5.6 g, 56 mmol) was added, the temperature was raised to 20 °C. It was stirred for 4 hours, and then was spin-dried to obtain a product which was directly used for the next reaction.

**3. Preparation of 4-(benzyloxy)-2-((2-chloro-5-nitropyrimidin-4-yl) amino)-2-methylbutanenitrile**

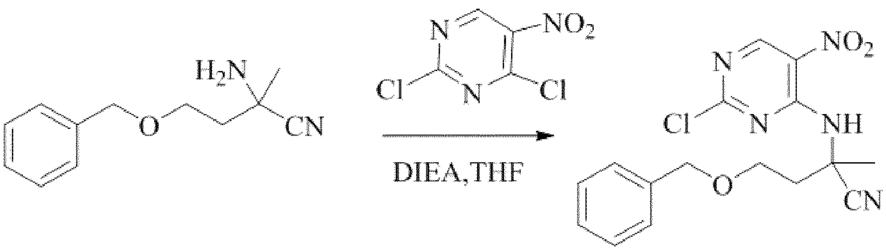


2,4-dichloro-5-nitropyrimidine (7.7 g, 40 mmol) was dissolved in THF (100 mL), it was stirred at 0°C, 2-amino-4-(benzyloxy)-2-methylbutanenitrile (a crude product from the previous step) and DIEA (10.3 g, 80 mmol) were added, the temperature was raised to 20 °C and it was continuously stirred for 1 hour. It was spin-dried directly for the next reaction.

**4. Preparation of 2-((5-amino-2-chloropyrimidin-4-yl) amino)-4-(benzyloxy)-2-methylbutanenitrile**

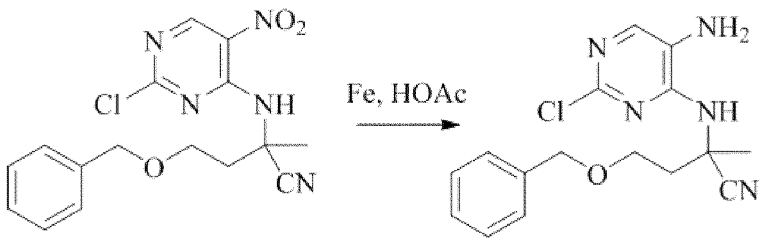


4-(benzyloxy)-2-((2-chloro-5-nitropyrimidin-4-yl)amino)-2-methylbutanenitrile (crude product) was dissolved in EtOH (50 ml), iron powder (9 g, 160 mmol), glacial acetic acid (50 mL), and water (50 mL) were added, the temperature was raised to 70 °C and it was stirred for 1 hour. It was filtered, and water (50 mL) was added. It was adjusted to pH>7 with NaOH aqueous solution, and extracted with dichloromethane. The organic phase was spin-dried and purified by a silica gel column chromatography (ethyl acetate/petroleum ether=50-90%), to obtain a product (3.3 g, three-step yield: 17.7%).

**5. Preparation of 4-(benzyloxy)-2-(2-chloro-8-thioxo-7,8-dihydro-9H-purin-9-yl)-2-methylbutanenitrile**

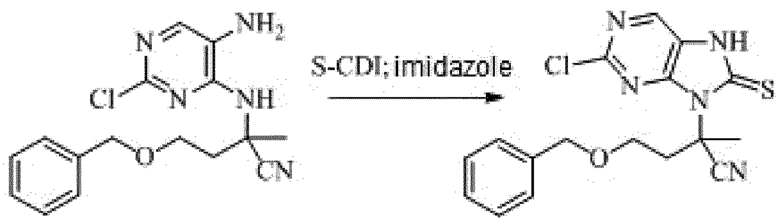


2-((5-amino-2-chloropyrimidin-4-yl)amino)-4-(benzyloxy)-2-methylbutanenitrile (3.3 g, 9.9 mmol) was dissolved in DCM (50 mL), imidazole (1 g, 14.9 mmol) and S-CDI (1.9 g, 10.9 mmol) were added. It was stirred at 25 °C for 1 hour, and filtered to obtain a product (540 mg, yield: 15%).

**6. Preparation of 8-(2-(benzyloxy) ethyl)-2-chloro-8-methyl-7,8-dihydro-6H-imidazo[1,2-e]purine**

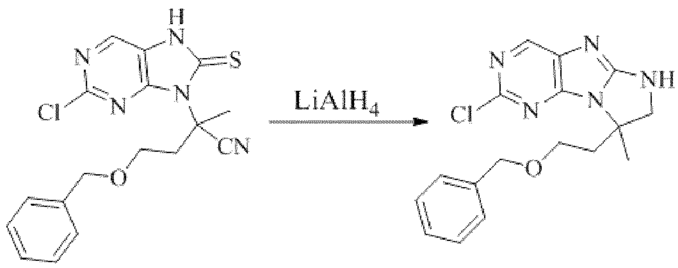


4-(benzyloxy)-2-(2-chloro-8-thioxo-7,8-dihydro-9H-purin-9-yl)-2-methylbutanenitrile (540 mg, 1.4 mmol) was dissolved in THF (15 mL), $LiAlH_4$ (165 mg, 4.3 mmol) was added, and it was stirred at 40°C for 30 minutes. Water was added for quenching the reaction. It was spin-dried and purified by a silica gel column chromatography (DCM/MeOH=20/1), to obtain a product (230 mg, yield: 46%).

**7. Preparation of 8-(2-(benzyloxy) ethyl)-2-chloro-5,8-dimethyl-7,8-dihydro-5H-imidazo[1,2-e]purine**

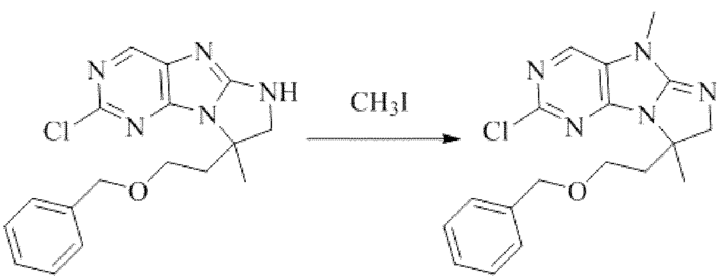


8-(2-(benzyloxy)ethyl)-2-chloro-8-methyl-7,8-dihydro-6H-imidazo[1,2-e]purine (230 mg, 0.67 mmol) was dissolved in acetonitrile (5 mL), iodomethane (1.9 g, 13.4 mmol) was added. It was stirred at 100 °C under microwave for 1 hour, spin-dried and purified by a silica gel column chromatography (MeOH/DCM=0-10%), to obtain a product (200 mg, yield: 83%).

**8. Preparation of 8-(2-(benzyloxy) ethyl)-5,8-dimethyl-N-(7-methyl-[1,2,4]triazolo[1,5-a]pyridin-6-yl)-7,8-dihydro-5H-imidazo[1,2-e]purin-2-amine**

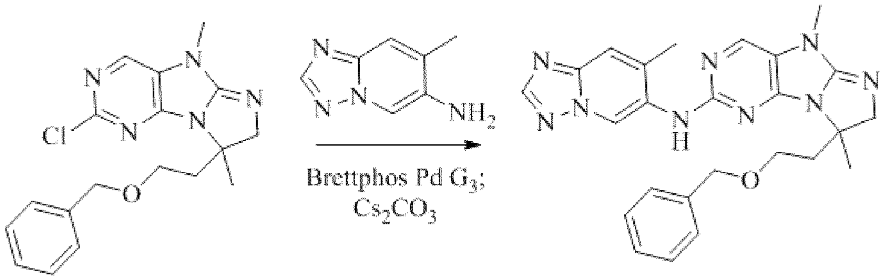


8-(2-(benzyloxy)ethyl)-2-chloro-5,8-dimethyl-7,8-dihydro-5H-imidazo[1,2-e]purine (200 mg, 0.56 mmol) was dissolved in dioxane (10 mL), 7-methyl-[1,2,4]triazolo[1,5-a]pyridin-6-amine (100 mg, 0.67 mmol), Brettphos Pd G3 (153 mg, 0.17 mmol), and $Cs_2CO_3$ (438 mg, 1.3 mmol) were added, and it was stirred under nitrogen protection at 110 °C for 24 hours. The solvent was spin-dried and it was purified by a silica gel column chromatography (MeOH/DCM=0-10%), to obtain a product (190 mg, yield: 72%).

**9. Preparation of 2-(5,8-dimethyl-2-((7-methyl-[1,2,4]-triazolo[1,5-a]pyridin-6-yl) amino)-7,8-dihydro-5H-imidazo[1,2-e]purin-8-yl) ethan-1-ol**

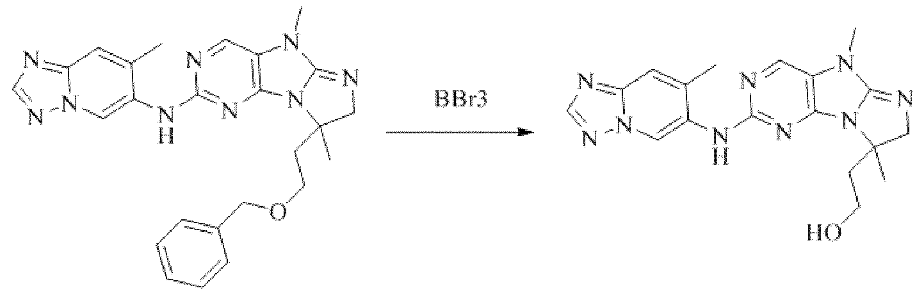


8-(2-(benzyloxy)ethyl)-5,8-dimethyl-N-(7-methyl-[1,2,4]triazolo[1,5-a]pyridin-6-yl)-7,8-dihyd ro-5H-imidazo[1,2-e]purin-2-amine (60 mg, 0.13 mmol) was dissolved in dichloromethane (10 mL), 1M BBrs (390 uL, 0.39 mmol) was added, it was stirred at 20 °C for 1 hour, and methanol was added for quenching the reaction. It was washed with saturated sodium bicarbonate, extracted with the dichloromethane, and dried with anhydrous sodium sulfate, and separated by prepared TLC plate (MeOH/DCM=1/3), to obtain a product (13 mg, yield: 26%).

Molecular formula: $C_{18}H_{21}N_9O$

Molecular weight: 379.4

LC-MS (M/e): 380.2 ($M+H^+$)

$^1$H-NMR (400MHz,MeOD) δ: 9.37 (s,1H), 8.32 (s,1H), 7.74 (s,1H), 7.64 (s,1H), 4.25-3.97 (m,2H), 3.85-3.65 (m,2H), 3.32 (s,3H), 2.52 (s,3H), 2.37-2.02 (m,2H), 1.73 (s,3H).

**Example 13: Preparation of 5'-methyl-N-(7-methyl-[1,2,4]-triazolo[1,5-a]pyridin-6-yl)-4,5,5',7'-tetrahydro-2H-spiro[furan-3, 8'-imidazo[1,2-e]purin]-2'-amine (Compound 18)**

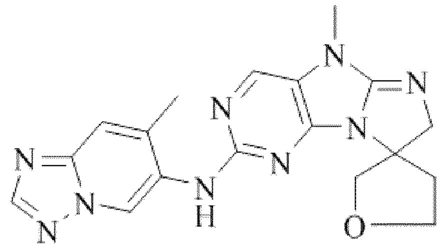

Dihydrofuran-3-(2H)-one was used as a starting material, and the preparation process of Example 4 (Compound 4) was referenced, to prepare a target compound 5'-methyl-N-(7-methyl-[1,2,4]triazolo[1,5-a]pyridin-6-yl)-4,5,5',7'-tetrahydro-2H-spiro[furan-3,8'-imid azo[1,2-e]purin]-2'-amine.

Molecular formula: $C_{18}H_{19}N_9O$

Molecular weight: 377.2

LC-MS (M/e): 378.2 (M+H$^+$)

$^1$H-NMR (400MHz,$CDCl_3$) δ: 9.84 (s,1H), 8.27 (s,1H), 7.63 (s,1H), 7.56 (s,1H), 6.65 (s,1H), 4.48-4.40 (m,1H), 4.37 (s,2H), 4.31 (d,1H), 4.12-4.10 (m,1H), 3.84 (d,1H), 3.5 (s,3H), 2.77-2.71 (m, 1H), 2.5 (s,3H), 2.26-2.17 (m, 1H).

**Example 14: Preparation of 5-methyl-N-(7-methyl-[1,2,4]triazolo[1,5-a]pyridin-6-yl)-5,7-dihydrospiro[imidazo[1,2-e]purine -8,4'-oxepan]-2-amine (Compound 19)**

**1. Preparation of 4-oxepanone**

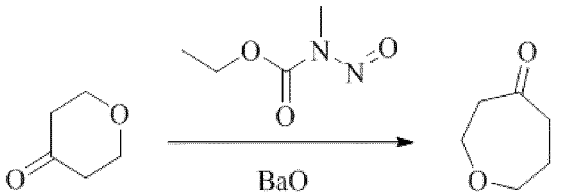


Tetrahydro-4H-pyran-4-one (1.0 g, 10.0 mmol) was dissolved in methanol (10.0 mL), barium oxide (0.3 g, 1.96 mmol) and N-methyl-N-nitroso ethyl formate (1.3 g, 9.8 mmol) were added at 0°C, and the temperature was raised to 25°C. It was stirred for 10 hours, and concentrated by a column chromatography (EA/PE=50%) to obtain a product (450 mg, yield: 39.4%).

**2. Preparation of 4-aminooxepane-4-carbonitrile**

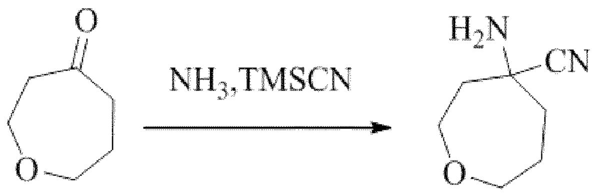


4-oxepanone (3.4 g, 29.8 mmol) was dissolved in 7M ammonia/methanol solution (17.0 mL), and it was reacted for 5 h at 25 °C, then trimethylsilyl cyanide (3.0 g, 30.2 mmol) was added. It was stirred for 10 h, and concentrated to obtain a product which was directly used for the next reaction.

**3. Preparation of 4-((2-chloro-5-nitropyrimidin-4-yl) amino) oxepane-4-carbonitrile**

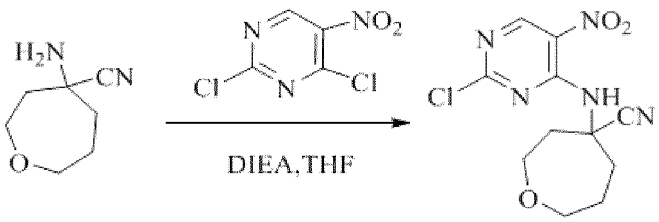


A crude product in the previous step of 4-aminooxepane-4-carbonitrile was dissolved in THF (50 mL), DIEA (7.9 g, 61.2 mmol) and 2,4-dichloro-5-nitropyrimidine (5.8 g, 29.9 mmol) were added. It was reacted at 25 °C for 2 hours, suction-filtered, and dried, to obtain a product (5.5 g, two-step yield: 62.1%).

**4. Preparation of 4-((5-amino-2-chloropyrimidin-4-yl) amino) oxepane-4-carbonitrile**

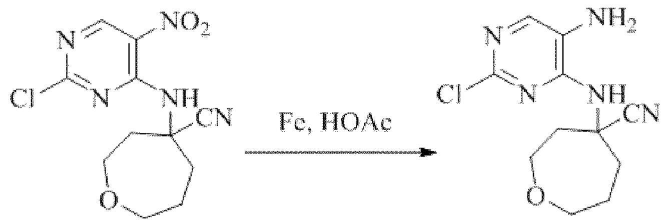


4-((2-chloro-5-nitropyrimidin-4-yl) amino) oxepane-4-carbonitrile (4.5 g, 15.1 mmol) and iron powder (4.5 g, 80.4 mmol) were dissolved in a mixed solvent of acetic acid (40 mL), ethanol (40 mL) and water (40 mL). It was reacted for 2 hours at 80°C, and suction-filtered, and a filter cake was washed with water, and dried, to obtain a product (3.8 g, yield: 95%).

**5. Preparation of 4-(2-chloro-8-thioxo-7,8-dihydro-9H-purin-9-yl) oxepane-4-carbonitrile**

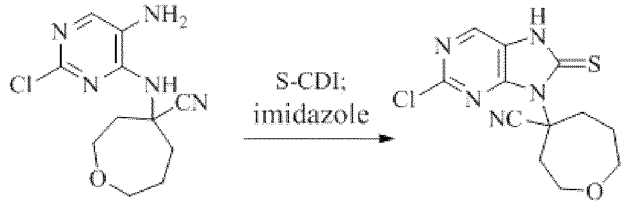


4-((5-amino-2-chloropyrimidin-4-yl)amino)oxepane-4-carbonitrile (2.2 g, and 8.22 mmol) and imidazole (1.1 g, 16.44 mmol) were dissolved in DCM (40 mL), and then S-CDI (2.9 g, and 16.44 mmol) was added. It was reacted for 4 hours at 25 °C, and the reaction was completed by an LCMS detection. It was adjusted with a dilute hydrochloric acid (2M) until pH is 5, and a solid was precipitated. It was suction-filtered, and a filter cake was washed with water and dried, to obtain a product (1.2 g, yield: 47.2%).

**6. Preparation of 2-chloro-6,7-dihydrospiro[imidazo[1,2-e]purine-8,4'-oxepane]**

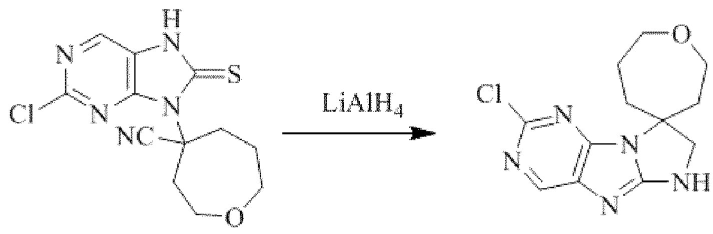


4-(2-chloro-8-thioxo-7,8-dihydro-9H-purin-9-yl)oxepane-4-carbonitrile (1.05 g, 3.40 mmol) was dissolved in THF (15 mL), $LiAlH_4$ (383 mg, 10.08 mmol) was added in batches at 40°C, and the reaction was continued for 20 min. After the reaction was completed by an LCMS detection, water (3 mL) was added to quench the reaction. It was suction-filtered. The filter cake was washed with (MeOH:DCM=1:10), and the filtrate was spin-dried, and separated by a silica gel column chromatography (MeOH:DCM=1:10) to obtain a product (450 mg, yield: 47.3%).

**7. Preparation of 2-chloro-5-methyl-5,7-dihydrospiro[imidazo[1,2-e]purine-8,4'-oxepane]**

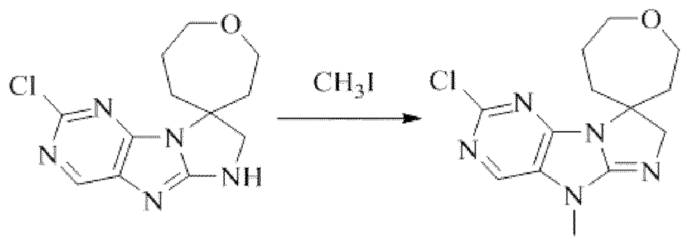


2-chloro-6,7-dihydrospiro[imidazo[1,2-e]purine-8,4'-oxepane] (260 mg, 0.93 mmol) was dissolved in acetonitrile (13 mL), iodomethane (1.5 mL, 24 mmol) was added, and it was reacted for 1 hour at 100°C. After the reaction was completed by an LCMS detection, saturated NaHCOswas added for a quenching reaction, and the reaction solution was spin-dried, and separated by a silica gel column chromatography (MeOH:DCM=1:10) to obtain a product (100 mg, yield: 36.6%).

**8. Preparation of 5-methyl-N-(7-methyl-[1,2,4]triazolo[1,5-a]pyridin-6-yl)-5,7-dihydrospiro[imidazo[1,2-e]purine -8,4'-oxepane]-2-amine**

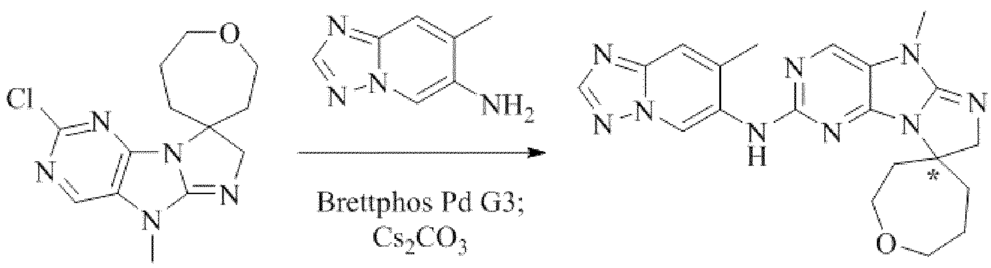


2-chloro-5-methyl-5,7-dihydrospiro[imidazo[1,2-e]purine-8,4'-oxepane] (100 mg, 0.34 mmol), 7-methyl-[1,2,4]triazolo[1,5-a]pyridin-6-amine (53 mg, 0.36 mmol), Brettphos Pd G3 (36 mg, 0.04 mmol) and caesium carbonate (280 mg, 0.86 mmol) were dissolved in 1,4-dioxane (10 mL), and it was reacted at 100 °C for 2 hours under nitrogen protection. After the reaction was completed by an LCMS detection, reaction solution was spin-dried, and separated by a silica gel column chromatography (MeOH: DCM=1:10) to obtain a crude product, and then, a mixed solvent of MeOH: hexane: DCM (2 mL: 20 mL: 2 mL) was added to obtain an insoluble substance by ultrasound. It was filtered under a reduced pressure and the filter cake was dried to obtain a product (63.7 mg, yield: 46.2%).

Molecular formula: $C_{20}H_{23}N_9O$

Molecular weight: 405.5

LC-MS (M/e): 406.2 ($M+H^+$)

$^1$H-NMR (400MHz, $CDCl_3$) δ: 9.77 (s,1H), 8.27 (s,1H), 7.56-7.60 (m,2H), 6.57 (s,1H), 4.12-4.22 (m,2H), 3.95-4.05 (m,1H), 3.80-3.90 (m,2H), 3.70-3.79 (m,1H), 3.37 (s,3H), 2.52-2.62 (m,2H), 2.61 (s,3H), 2.00-2.16 (m,3H), 1.75-1.85 (m,1H).

**Chiral isomer resolution**

Resolution method: High performance liquid chromatography (chiral column)

| Chromatographic column model | HC-C8 | Flow rate | 1.0mL/min |
|---|---|---|---|
| Chromatographic column specification | 4.6×250mm, 5μm | Wavelength | 214nm |
| Injection volume | 10μL | Column temperature | 35°C |
| Mobile phase and ratio | N-hexane: anhydrous ethanol: diethylamine =50:50:01 | | |

Resolution result:

Two compounds were obtained, and the retention time, enantiomeric excess (ee) value, and corresponding hydrogen spectrum were respectively as follows:

| | Retention time (min) | ee value (%) | $^1$H-NMR (400MHz, $CDCl_3$) |
|---|---|---|---|
| (1) | 13.20 | 100% | δ: 9.77 (s, 1H), 8.27 (s, 1H), 7.56-7.60 (m, 2H), 6.57 (s, 1H), 4.12-4.22 (m, 2H), 3.95-4.05 (m, 1H), 3.80-3.90 (m, 2H), 3.70-3.79 (m, 1H), 3.37 (s, 3H), 2.52-2.62 (m, 2H), 2.61 (s, 3H), 2.00-2.16 (m, 3H), 1.75-1.85 (m, 1H). |
| (2) | 16.60 | 97.84% | δ: 9.77 (s, 1H), 8.27 (s, 1H), 7.56-7.60 (m, 2H), 6.57 (s, 1H), 4.12-4.22 (m, 2H), 3.95-4.05 (m, 1H), 3.80-3.90 (m, 2H), 3.70-3.79 (m, 1H), 3.37 (s, 3H), 2.52-2.62 (m, 2H), 2.61 (s, 3H), 2.00-2.16 (m, 3H), 1.75-1.85 (m, 1H). |

**Example 15: Preparation of 5-methyl-2-((7-methyl-[1,2,4]-triazolo[1,5-a]pyridin-6-yl) amino)-2',3',5,5',6',7-hexahydrospiro[imidazo[1,2-e]purine-8,4'-thiopyran]-1'-oxide (Compound 20)**

Referenced to the method of Example 9, in the first step of the oxidation reaction, the amount of mCPBA used was 1.2 equivalents.

The compounds shown in the following table were prepared by using the same or similar methods as the above examples:

| Number | Structure | LC-MS (M/e, M+H$^+$) | Number | Structure | LC-MS (M/e, M+H$^+$) |
|---|---|---|---|---|---|
| 10 | 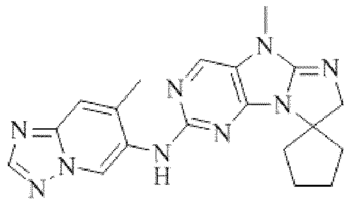 | 376.2 | 11 | 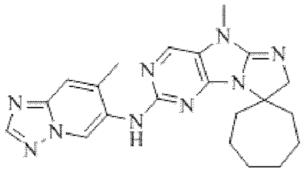 | 404.2 |
| 12 | 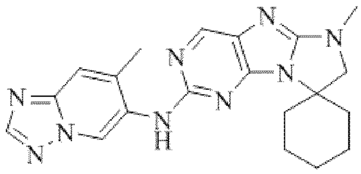 | 390.2 | 13 | 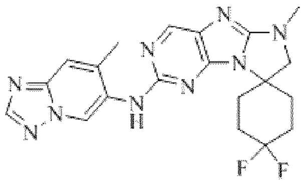 | 426.2 |
| 14 | 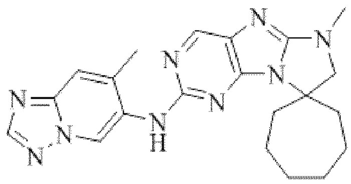 | 404.2 | | | |

**Experimental scheme**

Exemplary experimental schemes for some compounds of the present disclosure are provided below, to demonstrate the beneficial activities and technical effects of the compounds of the present disclosure. However, it should be understood that the following experimental schemes are only examples of the content of the present disclosure, and not limitation to the scope of the present disclosure.

**Experimental example 1: In vitro enzymatic activity of compounds of the present invention**

Abbreviation

EDTA: ethylenediaminetetraacetic acid

DMSO: dimethyl sulfoxide

Tris: trihydroxymethyl aminomethane

Brij-35: lauryl alcohol polyoxyethylene ether

DTT: dithiothreitol

Test substances: the compounds of the present disclosure, whose structural formulas and preparation methods are shown in the Examples.

Experimental reagent:

| Name | Brand |
|---|---|
| ADP-Glo Kinase Assay | Promege |
| DNA-PK | Promege |

Experimental method:

1. Preparation of 1-fold kinase buffer

1) 1-fold kinase buffer

40 mM Tris, pH 7.5

0.0055% Brij-35

20 mM $MgCl_2$

0.05 mM DTT

2. Compound preparation

1) The detection starting concentration of the compound was 1 μM, and it was prepared to a 100-fold concentration, namely 100 μM. 2 μl of 10 mM compound was taken, 198 μl of 100% DMSO was added, and it was prepared into 100 μM compound solution. 100 μl of the compound of 100-fold concentration was added to a second well on a 96-well plate, and 60 μl of 100% DMSO was added to other wells. 30 μl of the compound was taken from the second well and added to a third well, it was diluted downwards sequentially by 3 times, and there was a total of 10 concentrations diluted.

2) 100 μl of 100% DMSO and positive control wortmannin with the highest concentration (400 nM) were transferred to two empty wells as a Max well and a Min well respectively.

3) 50 nl of the compound was transferred to a 384-well plate by Echo.

3. Preparation of 2x kinase solution

1) The 1-fold kinase buffer was used to prepare 2-fold DNA-PK kinase solution.

2) 2.5 μl of the 2-fold kinase solution was transferred to a reaction well of the 384-well plate.

3) It was shaken, mixed uniformly, and stilly placed at a room temperature.

4. Preparation of 2x substrate solution

1) The 1-fold kinase buffer was used to prepare 2-fold substrate solution.

2) 2.5 μl of the 2-fold substrate solution was transferred to the reaction well of the 384-well plate for a starting reaction.

3) It was shaken, and mixed uniformly.

5. Kinase reaction and termination

1) The 384-well plate was covered by a cover, and incubated at 28°C for 3 hours.

2) 5 μl of ADP-Glo reagent was transferred, and incubated at 28°C for 2 hours.

6. Detection of reaction result

1) 10 μl of a kinase detection reagent was transferred into the reaction well of the 384-well plate for terminating the reaction.

2) It was stilly placed at the room temperature for 30 minutes.

7. Data reading

Sample values were read on Envision.

8. Inhibition rate calculation

1) Data was copied from Envision.

2) It was converted into inhibition rate data.
Inhibition percentage= (max-conversion) / (max-min) *100. Wherein, max refers to the conversion rate of a DMSO control, min refers to the conversion rate of a control without enzyme activity, and conversion refers to the conversion rate of the test compound at each concentration.

3) The data was imported into MS Excel and XLFit excel add-in version 5.4.0.8 was used for curve fitting.

Experimental result:

Table 1: In vitro enzymatic activity data of compound of present disclosure

| Compound | DNA-PK $IC_{50}$ (nM) | Compound | DNA-PK $IC_{50}$ (nM) |
|---|---|---|---|
| Compound 1 | 0.82 | Compound 2 | 22 |
| Compound 3 | 10.6 | Compound 4 | 0.8 |
| Compound 5 | 1.63 | Compound 6 | 4.8 |
| Compound 7-1 | 0.62 | Compound 7-2 | 0.72 |
| Compound 8 | 0.89 | Compound 9 | 21 |
| Compound 15-1 | 1.37 | Compound 16-1 | 3.0 |
| Compound 16-2 | 2.2 | Compound 17 | 9.8 |
| Compound 18 | 1.4 | Compound 19 | 1.6 |
| Compound 19-1 | 1.1 | Compound 19-2 | 1.7 |
| Compound 20 | 18.7 | | |

Experimental conclusion:

The compounds of the present disclosure have the better inhibitory effects on the activity of DNA-PK kinase. The compounds 1-20 specifically implemented all have the lower $IC_{50}$ values.

**Experimental example 2: Metabolic stability experiment of compounds of present disclosure in different species of liver microsomes**

Test substance: the compounds of the present disclosure were self-made, and their chemical names and preparation methods were shown in the preparation examples of the compounds.

Experimental materials:

Mixed liver microsomes of cynomolgus monkeys, and the protein concentration of the liver microsomes was 20 $mg{\cdot}mL^{-1}$.
Mixed liver microsomes of SD rats and CD-1 mice, and the protein concentrations of the liver microsomes were both 20 $mg{\cdot}mL^{-1}$.
Mixed liver microsomes of humans, and the protein concentration of the liver microsomes was 20 $mg{\cdot}mL^{-1}$.
Experimental initiation factor β-NADPH was purchased from Solarbio Company; and phosphate buffer solution (PBS) with pH 7.4 was self-made by this laboratory.

Preparation of test substance solution:

An appropriate amount of test compound powder was accurately weighed, an appropriate amount of dimethyl sulfoxide (DMSO) was added and dissolved to 1 mM, and then it was diluted to 50 μM of working solution by 20 times with methanol.

Experimental method:

Table 2: Composition of the incubation system for liver microsome metabolic stability experiment

| Substances to be added | Initial concentration | Proportion (%) | Final concentration |
|---|---|---|---|
| Phosphate buffer solution | 100 mM | 50 | 50 mM |
| Anhydrous magnesium chloride | 20 mM | 5 | 1 mM |
| Liver microsome | 20 mg protein/mL | 2.5 | 0.5 mg protein/mL |
| Water to be replenished | -- | 30.5 | -- |
| Test substance | 50 μM | 2 | 1 μM |
| β-NADPH | 10 mM | 10 | 1 mM |

Experimental operating steps:

(1) According to the proportion in Table 2 "Composition of experimental incubation system" above, 5.85 mL of 100 mM PBS, 0.585 mL of 20 mM $MgCl_2$ solution and 3.57 mL of $H_2O$ were taken for each compound, and Mixed solution 1 of the incubation system (excluding microsomes, test compounds and β-NADPH) was prepared. At the same time, positive drug Verapamil of the experimental incubation system was used to prove that the liver microsome enzyme activity was normal in this experiment.

(2) The liver microsomes (20 mg protein/mL) were taken out from a -80 °C refrigerator, and placed on a 37°C water bath thermostatic oscillator to pre-incubate for 3 min.

(3) 1.9 mL of Mixed solution 1 of the incubation system was taken for each compound and each specie, and 56 μL of microsomes of different species were added, to prepare Mixed solution 2 of the incubation system (excluding the test compounds and β-NADPH).

(4) Sample group (containing the microsomes and β-NADPH): 616 μL of mixed solution 2 of the incubation system was taken, 14 μL of working solution of the test compound with a concentration of 50 μM was added, and 70 μL of working solution of 10 mM β-NADPH was added. It was mixed uniformly, and a sample was duplicated. The sampling time points were 0 min, 5 min, 10 min, 20 min, 30 min, and 60 min. This sample group was used to evaluate the metabolic stability of the compounds mediated by β-NADPH.

(5) Control group (containing the microsomes, excluding β-NADPH, and β-NADPH was replaced by water): 264 μL of Mixed solution 2 of the incubation system was taken, 6 μL of working solution of 50 μM test compound was added, and 30 μL of the water was added. It was mixed uniformly, and a sample was duplicated. The sampling time points were 0 min and 60 min. The negative control group was used to evaluate whether the compound in the liver microsome incubation system has non-β-NADPH mediated metabolism.

(6) 50 μL of the sample was taken from an incubation sample tube at each predetermined time point, and added to a termination sample tube (containing 300 μL of a cold terminating agent, and acetonitrile solution containing 50 ng/mL of internal standard tolbutamide), and vortex was performed, to terminate the reaction.

(7) After the vortex for 10 min, it was centrifuged for 5 min (12000 rpm).

(8) 100 μL of a supernatant was taken, and 100 μL of water was added. It was mixed uniformly by the vortex, and analyzed by LC-MS/MS.

Data analysis:

The ratio of the peak area of the test compound to that of the internal standard according to the following formula was converted into the remaining percentage.

$$\text{\% remaining amount} = \frac{\text{ratio of the peak area of test compound at any one time point to that of internal standard}}{\text{ratio of the peak area of test compound at time 0 to that of internal standard}} \times 100\%$$

Experimental result:

The compounds of the present disclosure have good stabilities in the liver microsomes of the tested species.

Experimental example 3: In vivo pharmacokinetic experiment of compounds of present disclosure in CD1 mouse

Test substances: the compounds of the present disclosure were self-made, and their chemical names and preparation methods were shown in the preparation examples of each compound.

Test animals: CD1 mice, female, were purchased from Beijing Weitong Lihua Experimental Animal Technology Co., Ltd., 6 animals/each compound/one route of administration.

Preparation of test substance solution:

Preparation method of blank vehicle (1): 28 g of HP-β-CD was weighed, and dissolved in an appropriate amount of injection water, then the volume was fixed to 100 mL with the injection water. It was mixed uniformly by vortex, to obtain 28% HP-β-CD.

Preparation method of blank vehicle (2): 20 g of HPC was weighed, and added slowly to 500 mL of purified water stirred, then 1 mL of Tween 80 was added. It was stirred until it was clear and transparent, then the volume was fixed to 1000 mL, and it was stirred evenly to obtain 2% HPC + 0.1% Tween 80.

Intravenous injection (IV) administration:

An appropriate amount of the compound of the present disclosure was weighed, DMA (10%-20%, v/v) was added, it was dissolved by vortex, then PEG400 (5%-10%, v/v) (the amounts of DMA and PEG400 might be appropriately

increased or decreased according to the solubility of the specific compound) was added, and it was mixed uniformly by vortex. Finally, the blank vehicle (1) (an appropriate amount, if the compound was difficult to dissolve in the blank vehicle (1), acid solution or alkali solution with certain pH might be used as a substitute) was added, and it was mixed uniformly by vortex. The temperature was kept at 50 °C for 10-20 min, and clear solution with an appropriate concentration (such as 0.2 mg/mL, and 1 mg/mL) was prepared, as IV administration solution of the compound tested.

Peros PO (gavage) administration:

The compound (appropriate amount) of the present disclosure was weighed, and placed in a tissue grinder, the blank vehicle (2) (appropriate amount) was added. It was ground evenly at a rotation speed of 1000 rpm, and suspension solution with an appropriate concentration (such as 1 mg/mL, and 5mg/mL) was prepared, as PO administration solution of the compound tested.

Experimental method

The IV administration volume was 5 mL/kg, and if the IV administration dose of Compound was 1 mg/kg, the administration concentration was 0.2 mg/mL; and if the IV administration dose of Compound was 5 mg/kg, the administration concentration was 1 mg/mL.

The PO administration volume was 10 mL/kg, and if the PO administration dose of the compound was 10 mg/kg, the administration concentration was 1 mg/mL; and if the PO administration dose of the compound was 50 mg/kg, the administration concentration was 5 mg/mL.

Blood collection time point: 0.083, 0.25, 0.5, 1, 2, 4, 6, 8, and 24 h after administration, and blood was specifically collected according to modes shown in the following table:

| Group | Number (animals) | Blood collection time point |
|---|---|---|
| IV | 3 | 0.083h, 0.5h, 2h, 6h, 24h |
| | 3 | 0.25h, 1h, 4h, 8h |
| PO | 3 | 0.083h, 0.5h, 2h, 6h, 24h |
| | 3 | 0.25h, 1h, 4h, 8h |

About 50 μL of whole blood was collected by an inner canthus at each time point, and placed in an anticoagulant tube containing a EDTA-K 2 anticoagulant, it was centrifuged at 4°C for 6 min under a condition of 8000 rpm to obtain a plasma sample, and the plasma was frozen in a -80°C refrigerator for further analysis.

Plasma sample analysis

Protein precipitation method adopted: 20 μL of the plasma sample was taken, 200 μL of the internal standard (acetonitrile solution containing 50 ng/mL of tolbutamide) was added, and after vortex was performed for 10 min, it was centrifuged at 4000 rpm for 20 min. 100 μL of a supernatant was taken, and 100 μL of water was added. After it was mixed uniformly for 3 min by the vortex, the drug concentration in the plasma was analyzed by LC-MS/MS.

Experimental result and conclusion

Pharmacokinetic parameters were calculated by a drug concentration-time curve, such as exposure dose AUCo-t (representing an area under a drug-time curve 0→t), clearance rate CL, elimination half-life $T_{1/2}$, peak time $T_{max}$, peak concentration $C_{max}$, steady-state apparent distribution volume $V_{ss}$, and absolute bioavailability F%. According to the test results, it may be seen that the compounds of the present disclosure have the good pharmacokinetic properties, and have the higher exposure dose and bioavailability.

## Claims

**1.** A compound of formula (I), a pharmaceutically acceptable salt thereof, or an isomer thereof,

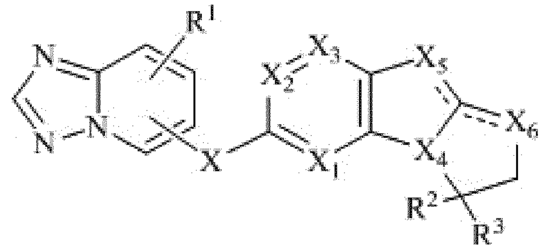

(I)

wherein,

$X_1$, $X_2$, $X_3$, and $X_4$ are respectively independently selected from C ($R^4$) or N;
$X_5$ and $X_6$ are respectively independently selected from CH ($R^5$), C ($R^6$), N ($R^7$), or N;
X is $CH_2$, NH, O, or S;
$R^1$ is selected from a group consisting of H, halogen, hydroxyl, amino, nitro, cyano, $C_{1-6}$ alkyl, $C_{1-6}$ alkyl amino, di ($C_{1-6}$ alkyl) amino, halo $C_{1-6}$ alkyl, hydroxy $C_{1-6}$ alkyl, amino $C_{1-6}$ alkyl, $C_{1-6}$ alkoxy, $C_{1-6}$ alkylthio, halo $C_{1-6}$ alkoxy, halo $C_{1-6}$ alkylthio, hydroxy $C_{1-6}$ alkoxy, hydroxy $C_{1-6}$ alkylthio, amino $C_{1-6}$ alkoxy, and amino $C_{1-6}$ alkylthio;
$R^2$ and $R^3$ are respectively independently selected from a group consisting of H, halogen, hydroxyl, amino, nitro, cyano, $C_{1-6}$ alkyl, halo $C_{1-6}$ alkyl, hydroxy $C_{1-6}$ alkyl, amino $C_{1-6}$ alkyl, $C_{1-6}$ alkoxy, halo $C_{1-6}$ alkoxy, hydroxy $C_{1-6}$ alkoxy, amino $C_{1-6}$ alkoxy, and 3-8 membered cycloalkyl or 3-8 membered heterocyclic group substituted optionally by 1-3 Q1; and each Q1 is independently selected from a group consisting of halogen, hydroxyl, amino, nitro, cyano, $C_{1-6}$ alkyl, halo $C_{1-6}$ alkyl, $C_{1-6}$ alkoxy and halo $C_{1-6}$ alkoxy;
or $R^2$, $R^3$, and the carbon atom linked to them together form 3-8 membered cycloalkyl or 3-8 membered heterocyclic group substituted optionally by 1-3 Q2; and each Q2 is independently selected from a group consisting of halogen, hydroxyl, amino, nitro, cyano, carbonyl, $C_{1-6}$ alkyl, halo $C_{1-6}$ alkyl, $C_{1-6}$ alkyl amino, $(C_{1-6}$ alkyl$)_2$ amino, $C_{1-6}$ alkoxy and halo $C_{1-6}$ alkoxy;
each $R^4$, each $R^5$, and each $R^6$ are respectively independently selected from a group consisting of H, halogen, hydroxyl, amino, nitro, cyano, $C_{1-6}$ alkyl, halo $C_{1-6}$ alkyl, hydroxy $C_{1-6}$ alkyl, amino $C_{1-6}$ alkyl, $C_{1-6}$ alkoxy, halo $C_{1-6}$ alkoxy, hydroxy $C_{1-6}$ alkoxy and amino $C_{1-6}$ alkoxy;
each $R^7$ is respectively independently selected from a group consisting of H, $C_{1-6}$ alkyl, halo $C_{1-6}$ alkyl, hydroxy $C_{1-6}$ alkyl and amino $C_{1-6}$ alkyl; and
the virtual bond --- is a chemical bond or does not exist, and the adjacent virtual bond is not a chemical bond at the same time.

**2.** The compound, the pharmaceutically acceptable salt thereof, or the isomer thereof according to claim 1, wherein it further has a structure of formula (IIa),

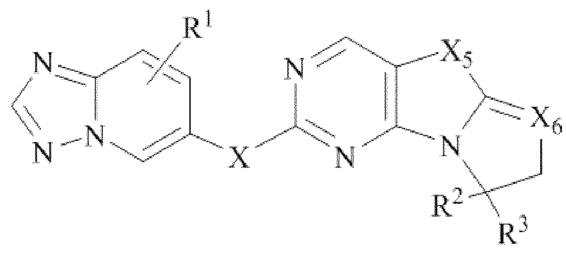

(IIa)

wherein, $X_5$ is CH ($R^5$) or N ($R^7$);
$X_6$ is C ($R^6$) or N;
X is $CH_2$, NH, O, or S;
$R^1$ is selected from a group consisting of H, halogen, hydroxyl, amino, nitro, cyano, $C_{1-6}$ alkyl, halo $C_{1-6}$ alkyl, hydroxy $C_{1-6}$ alkyl, amino $C_{1-6}$ alkyl, $C_{1-6}$ alkoxy, halo $C_{1-6}$ alkoxy, hydroxy $C_{1-6}$ alkoxy and amino $C_{1-6}$ alkoxy;
$R^2$ and $R^3$ are respectively independently selected from a group consisting of H, halogen, hydroxyl, amino, nitro, cyano, $C_{1-6}$ alkyl, halo $C_{1-6}$ alkyl, hydroxy $C_{1-6}$ alkyl, amino $C_{1-6}$ alkyl, $C_{1-6}$ alkoxy, halo $C_{1-6}$ alkoxy, hydroxy $C_{1-6}$ alkoxy, amino $C_{1-6}$ alkoxy, and 3-8 membered cycloalkyl or 3-8 membered heterocyclic group substituted optionally by 1-2 Q1; and each Q1 is independently selected from a group consisting of halogen, hydroxyl,

amino, nitro, cyano, $C_{1-6}$ alkyl, halo $C_{1-6}$ alkyl, $C_{1-6}$ alkoxy and halo $C_{1-6}$ alkoxy;

or $R^2$, $R^3$, and the carbon atom linked to them together form 3-8 membered cycloalkyl or 3-8 membered heterocyclic group substituted optionally by 1-2 Q2; and each Q2 is independently selected from a group consisting of halogen, hydroxyl, amino, nitro, cyano, carbonyl, $C_{1-6}$ alkyl, halo $C_{1-6}$ alkyl, $C_{1-6}$ alkyl amino, ($C_{1-6}$ alkyl)$_2$ amino, $C_{1-6}$ alkoxy and halo $C_{1-6}$ alkoxy;

$R^5$ and $R^6$ are respectively independently selected from a group consisting of H, halogen, hydroxyl, amino, nitro, cyano, $C_{1-6}$ alkyl, halo $C_{1-6}$ alkyl, hydroxy $C_{1-6}$ alkyl, amino $C_{1-6}$ alkyl, $C_{1-6}$ alkoxy, halo $C_{1-6}$ alkoxy, hydroxy $C_{1-6}$ alkoxy and amino $C_{1-6}$ alkoxy; and

$R^7$ is selected from a group consisting of H, $C_{1-6}$ alkyl, halo $C_{1-6}$ alkyl, hydroxy $C_{1-6}$ alkyl and amino $C_{1-6}$ alkyl.

**3.** The compound, the pharmaceutically acceptable salt thereof, or the isomer thereof according to claim 1, wherein it further has a structure of formula (IIb),

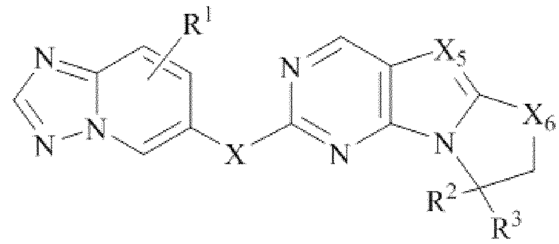

(IIb)

wherein, $X_5$ is C ($R^6$) or N;

$X_6$ is CH ($R^5$) or N ($R^7$);

Xis $CH_2$, NH, O, or S;

$R^1$ is selected from a group consisting of H, halogen, hydroxyl, amino, nitro, cyano, $C_{1-6}$ alkyl, halo $C_{1-6}$ alkyl, hydroxy $C_{1-6}$ alkyl, amino $C_{1-6}$ alkyl, $C_{1-6}$ alkoxy, halo $C_{1-6}$ alkoxy, hydroxy $C_{1-6}$ alkoxy and amino $C_{1-6}$ alkoxy;

$R^2$ and $R^3$ are respectively independently selected from a group consisting of H, halogen, hydroxyl, amino, nitro, cyano, $C_{1-6}$ alkyl, halo $C_{1-6}$ alkyl, hydroxy $C_{1-6}$ alkyl, amino $C_{1-6}$ alkyl, $C_{1-6}$ alkoxy, halo $C_{1-6}$ alkoxy, hydroxy $C_{1-6}$ alkoxy, amino $C_{1-6}$ alkoxy, and 3-8 membered cycloalkyl or 3-8 membered heterocyclic group substituted optionally by 1-2 Q1; and each Q1 is independently selected from a group consisting of halogen, hydroxyl, amino, nitro, cyano, $C_{1-6}$ alkyl, halo $C_{1-6}$ alkyl, $C_{1-6}$ alkoxy and halo $C_{1-6}$ alkoxy;

or $R^2$, $R^3$, and the carbon atom linked to them together form 3-8 membered cycloalkyl or 3-8 membered heterocyclic group substituted optionally by 1-2 Q2; and each Q2 is independently selected from a group consisting of halogen, hydroxyl, amino, nitro, cyano, carbonyl, $C_{1-6}$ alkyl, halo $C_{1-6}$ alkyl, $C_{1-6}$ alkyl amino, ($C_{1-6}$ alkyl)$_2$ amino, $C_{1-6}$ alkoxy and halo $C_{1-6}$ alkoxy;

$R^5$ and $R^6$ are respectively independently selected from a group consisting of H, halogen, hydroxyl, amino, nitro, cyano, $C_{1-6}$ alkyl, halo $C_{1-6}$ alkyl, hydroxy $C_{1-6}$ alkyl, amino $C_{1-6}$ alkyl, $C_{1-6}$ alkoxy, halo $C_{1-6}$ alkoxy, hydroxy $C_{1-6}$ alkoxy and amino $C_{1-6}$ alkoxy; and

$R^7$ is selected from a group consisting of H, $C_{1-6}$ alkyl, halo $C_{1-6}$ alkyl, hydroxy $C_{1-6}$ alkyl and amino $C_{1-6}$ alkyl.

**4.** The compound, the pharmaceutically acceptable salt thereof, or the isomer thereof according to any one of claims 1-3, wherein $R^2$, $R^3$, and the carbon atom linked to them together form the following groups substituted optionally by 1-2 Q2:

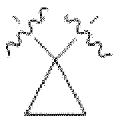 , 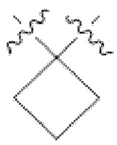 , 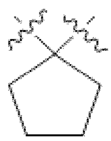 , 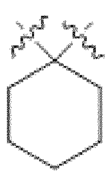 , 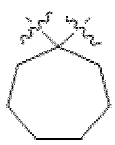 , 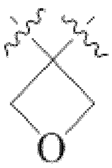 , 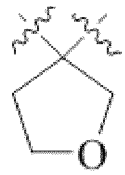 , 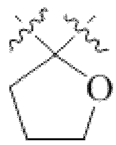 ,

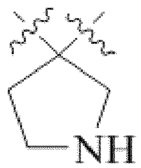, 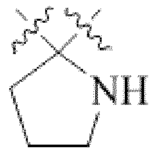, 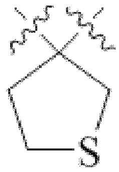, 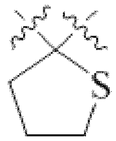, 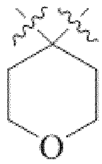, 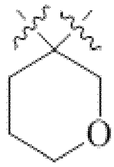, 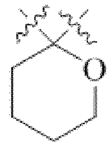, 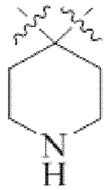,

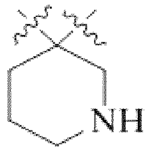, 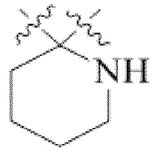, 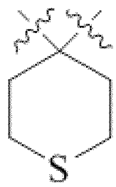, 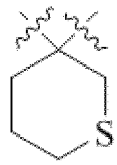, 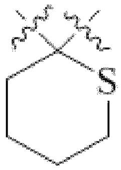, 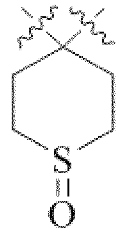, 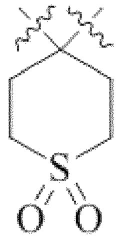,

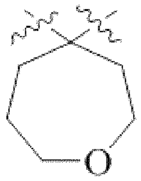, 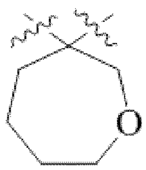, 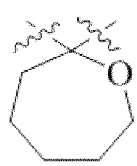, 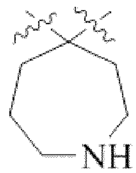, 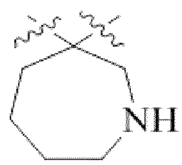, 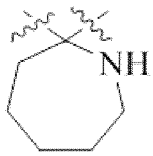, 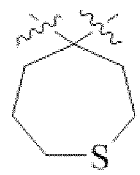,

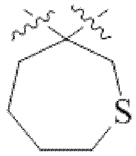 or 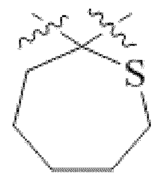;

each Q2 is independently selected from a group consisting of halogen, hydroxyl, amino, nitro, cyano, carbonyl, $C_{1-6}$ alkyl, halo $C_{1-6}$ alkyl, $C_{1-6}$ alkyl amino, $(C_{1-6}$ alkyl$)_2$ amino, $C_{1-6}$ alkoxy and halo $C_{1-6}$ alkoxy.

**5.** The compound, the pharmaceutically acceptable salt thereof, or the isomer thereof according to any one of claims 1-4, wherein,

$R^1$ is selected from a group consisting of H, halogen, hydroxyl, amino, nitro, cyano, $C_{1-6}$ alkyl, halo $C_{1-6}$ alkyl, hydroxy $C_{1-6}$ alkyl, amino $C_{1-6}$ alkyl, $C_{1-6}$ alkoxy, halo $C_{1-6}$ alkoxy, hydroxy $C_{1-6}$ alkoxy and amino $C_{1-6}$ alkoxy;
$R^2$ and $R^3$ are respectively independently selected from a group consisting of H, halogen, hydroxyl, amino, nitro, cyano, $C_{1-6}$ alkyl, halo $C_{1-6}$ alkyl, hydroxy $C_{1-6}$ alkyl, amino $C_{1-6}$ alkyl, $C_{1-6}$ alkoxy, halo $C_{1-6}$ alkoxy, hydroxy $C_{1-6}$ alkoxy and amino $C_{1-6}$ alkoxy;
or $R^2$, $R^3$, and the carbon atom linked to them together form the following groups substituted optionally by 1-2 Q2:

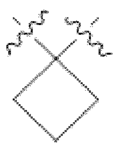, 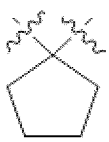, 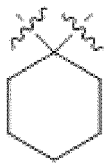, 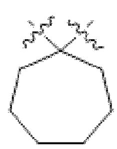, 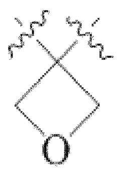, 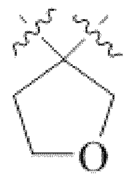, 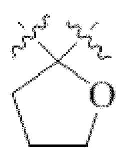, 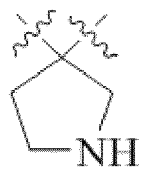,

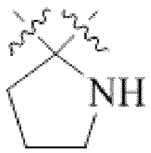, 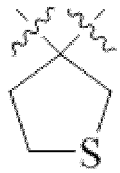, 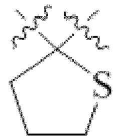, 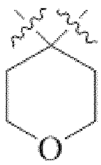, 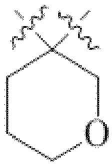, 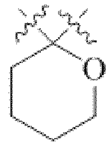, 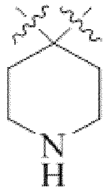, 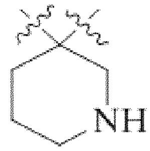,

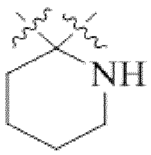, 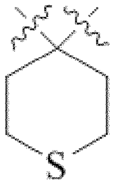, 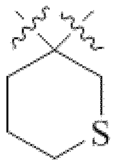, 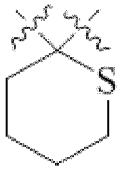, 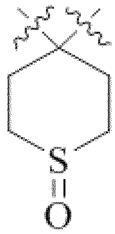, 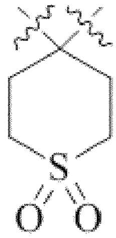, 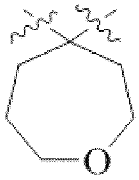,

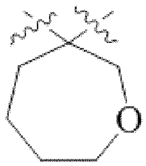, 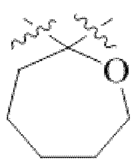, 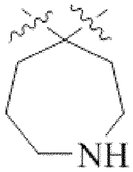, 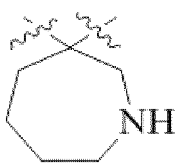, 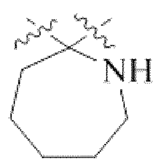, 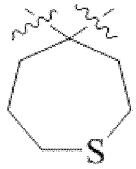, 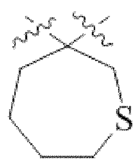

or

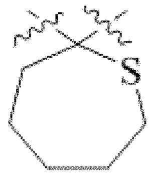;

each Q2 is independently selected from a group consisting of halogen, hydroxyl, amino, nitro, cyano, carbonyl, $C_{1-6}$ alkyl, halo $C_{1-6}$ alkyl, $C_{1-6}$ alkyl amino, ($C_{1-6}$ alkyl)$_2$ amino, $C_{1-6}$ alkoxy and halo $C_{1-6}$ alkoxy;

$R^4$ is H; $R^5$ and $R^6$ are respectively independently selected from a group consisting of H, halogen, hydroxyl, amino, nitro, cyano, $C_{1-6}$ alkyl, halo $C_{1-6}$ alkyl, hydroxy $C_{1-6}$ alkyl, amino $C_{1-6}$ alkyl, $C_{1-6}$ alkoxy and halo $C_{1-6}$ alkoxy; and

$R^7$ is selected from a group consisting of H, $C_{1-6}$ alkyl, halo $C_{1-6}$ alkyl, hydroxy $C_{1-6}$ alkyl and amino $C_{1-6}$ alkyl.

**6.** The compound, the pharmaceutically acceptable salt thereof, or the isomer thereof according to any one of claims 1-5, wherein,

$R^1$ is selected from a group consisting of H, halogen, hydroxyl, amino, nitro, cyano, methyl, ethyl, propyl, isopropyl, monofluoromethyl, difluoromethyl, trifluoromethyl, hydroxymethyl, aminomethyl, methoxy, ethoxy, propoxy, isopropoxy, monofluoromethoxy, difluoromethoxy and trifluoromethoxy;

$R^2$ and $R^3$ are respectively independently selected from a group consisting of H, halogen, hydroxyl, amino, nitro, cyano, methyl, ethyl, propyl, isopropyl, trifluoromethyl, trifluoroethyl, trifluoropropyl, hydroxymethyl, hydroxyethyl, hydroxypropyl, aminomethyl, aminoethyl, aminopropyl, methoxy, ethoxy and propoxy, and cyclobutyl, cyclopentyl, cyclohexyl, tetrahydrofuran, tetrahydrothienyl, tetrahydropyrrolidinyl, tetrahydropyrazolyl, tetrahydroimidazolidinyl, tetrahydropyranyl, tetrahydrothiopyranyl, piperidinyl, piperazinyl, hexahydropyrimidinyl, or morpholinyl substituted optionally by 1-2 Q1; and each Q1 is independently selected from a group consisting of halogen, hydroxyl, amino, nitro, cyano, methyl, ethyl, propyl, isopropyl, trifluoromethyl, methoxy, ethoxy and trifluoromethoxy;

or $R^2$, $R^3$, and the carbon atom linked to them together form the following groups substituted optionally by 1-2 Q2:

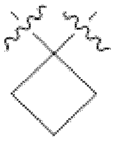, 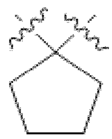, 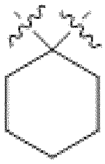, 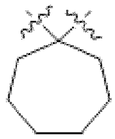, 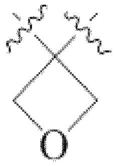, 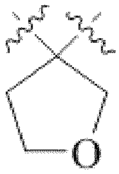, 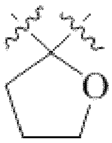, 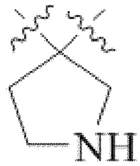,

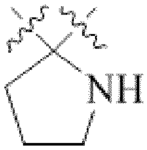, 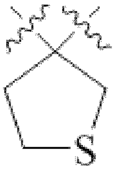, 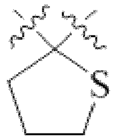, 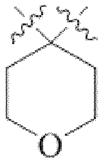, 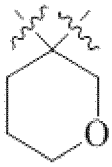, 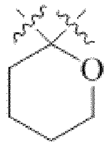, 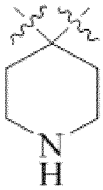, 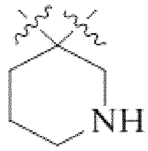,

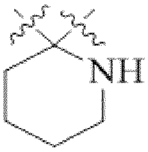, 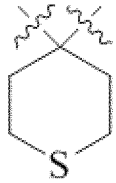, 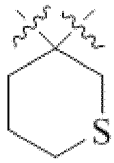, 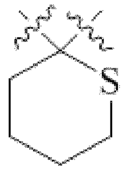, 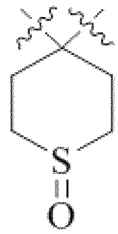, 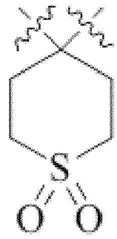, 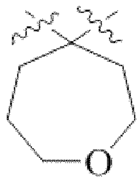,

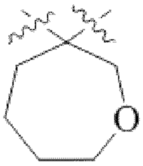, 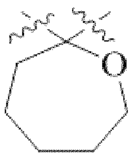, 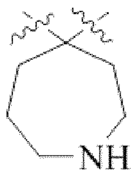, 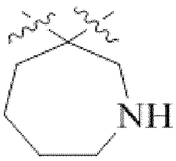, 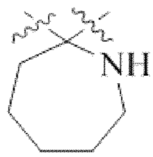, 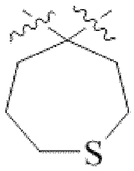, 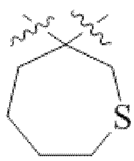,

or

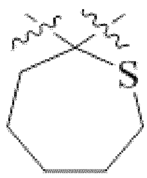;

each Q2 is independently selected from a group consisting of halogen, hydroxyl, amino, nitro, cyano, carbonyl, methyl, ethyl, propyl, isopropyl, trifluoromethyl, methylamino, ethylamino, dimethylamino, diethylamino, methoxy, ethoxy and trifluoromethoxy;
$R^4$ is H; $R^5$ and $R^6$ are respectively independently selected from a group consisting of H, halogen, hydroxyl, amino, nitro, cyano, methyl, ethyl, propyl, isopropyl, trifluoromethyl, methoxy, ethoxy and trifluoromethoxy; and $R^7$ is selected from a group consisting of H, methyl, ethyl, propyl, isopropyl, trifluoromethyl, hydroxymethyl and aminomethyl.

**7.** The compound, the pharmaceutically acceptable salt thereof, or the isomer thereof according to claim 1, wherein it is selected from the following compounds:

| number | structure | number | structure |
|---|---|---|---|
| compound 1 | 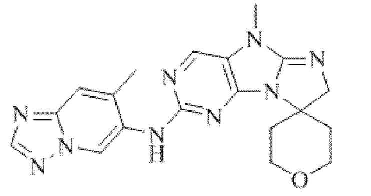 | compound 2 | 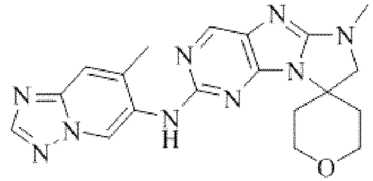 |
| compound 3 | 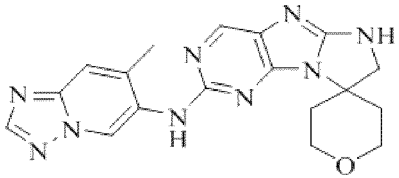 | compound 4 | 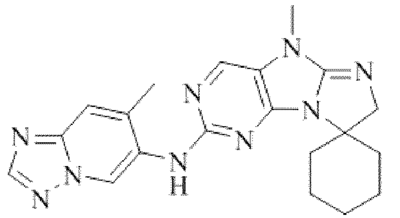 |
| compound 5 | 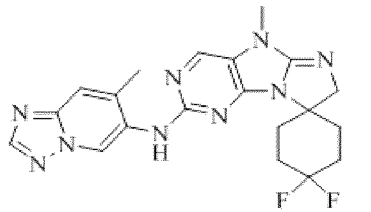 | compound 6 | 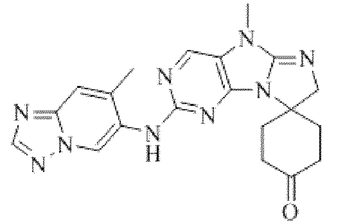 |
| compound 7 | 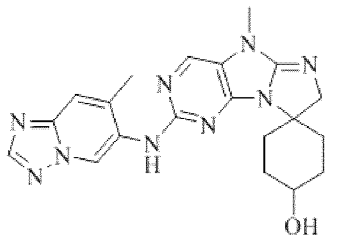 | compound 7-1 | 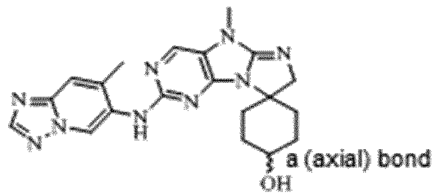 |

(continued)

| number | structure | number | structure |
|---|---|---|---|
| compound 7-2 | 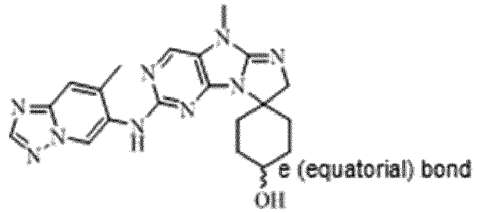 | compound 8 | 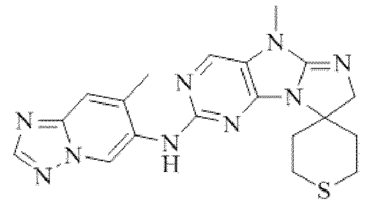 |
| compound 9 | 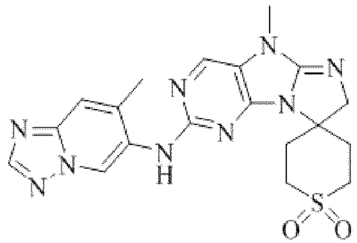 | compound 10 | 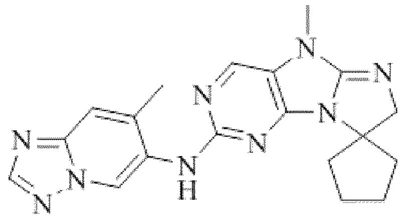 |
| compound 11 | 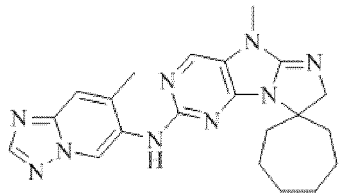 | compound 12 | 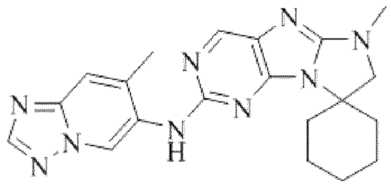 |
| compound 13 | 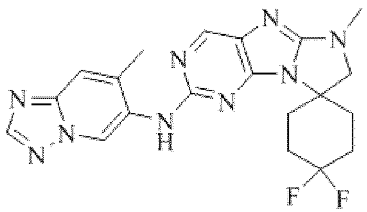 | compound 14 | 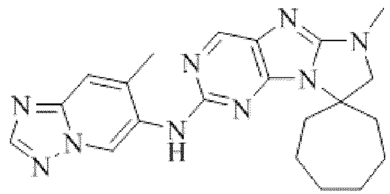 |

(continued)

| number | structure | number | structure |
|---|---|---|---|
| compound 15 | 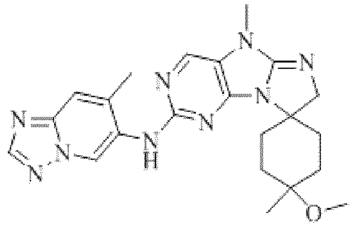 | compound 15-1 | 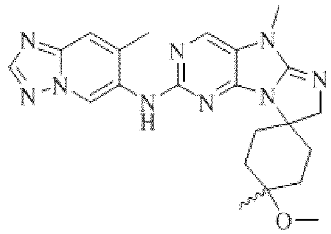 |
| compound 16 | 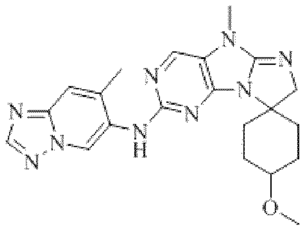 | compound 16-1 | 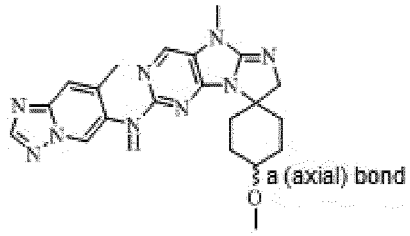 |
| compound 16-2 | 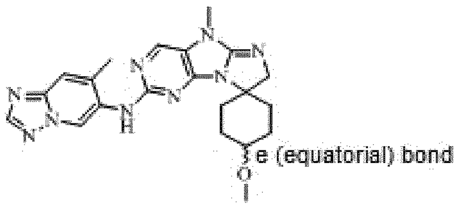 | compound 17 | 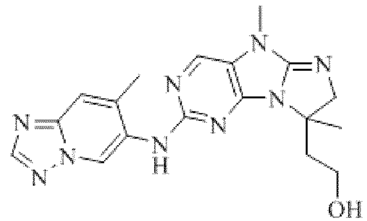 |
| compound 18 | 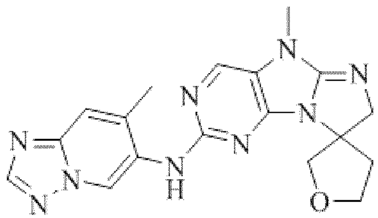 | compound 19 | 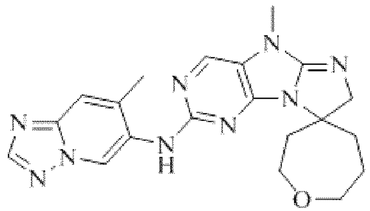 |

(continued)

| number | structure | number | structure |
|---|---|---|---|
| compound 19-1 | 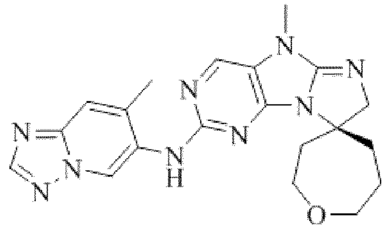 | compound 19-2 | 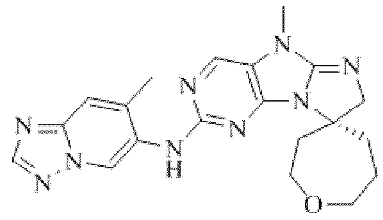 |
| compound 20 | 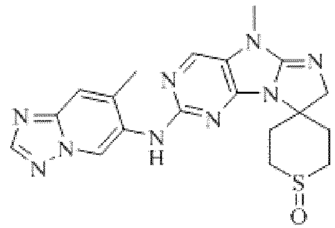 | | |

**8.** A pharmaceutical formulation comprising the compound, the pharmaceutically acceptable salt thereof, or the isomer thereof according to any one of claims 1-7, wherein it comprises one or more of pharmaceutically acceptable excipients, and the pharmaceutical formulation is any one pharmaceutically acceptable dosage form.

**9.** A pharmaceutical composition comprising the compound, the pharmaceutically acceptable salt thereof, or the isomer thereof according to any one of claims 1-7, wherein it comprises one or more of second therapeutic active agent, and the second therapeutic active agent is anticancer agents, which comprise a mitosis inhibitor, an alkylating agent, an antimetabolite, a DNA intercalate agent, an anti-tumor antibiotic, a growth factor inhibitor, a signal transduction inhibitor, a cell cycle inhibitor, an enzyme inhibitor, a vitamin A-like receptor regulator, a proteasome inhibitor, a topoisomerase inhibitor, a biological response regulator, a hormone drug, an angiogenesis inhibitor, a cell growth inhibitor, a targeted antibody, an HMG-CoA reductase inhibitor and an isoprene based protein transferase inhibitor.

**10.** A use of the compound, the pharmaceutically acceptable salt thereof or the isomer thereof according to any one of claims 1-7, the pharmaceutical formulation according to claim 8, or the pharmaceutical composition according to claim 9 in preparation of a drug for preventing and/or treating a benign tumor or cancer, and the cancer comprises carcinoma in situ and metastatic cancer.

**11.** A use of the compound, the pharmaceutically acceptable salt thereof or the isomer thereof according to any one of claims 1-7, the pharmaceutical formulation according to claim 8, or the pharmaceutical composition according to claim 9 in preparation of a drug for preventing and/or treating a benign tumor or cancer, the drug is used in combination with radiotherapy and/or one or more anticancer agents, and the cancer comprises carcinoma in situ and metastatic cancer.

**12.** A use of the compound, the pharmaceutically acceptable salt thereof or the isomer thereof according to any one of claims 1-7, the pharmaceutical formulation according to claim 8, or the pharmaceutical composition according to claim 9 in preparation of a drug for making cancer cells sensitive to anticancer agents and/or radiotherapy.

**13.** A kit, comprising:

(a) an effective amount of one or more of the compound, the pharmaceutically acceptable salt thereof or the isomer thereof according to any one of claims 1-7;
(b) an effective amount of one or more of anticancer agents.

**14.** An intermediate shown in formula (V),

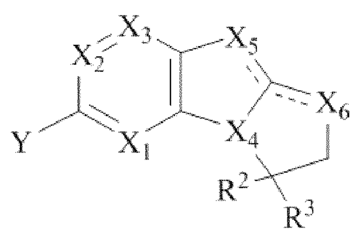

(V)

wherein, $X_1$, $X_2$, $X_3$, $X_4$, $X_5$, $X_6$, $R^1$, $R^2$, $R^3$, $R^4$, $R^5$, $R^6$, $R^7$, Q1, Q2, and virtual bond --- are described according to any one of claims 1-6; and
Y is halogen, amino, hydroxyl, or sulfydryl.

**INTERNATIONAL SEARCH REPORT**

International application No. **PCT/CN2022/070195**

**A. CLASSIFICATION OF SUBJECT MATTER**

C07D 519/00(2006.01)i; A61K 31/527(2006.01)i; A61K 31/519(2006.01)i; A61P 35/00(2006.01)i

According to International Patent Classification (IPC) or to both national classification and IPC

**B. FIELDS SEARCHED**

Minimum documentation searched (classification system followed by classification symbols)

C07D, A61K, A61P

Documentation searched other than minimum documentation to the extent that such documents are included in the fields searched

Electronic data base consulted during the international search (name of data base and, where practicable, search terms used)

CNTXT, CNKICNABS, DWPI, VEN, CNTXT, REGISTRY, CAPLUS: 癌症, 肿瘤, 增殖, 稠合, 螺, 激酶, 抑制剂, 山东轩竹, sub-structure search on the basis of claims 1 and 14, PI3k, dna pk, spiro, fused, cancer, tumor, tumour, proliferation, xuanzhu, kinase, inhibitor

**C. DOCUMENTS CONSIDERED TO BE RELEVANT**

| Category* | Citation of document, with indication, where appropriate, of the relevant passages | Relevant to claim No. |
|---|---|---|
| A | WO 2020238900 A1 (DIZAL (JIANGSU) PHARMACEUTICAL CO., LTD.) 03 December 2020 (2020-12-03) entire document | 1-14 |
| A | WO 2006126010 A2 (KUDOS PHARM LTD et al.) 30 November 2006 (2006-11-30) entire document | 1-14 |
| A | CN 111757876 A (VERTEX PHARMACEUTICALS INCORPORATED) 09 October 2020 (2020-10-09) entire document | 1-14 |
| A | CN 101754964 A (KUDOS PHARM LTD. et al.) 23 June 2010 (2010-06-23) entire document | 1-14 |
| A | Ruiz Ruiz, Maria del Carmen et al. "The 't-Amino Effect' of ortho-nitroso amines. synthesis of 2, 6-diaminoadenine derivatives from 6-(dialkylamino)-5-nitrosopyrimidines" *Helvetica Chimica Acta,* Vol. 94, No. 5, 31 December 2011 (2011-12-31), pages 785-800, see scheme 2 compound 24 | 1-14 |
| PA | WO 2021098813 A1 (MEDSHINE DISCOVERY INC.) 27 May 2021 (2021-05-27) entire document | 1-14 |

☑ Further documents are listed in the continuation of Box C. ☑ See patent family annex.

* Special categories of cited documents:
- "A" document defining the general state of the art which is not considered to be of particular relevance
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **06 March 2022** | **11 March 2022** |
| Name and mailing address of the ISA/CN<br>**China National Intellectual Property Administration (ISA/CN)**<br>**No. 6, Xitucheng Road, Jimenqiao, Haidian District, Beijing 100088, China**<br>Facsimile No. **(86-10)62019451** | Authorized officer<br><br>Telephone No. |

Form PCT/ISA/210 (second sheet) (January 2015)

**INTERNATIONAL SEARCH REPORT**

International application No.
**PCT/CN2022/070195**

**C. DOCUMENTS CONSIDERED TO BE RELEVANT**

| Category* | Citation of document, with indication, where appropriate, of the relevant passages | Relevant to claim No. |
|---|---|---|
| PA | WO 2021213460 A1 (SHANDONG XUANZHU PHARMA CO., LTD.) 28 October 2021 (2021-10-28)<br>entire document | 1-14 |

Form PCT/ISA/210 (second sheet) (January 2015)

**INTERNATIONAL SEARCH REPORT**
**Information on patent family members**

International application No.
**PCT/CN2022/070195**

| Patent document cited in search report | Publication date (day/month/year) | Patent family member(s) | Publication date (day/month/year) |
|---|---|---|---|
| WO 2020238900 A1 | 03 December 2020 | AR 119018 A1 | 17 November 2021 |
| | | TW 202110849 A | 16 March 2021 |
| | | CA 3137598 A1 | 03 December 2020 |
| | | BR 112021023824 A2 | 04 January 2022 |
| | | AU 2020281332 A1 | 04 November 2021 |
| | | AU 2020281332 A8 | 18 November 2021 |
| WO 2006126010 A2 | 30 November 2006 | WO 2006126010 A3 | 29 March 2007 |
| | | US 2009035394 A1 | 05 February 2009 |
| | | JP 2008542253 A | 27 November 2008 |
| | | EP 1895997 A2 | 12 March 2008 |
| CN 111757876 A | 09 October 2020 | US 2020353101 A1 | 12 November 2020 |
| | | BR 112020014315 A2 | 08 December 2020 |
| | | IL 276080 D0 | 31 August 2020 |
| | | EP 3740480 A1 | 25 November 2020 |
| | | KR 20200110685 A | 24 September 2020 |
| | | CA 3088792 A1 | 25 July 2019 |
| | | MA 51619 A | 14 April 2021 |
| | | EA 202091708 A1 | 10 November 2020 |
| | | AU 2019209293 A1 | 03 September 2020 |
| | | JP 2021511314 A | 06 May 2021 |
| | | WO 2019143678 A1 | 25 July 2019 |
| CN 101754964 A | 23 June 2010 | KR 20100063701 A | 11 June 2010 |
| | | WO 2009010761 A1 | 22 January 2009 |
| | | CL 2008002130 A1 | 02 January 2009 |
| | | TW 200918528 A | 01 May 2009 |
| | | BR PI0814797 A2 | 03 February 2015 |
| | | AU 2008277418 A1 | 22 January 2009 |
| | | JP 2010533694 A | 28 October 2010 |
| | | RU 2009149210 A | 27 August 2011 |
| | | CA 2693926 A1 | 22 January 2009 |
| | | AR 067613 A1 | 14 October 2009 |
| | | UY 31232 A1 | 02 March 2009 |
| | | EP 2176254 A1 | 21 April 2010 |
| | | US 2009042865 A1 | 12 February 2009 |
| | | IN 25DELNP2010 A | 23 July 2010 |
| WO 2021098813 A1 | 27 May 2021 | TW 202120505 A | 01 June 2021 |
| WO 2021213460 A1 | 28 October 2021 | CN 113549092 A | 26 October 2021 |

Form PCT/ISA/210 (patent family annex) (January 2015)